# EUROPÄISCHE PATENTSCHRIFT

(11) **EP 3 266 766 B1**
(45) Veröffentlichungstag und Bekanntmachung des Hinweises auf die Patenterteilung: **12.09.2018**
(21) Anmeldenummer: 17178003.4
(22) Anmeldetag: 27.06.2017
(51) Int. Cl.: C07D 211/58

(54) **SYNTHESE VON TRIACETONDIAMINVERBINDUNGEN DURCH REDUKTIVE AMINIERUNG AUSGEHEND VON TRIACETONDIAMIN UND DESSEN DERIVATEN**
SYNTHESIS OF TRIACETONE DIAMINE COMPOUNDS BY MEANS OF REDUCTIVE AMINATION BASED ON TRIACETONE DIAMINE AND ITS DERIVATIVES
SYNTHÈSE DES LIAISONS DE DIAMINE DE TRIACÉTONE PAR AMINATION RÉDUCTRICE PROVENANT DE DIAMINE DE TRIACÉTONE ET LEURS DÉRIVÉS

(30) Priorität: 07.07.2016 DE 102016212378
(43) Veröffentlichungstag der Anmeldung: 10.01.2018
(73) Patentinhaber: Evonik Degussa GmbH, 45128 Essen (DE)
(72) Erfinder: Minke, Katharina, 45130 Essen (DE); Willy, Benjamin, 40211 Düsseldorf (DE)

(56) Entgegenhaltungen:
- EP-A1- 0 857 719
- EP-A1- 3 048 097
- EP-A2- 0 302 020
- EP-A2- 0 319 480

## Beschreibung

Die vorliegende Erfindung betrifft ein Verfahren, in welchem 4-Amino-2,2,6,6-tetramethylpiperidin ("Triacetondiamin";TAD) bzw. eine 4-Amino-2,2,6,6-tetramethylpiperidinylgruppe mit einer Carbonylverbindung in einer reduktiven Aminierung umgesetzt wird. Das erfindungsgemäße Verfahren eignet sich insbesondere zur Herstellung von Derivaten des TAD.

### Hintergrund der Erfindung

Die Gruppe der sogenannten "*hindered amine light stabilizers* (HALS)" wird zur Stabilisierung von Polyolefinen wie zum Beispiel Polyethylen und Polypropylen gegen die Einwirkung von äußeren Einflüssen wie zum Beispiel UV-Licht und hohen Temperaturen eingesetzt. Dabei enthalten die HALS-Derivate als funktionale Einheit eine 2,2,6,6-Tetramethylpiperidinyl-Gruppe, welche elementar für die stabilisierende Wirkung ist. Die stabilisierende Wirkung ist dadurch gekennzeichnet, dass zum Beispiel die optischen oder mechanischen Eigenschaften des stabilisierten Polymers gegenüber dem unstabilisierten Polymer länger erhalten bleiben, also zum Beispiel ein Vergilbungsprozess des Polymers verlangsamt wird.

Die Einführung der 2,2,6,6-Tetramethylpiperidinyl-Gruppe erfolgt im Allgemeinen durch Verwendung von 2,2,6,6-Tetramethyl-4-piperidinon (Triacetonamin; TAA) als Edukt in der Synthesereaktion. Die Umsetzung von TAA erfolgt dabei meist durch reduktive Aminierung, das heißt durch Reaktion mit einem Amin unter reduktiven Bedingungen, wobei die entsprechenden Derivate des 4-Amino-2,2,6,6-tetramethylpiperidins erhalten werden. Diese werden dann entweder direkt oder nach weiteren chemischen Modifikationen als HALS eingesetzt.

Die Herstellung von Derivaten des TAD durch reduktive Aminierung von TAA mit Aminen ist im Stand der Technik, zum Beispiel in der EP 0 857 719 B1 (als kontinuierliches Verfahren), der DE 692 20 708 T2 (als nicht kontinuierliches Verfahren) und der EP 0 302 202 A2 beschrieben. Den Verfahren des Standes der Technik ist gemein, dass man TAA mit einem Amin (RNH₂; der Rest R ist dabei z. B. ein Kohlenwasserstoffrest) umsetzt. Dadurch erhält man intermediär ein Imin, welches in Anwesenheit von molekularem Wasserstoff mittels reduktiver Aminierung an einem Edelmetall- oder Nichtedelmetallkatalysator [M] (zum Beispiel Ru, Pd, Pt, Co, Ni) zum gewünschten Zielprodukt reagiert. Dabei werden hohe (EP 0 857 719 B1) oder niedrige (DE 692 20 708 T2) Wasserstoffpartialdrücke angewandt und das entsprechende TAD-Derivat erhalten.

Das Reaktionsschema **<1>** gemäß dieser herkömmlichen Verfahren lässt sich wie folgt zusammenfassen (wobei R und [M] die oben angegebenen Bedeutungen haben):

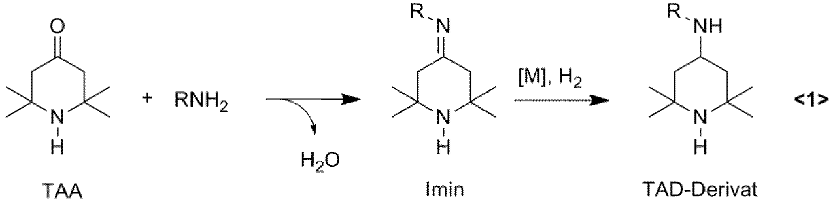

Gerade durch das in der EP 0 857 719 B1 beschriebene Verfahren werden hohe Ausbeuten an N-substituiertem TAD-Derivaten mit einer Reinheit von teilweise > 99 % gewonnen. Allerdings basiert dieses Verfahren (und natürlich auch das in DE 692 20 708 T2 beschriebene) auf TAA als Edukt, und ein gewisser Restgehalt an TAA im Endprodukt ist dadurch unvermeidlich.

Dies bringt insbesondere beim Einsatz der nach den Verfahren des Standes der Technik hergestellten TAD-Derivate einen entscheidenden Nachteil mit sich. Wenn diese so gewonnenen TAD-Derivate oder ihre Folgeprodukte als stabilisierende Additive für Polyolefine zum Einsatz kommen, so ist es nachteilig, wenn die Additive selbst zur einer Verfärbung des Materials führen. Hier wirkt sich besonders nachteilig aus, dass TAA in Anwesenheit von Sauerstoff stark gefärbte Zersetzungsprodukte bildet, welche die Qualität der Kunststoffe hinsichtlich deren Farbeigenschaften stark beeinträchtigen. Schon Spuren von TAA im Endprodukt führen zu einer merklichen Verfärbung des Materials über die Zeit.

Eine komplette Vermeidung oder zumindest weitestmögliche Reduzierung jeglichen Restgehalts an TAA ist daher beim Einsatz von Lichtstabilisatoren, welche über 2,2,6,6-Piperidinyl-Substiuenten verfügen, bedeutsam. Dies können die Verfahren des Standes der Technik per se nicht leisten, da sie zwangsläufig von TAA als Edukt ausgehen.

Daneben bringt der Einsatz von TAA als Rohstoff weitere Nachteile mit sich, die sich besonders in großtechnischen Anlagen auswirken. So ist TAA bei Raumtemperatur ein Feststoff und schmilzt erst bei ∼ 35 °C. Dies erfordert zusätzlichen apparativen und energetischen Aufwand, um TAA in einen verarbeitungsfähigen Zustand zu überführen.

Zusätzlich ist eine Herstellung von am exozyklischen Stickstoffatom disubstituierten TAD-Derivate durch reduktive Aminierung mit den Verfahren des Standes der Technik nicht oder nur mit sehr geringem Umsatz möglich. Solche disubstituierten TAD-Derivate sind aber auch als Lichtstabilisatoren von Interesse.

Die Aufgabe der vorliegenden Erfindung bestand somit darin, ein Verfahren zur Herstellung von TAD-Verbindungen, welche die vorher beschriebenen Nachteile nicht aufweist, bereitzustellen. Insbesondere soll das Verfahren die Herstellung von TAD-Verbindungen mit einer höheren Farbstabilität ermöglichen und eine breitere Palette von TAD-Verbindungen zugänglich machen.

Es wurde nun überraschend ein Verfahren gefunden, welches die beschriebene Aufgabe löst.

### Kurzbeschreibunq der Erfindung

Die vorliegende Erfindung betrifft in einem ersten Aspekt ein Verfahren gemäß den folgenden Punkten 1.1 bis 1.14.
1.1 Verfahren zur Herstellung einer *N*-substituierten Triacetondiaminverbindung,
   dadurch gekennzeichnet, dass man mindestens eine Triacetondiaminverbindung **(I)** mit mindestens einer Carbonylverbindung **(II)** unter reduktiven Bedingungen umsetzt,
   wobei die Triacetondiaminverbindung **(I)** ausgewählt ist aus der Gruppe bestehend aus den chemischen Strukturen **(I-A)**, **(I-B)**, **(I-C)**, **(I-D)**, **(I-E)** mit 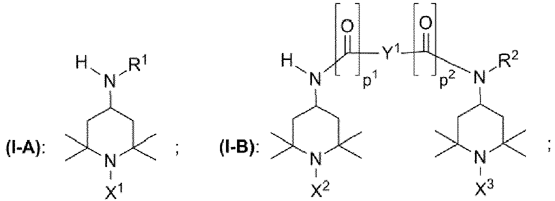 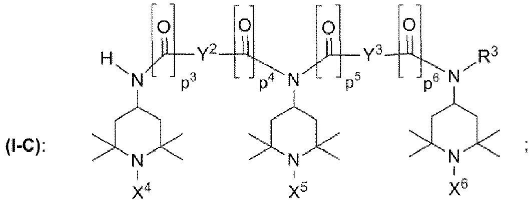 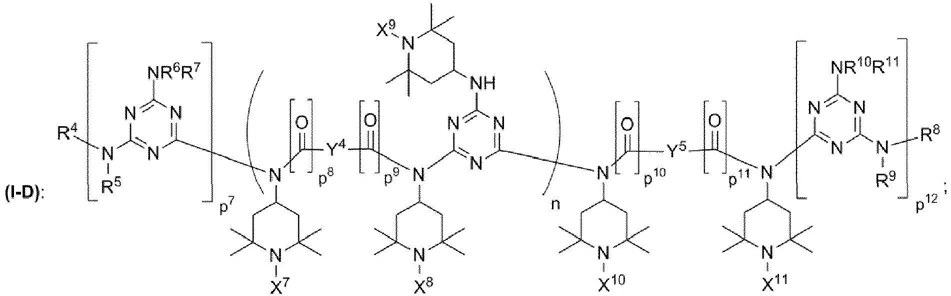 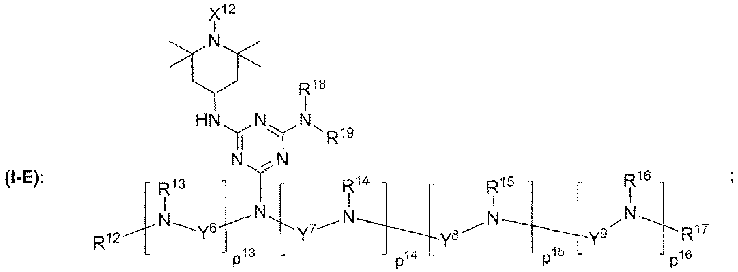
   wobei n eine ganze Zahl aus dem Bereich 1 bis 20 ist;
   wobei p¹, p², p³, p⁴, p⁵, p⁶, p⁷, p⁸, p⁹, p¹⁰, p¹¹, p¹², p¹³, p¹⁴, p¹⁵, p¹⁶ unabhängig voneinander jeweils 0 oder 1 ist;
   wobei X¹, X², X³, X⁴, X⁵, X⁶, X⁷, X⁸, X⁹, X¹⁰, X¹¹, X¹² unabhängig voneinander jeweils ausgewählt ist aus der Gruppe bestehend aus Wasserstoff, OH, -O·, unverzweigte oder verzweigte Alkylgruppe mit 1 bis 10 Kohlenstoffatomen, unverzweigte oder verzweigte Alkoxygruppe mit 1 bis 10 Kohlenstoffatomen;
   wobei Y¹, Y², Y³, Y⁴, Y⁵, Y⁶, Y⁷, Y⁸, Y⁹ unabhängig voneinander jeweils ausgewählt ist aus der Gruppe bestehend aus
      unverzweigte oder verzweigte Alkylengruppe mit 1 bis 30 Kohlenstoffatomen,
      zweiwertige gesättigte Kohlenwasserstoffgruppe mit 3 bis 30 Kohlenstoffatomen, welche mindestens einen gesättigten Ring aus 3 bis 30 Kohlenstoffatomen aufweist,
      zweiwertige Kohlenwasserstoffgruppe mit 6 bis 30 Kohlenstoffatomen, von denen mindestens 6 Kohlenstoffatome in einem aromatischen System vorliegen und die übrigen Kohlenstoffatome, falls vorhanden, gesättigt sind,
      ein verbrückender Rest, der eine chemische Struktur ausgewählt aus der Gruppe bestehend aus **(i)**, **(ii)** mit 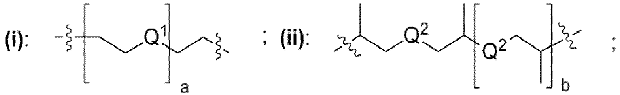
      aufweist, wobei
         Q¹, Q² unabhängig voneinander jeweils ausgewählt aus der Gruppe bestehend aus -O-, -S-, -NH- oder -NR'- mit R' = unverzweigte oder verzweigte Alkylgruppe mit 1 bis 6 Kohlenstoffatomen sind,
         wobei a eine ganze Zahl ausgewählt aus dem Bereich 1 bis 50 ist,
         wobei b eine ganze Zahl ausgewählt aus dem Bereich 0 bis 50 ist,
   und wobei Y¹ auch eine direkte Bindung sein kann, falls mindestens einer von p¹ und p² den Wert 1 aufweist,
   und wobei Y² auch eine direkte Bindung sein kann, falls mindestens einer von p³ und p⁴ den Wert 1 aufweist,
   und wobei Y³ auch eine direkte Bindung sein kann, falls mindestens einer von p⁵ und p⁶ den Wert 1 aufweist,
   und wobei Y⁴ auch eine direkte Bindung sein kann, falls mindestens einer von p⁸ und p⁹ den Wert 1 aufweist,
   und wobei Y⁵ auch eine direkte Bindung sein kann, falls mindestens einer von p¹⁰ und p¹¹ den Wert 1 aufweist;
   wobei die Reste R¹, R², R³, R⁴, R⁵, R⁶, R⁷, R⁸, R⁹, R¹⁰, R¹¹ unabhängig voneinander jeweils ausgewählt sind aus der Gruppe bestehend aus
      Wasserstoff,
      unverzweigte oder verzweigte Alkylgruppe mit 1 bis 30 Kohlenstoffatomen, bei welcher mindestens ein Wasserstoffrest durch einen Rest ausgewählt aus der Gruppe bestehend aus -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann,
      unverzweigte oder verzweigte Acylgruppe mit 1 bis 30 Kohlenstoffatomen, bei welcher mindestens ein Wasserstoffrest durch einen Rest ausgewählt aus der Gruppe bestehend aus -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann,
      einem Rest, der eine chemischen Struktur ausgewählt aus der Gruppe bestehend aus **(iii), (iv), (v), (vi), (vii), (viii), (ix)** mit 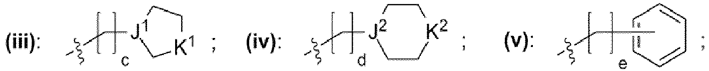 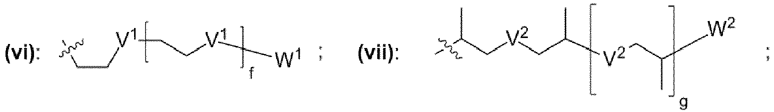 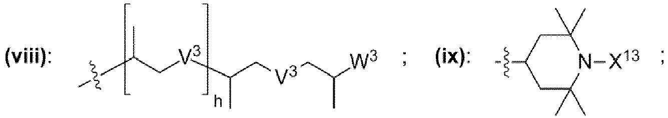
      aufweist,
         wobei J¹, J² unabhängig voneinander jeweils ausgewählt aus der Gruppe bestehend aus CH, N sind,
         wobei K¹, K² unabhängig voneinander jeweils ausgewählt aus der Gruppe bestehend aus -O-, -NH-, -N(CH₃)-, -N(CH₂CH₃)-, -S-, -CH₂- sind,
         wobei V¹, V², V³ unabhängig voneinander jeweils ausgewählt aus der Gruppe bestehend aus -O-, -S-, -NH-, -NR"- mit R" = unverzweigte oder verzweigte Alkylgruppe mit 1 bis 6 Kohlenstoffatomen sind,
         wobei W¹, W², W³ unabhängig voneinander jeweils ausgewählt aus der Gruppe bestehend aus H, Methyl, Ethyl sind,
         wobei c, d, e, f, g, h unabhängig voneinander jeweils eine ganze Zahl aus dem Bereich 0 bis 50 ist,
         wobei X¹³ ausgewählt ist aus der Gruppe bestehend aus Wasserstoff, -OH, -O·, unverzweigte oder verzweigte Alkylgruppe mit 1 bis 10 Kohlenstoffatomen, unverzweigte oder verzweigte Alkoxygruppe mit 1 bis 10 Kohlenstoffatomen,
      wobei in den chemischen Strukturen **(iii), (iv), (v), (vi), (vii), (viii), (ix)** mindestens ein an ein Kohlenstoffatom gebundener Wasserstoffrest durch einen Rest ausgewählt aus der Gruppe bestehend aus
      -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann;
   wobei die Reste R¹², R¹³, R¹⁴, R¹⁵, R¹⁶, R¹⁷ unabhängig voneinander jeweils ausgewählt sind aus der Gruppe bestehend aus
      Wasserstoff,
      unverzweigte oder verzweigte Alkylgruppe mit 1 bis 30 Kohlenstoffatomen,
      einer Gruppe mit der chemischen Struktur (x) mit 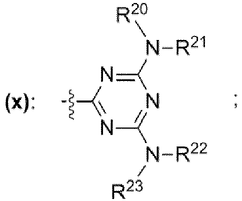
   wobei die Reste R¹⁸, R¹⁹, R²⁰, R²¹, R²², R²³ unabhängig voneinander jeweils ausgewählt sind aus der Gruppe bestehend aus
      Wasserstoff,
      unverzweigte oder verzweigte Alkylgruppe mit 1 bis 30 Kohlenstoffatomen, bei welcher mindestens ein Wasserstoffrest durch einen Rest ausgewählt aus der Gruppe bestehend aus -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann,
      unverzweigte oder verzweigte Acylgruppe mit 1 bis 30 Kohlenstoffatomen, bei welcher mindestens ein Wasserstoffrest durch einen Rest ausgewählt aus der Gruppe bestehend aus -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann,
      einem Rest, der eine chemischen Struktur ausgewählt aus der Gruppe bestehend aus (xi), **(xii), (xiii), (xiv), (xv), (xvi), (xvii)** mit 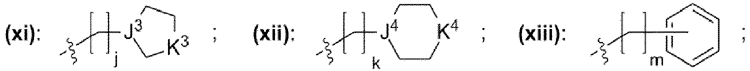 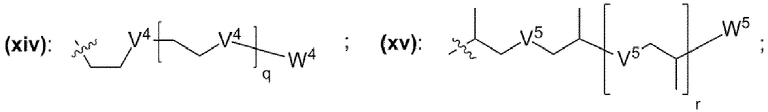 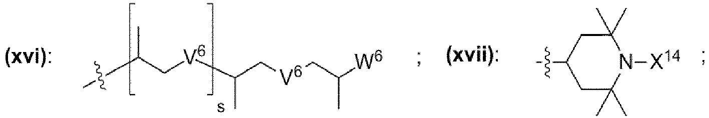
      aufweist,
         wobei J³, J⁴ unabhängig voneinander jeweils ausgewählt aus der Gruppe bestehend aus CH, N sind,
         wobei K³, K⁴ unabhängig voneinander jeweils ausgewählt aus der Gruppe bestehend aus -O-, -NH-, -N(CH₃)-, -N(CH₂CH₃)-, -S-, -CH₂- sind,
         wobei V⁴, V⁵, V⁶ unabhängig voneinander jeweils ausgewählt aus der Gruppe bestehend aus -O-, -S-, -NH-, -NR"'- mit R"' = unverzweigte oder verzweigte Alkylgruppe mit 1 bis 6 Kohlenstoffatomen sind,
         wobei W⁴, W⁵, W⁶ unabhängig voneinander jeweils ausgewählt aus der Gruppe bestehend aus H, Methyl, Ethyl sind,
            wobei j, k, m, q, r, s unabhängig voneinander jeweils eine ganze Zahl aus dem Bereich 0 bis 50 ist,
         wobei X¹⁴ ausgewählt ist aus der Gruppe bestehend aus Wasserstoff, -OH, -O·, unverzweigte oder verzweigte Alkylgruppe mit 1 bis 10 Kohlenstoffatomen, unverzweigte oder verzweigte Alkoxygruppe mit 1 bis 10 Kohlenstoffatomen,
         wobei in den chemischen Strukturen **(xi), (xii), (xiii), (xiv), (xv), (xvi), (xvii)** mindestens ein an ein Kohlenstoffatom gebundener Wasserstoffrest durch einen Rest ausgewählt aus der Gruppe bestehend aus -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann,
   und mit der Maßgabe, dass R¹² und R¹⁷ für p¹³ = p¹⁴ = p¹⁵ = p¹⁶ = 0 unabhängig voneinander jeweils auch eine Gruppe der chemischen Struktur **(xviii)** mit 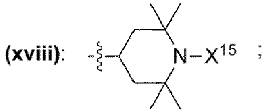
   sein können, wobei X¹⁵ ausgewählt ist aus der Gruppe bestehend aus Wasserstoff, OH, -O·, unverzweigte oder verzweigte Alkylgruppe mit 1 bis 10 Kohlenstoffatomen, unverzweigte oder verzweigte Alkoxygruppe mit 1 bis 10 Kohlenstoffatomen;
   und wobei die Carbonylverbindung **(II)** ausgewählt ist aus der Gruppe bestehend aus den chemischen Strukturen **(II-A)**, **(II-B)**, **(II-C)** mit 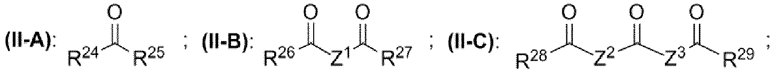
   wobei Z¹, Z², Z³ unabhängig voneinander jeweils ausgewählt sind aus der Gruppe bestehend aus
      direkte Bindung,
      unverzweigte oder verzweigte Alkylengruppe mit 1 bis 30 Kohlenstoffatomen,
      zweiwertige gesättigte Kohlenwasserstoffgruppe mit 3 bis 30 Kohlenstoffatomen, welche mindestens einen gesättigten Ring aus 3 bis 30 Kohlenstoffatomen aufweist,
      zweiwertige Kohlenwasserstoffgruppe mit 6 bis 30 Kohlenstoffatomen, von denen mindestens 6 Kohlenstoffatome in einem aromatischen System vorliegen und die übrigen Kohlenstoffatome, falls vorhanden, gesättigt sind,
      ein verbrückender Rest, der eine chemischen Struktur ausgewählt aus der Gruppe bestehend aus **(xix), (xx)** mit 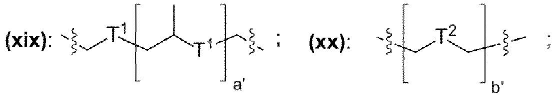
      aufweist, wobei
         T¹, T² unabhängig voneinander jeweils ausgewählt aus der Gruppe bestehend aus -O-, -S- oder -NR""- mit R"" = unverzweigte oder verzweigte Alkylgruppe mit 1 bis 6 Kohlenstoffatomen sind,
         wobei a' und b' unabhängig voneinander jeweils eine ganze Zahl ausgewählt aus dem Bereich 1 bis 50 ist;
   und wobei die Reste R²⁴, R²⁵, R²⁶, R²⁷, R²⁸, R²⁹ ausgewählt sind aus der Gruppe bestehend aus
      Wasserstoff,
      unverzweigte oder verzweigte Alkylgruppe mit 1 bis 30 Kohlenstoffatomen, bei welcher mindestens ein Wasserstoffrest durch einen Rest ausgewählt aus der Gruppe bestehend aus -OH, -OCH₃, -OCH₂CH₃, -N(CH₃)₂, -N(CH₂CH₃)₂, -N(CH₃)(CH₂CH₃) ersetzt sein kann,
      ein Rest, der eine chemischen Struktur ausgewählt aus der Gruppe bestehend aus **(xxi), (xxii), (xxiii), (xxiv), (xxv), (xxvi)** mit 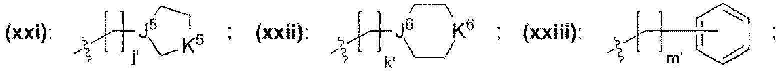 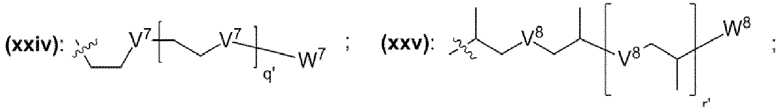 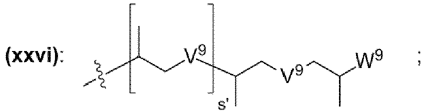
      aufweist,
         wobei J⁵, J⁶ unabhängig voneinander jeweils ausgewählt aus der Gruppe bestehend aus CH, N sind,
         wobei K⁵, K⁶ unabhängig voneinander jeweils ausgewählt aus der Gruppe bestehend aus -O-, -N(CH₃)-, -N(CH₂CH₃)-, -S-, -CH₂- sind,
         wobei V⁷, V⁸, V⁹ unabhängig voneinander jeweils ausgewählt aus der Gruppe bestehend aus -O-, -S-, -NR""'- mit R""' = unverzweigte oder verzweigte Alkylgruppe mit 1 bis 6 Kohlenstoffatomen sind,
         wobei W⁷, W⁸, W⁹ unabhängig voneinander jeweils ausgewählt aus der Gruppe bestehend aus H, Methyl, Ethyl sind,
         wobei j', k', m', q', r', s' unabhängig voneinander jeweils eine ganze Zahl ausgewählt aus dem Bereich 0 bis 50 ist,
         wobei in der chemischen Struktur **(xxi), (xxii), (xxiii), (xxiv), (xxv), (xxvi)** mindestens ein Wasserstoffrest durch einen Rest ausgewählt aus der Gruppe bestehend aus -OH, -OCH₃, -OCH₂CH₃, -N(CH₃)₂, -N(CH₂CH₃)₂, -N(CH₃)(CH₂CH₃) ersetzt sein kann;
   und wobei R²⁴, R²⁵, R²⁶, R²⁷, R²⁸, R²⁹ jeweils unabhängig voneinander ausgewählt sind, bis auf die Ausnahme, dass nicht gilt: R²⁴ = R²⁵ = Wasserstoff,
   und wobei man reduktive Bedingungen dadurch einstellt, dass man die mindestens eine Triacetondiaminverbindung **(I)** mit der mindestens einen Carbonylverbindung **(II)** in Gegenwart von Wasserstoff und in Gegenwart eines Vollkatalysators umsetzt, wobei der Vollkatalysator mindestens ein Metall **M** aufweist, wobei das Metall **M** ausgewählt ist aus der Gruppe bestehend aus Ag, V, Cr, Mo, Mn, Ni, Pd, Pt, Fe, Ru, Os, Co, Rh, Ir, Cu, insbesondere Ag, Fe, Ni, Co, Cu, bevorzugt Ni, Co, bevorzugter Ni.
1.2 Verfahren nach Punkt 1.1, wobei p¹ = p² = p³ = p⁴ = p⁵ = p⁶ = p⁸ = p⁹ = p¹⁰ = p¹¹ = 0 ist und wobei p⁷, p¹², p¹³, p¹⁴, p¹⁵, p¹⁶ unabhängig voneinander jeweils 0 oder 1 ist.
1.3 Verfahren nach Punkt 1.1 oder 1.2, wobei Y¹, Y², Y³, Y⁴, Y⁵, Y⁶, Y⁷, Y⁸, Y⁹ unabhängig voneinander jeweils ausgewählt ist aus der Gruppe bestehend aus
   unverzweigte oder verzweigte Alkylengruppe mit 1 bis 30 Kohlenstoffatomen,
   zweiwertige gesättigte Kohlenwasserstoffgruppe mit 3 bis 30 Kohlenstoffatomen, welche mindestens einen gesättigten Ring aus 3 bis 30 Kohlenstoffatomen aufweist;
      wobei bevorzugt Y¹, Y², Y³, Y⁴, Y⁵, Y⁶, Y⁷, Y⁸, Y⁹ unabhängig voneinander jeweils eine unverzweigte oder verzweigte Alkylengruppe mit 1 bis 12, noch bevorzugter mit 1 bis 6, Kohlenstoffatomen ist.
1.4 Verfahren nach einem oder mehreren der Punkte 1.1 bis 1.3, wobei die Reste R¹, R², R³, R⁴, R⁵, R⁶, R⁷, R⁸, R⁹, R¹⁰, R¹¹ unabhängig voneinander jeweils ausgewählt sind aus der Gruppe bestehend aus
   Wasserstoff,
   unverzweigte oder verzweigte Alkylgruppe mit 1 bis 30 Kohlenstoffatomen, bei welcher mindestens ein Wasserstoffrest durch einen Rest ausgewählt aus der Gruppe bestehend aus -OH, -NH₂, -OCH₃, -OCH₂CH₃, -NH(CH₃), -N(CH₃)₂, -NH(CH₂CH₃), -N(CH₂CH₃)₂, - N(CH₃)(CH₂CH₃) ersetzt sein kann,
   einem Rest, der eine chemischen Struktur **(ix)** mit 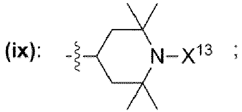
   aufweist,
   wobei X¹³ ausgewählt ist aus der Gruppe bestehend aus Wasserstoff, -OH, -O·, unverzweigte oder verzweigte Alkylgruppe mit 1 bis 10 Kohlenstoffatomen, unverzweigte oder verzweigte Alkoxygruppe mit 1 bis 10 Kohlenstoffatomen;
   und wobei die Reste R¹², R¹³, R¹⁴, R¹⁵, R¹⁶, R¹⁷ unabhängig voneinander jeweils ausgewählt sind aus der Gruppe bestehend aus
   Wasserstoff,
   unverzweigte oder verzweigte Alkylgruppe mit 1 bis 30 Kohlenstoffatomen,
   einer Gruppe mit der chemischen Struktur **(x)** mit 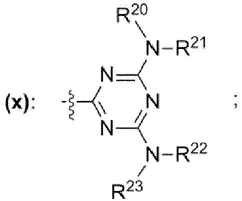
   wobei die Reste R¹⁸, R¹⁹, R²⁰, R²¹, R²², R²³ unabhängig voneinander jeweils ausgewählt sind aus der Gruppe bestehend aus
   Wasserstoff,
   unverzweigte oder verzweigte Alkylgruppe mit 1 bis 30 Kohlenstoffatomen, bei welcher mindestens ein Wasserstoffrest durch einen Rest ausgewählt aus der Gruppe bestehend aus -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann,
   einem Rest, der eine chemischen Struktur **(xvii)** mit 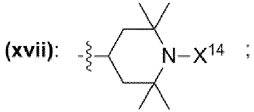
   aufweist,
      wobei X¹⁴ ausgewählt ist aus der Gruppe bestehend aus Wasserstoff, -OH, -O·, unverzweigte oder verzweigte Alkylgruppe mit 1 bis 10 Kohlenstoffatomen, unverzweigte oder verzweigte Alkoxygruppe mit 1 bis 10 Kohlenstoffatomen,
   und mit der Maßgabe, dass R¹² und R¹⁷ für p¹³ = p¹⁴ = p¹⁵ = p¹⁶ = 0 unabhängig voneinander jeweils auch eine Gruppe der chemischen Struktur **(xviii)** mit 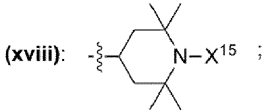 sein können, wobei X¹⁵ ausgewählt ist aus der Gruppe bestehend aus Wasserstoff, OH, -O·, unverzweigte oder verzweigte Alkylgruppe mit 1 bis 10 Kohlenstoffatomen, unverzweigte oder verzweigte Alkoxygruppe mit 1 bis 10 Kohlenstoffatomen.
1.5 Verfahren nach einem oder mehreren der Punkte 1.1 bis 1.4, wobei X¹ = X² = X³ = X⁴ = X⁵ = X⁶ = X⁷ = X⁸ = X⁹ = X¹⁰ = X¹¹ = X¹² = X¹³ = X¹⁴ = X¹⁵ = Wasserstoff ist.
1.6 Verfahren nach einem oder mehreren der Punkte 1.1 bis 1.5, wobei die Triacetondiaminverbindung **(I)** ausgewählt ist aus der Gruppe bestehend aus den chemischen Strukturen **(I-A)**, **(I-B)** und wobei die Reste R¹, R² unabhängig voneinander jeweils ausgewählt sind aus der Gruppe bestehend aus
   Wasserstoff,
   unverzweigte oder verzweigte Alkylgruppe mit 1 bis 12 Kohlenstoffatomen, bei welcher mindestens ein Wasserstoffrest durch einen Rest ausgewählt aus der Gruppe bestehend aus -OH, -NH₂, -OCH₃, -OCH₂CH₃, -NH(CH₃), -N(CH₃)₂, -NH(CH₂CH₃), -N(CH₂CH₃)₂, - N(CH₃)(CH₂CH₃) ersetzt sein kann.
1.7 Verfahren nach einem oder mehreren der Punkte 1.1 bis 1.6, wobei die Triacetondiaminverbindung **(I)** ausgewählt ist aus der Gruppe bestehend aus den chemischen Strukturen **(I-A)**, **(I-B)** und wobei die Reste R¹, R² unabhängig voneinander jeweils ausgewählt sind aus der Gruppe bestehend aus
   Wasserstoff,
   unverzweigte oder verzweigte Alkylgruppe mit 1 bis 12 Kohlenstoffatomen.
1.8 Verfahren nach einem oder mehreren der Punkte 1.1 bis 1.7, wobei
   Z¹, Z², Z³ unabhängig voneinander jeweils ausgewählt sind aus der Gruppe bestehend aus direkte Bindung,
   unverzweigte oder verzweigte Alkylengruppe mit 1 bis 30 Kohlenstoffatomen,
   zweiwertige gesättigte Kohlenwasserstoffgruppe mit 3 bis 30 Kohlenstoffatomen, welche mindestens einen gesättigten Ring aus 3 bis 30 Kohlenstoffatomen aufweist;
   wobei bevorzugt Z¹, Z², Z³ unabhängig voneinander jeweils eine unverzweigte oder verzweigte Alkylengruppe mit 1 bis 12 Kohlenstoffatomen ist.
1.9 Verfahren nach einem oder mehreren der Punkte 1.1 bis 1.8, wobei die Carbonylverbindung **(II)** ausgewählt ist aus der Gruppe bestehend aus den chemischen Strukturen **(II-A)**, **(II-B).**
1.10 Verfahren nach einem oder mehreren der Punkte 1.1 bis 1.9, wobei die Triacetondiaminverbindung **(I)** ausgewählt ist aus der Gruppe bestehend aus den chemischen Strukturen **(I-A)**, **(I-B)** und wobei die Carbonylverbindung **(II)** ausgewählt ist aus der Gruppe bestehend aus den chemischen Strukturen **(II-A)**, **(II-B)**, wobei
   p¹ = p² = 0;
   X¹ = X² = X³ = Wasserstoff;
   Y¹ und Z¹ unabhängig voneinander jeweils eine unverzweigte oder verzweigte Alkylengruppe mit 1 bis 12, bevorzugt 1 bis 6, Kohlenstoffatomen ist;
   R¹, R² unabhängig voneinander jeweils ausgewählt sind aus der Gruppe bestehend aus
   Wasserstoff,
   unverzweigte oder verzweigte Alkylgruppe mit 1 bis 12 Kohlenstoffatomen;
   R²⁴, R²⁵, R²⁶, R²⁷ ausgewählt sind aus der Gruppe bestehend aus
   Wasserstoff,
   unverzweigte oder verzweigte Alkylgruppe mit 1 bis 12 Kohlenstoffatomen;
   wobei R²⁴, R²⁵, R²⁶, R²⁷ jeweils unabhängig voneinander ausgewählt sind, bis auf die Ausnahme, dass nicht gilt: R²⁴ = R²⁵ = Wasserstoff.
1.11 Verfahren nach einem oder mehreren der Punkte 1.1 bis 1.10, wobei die Triacetondiaminverbindung **(I)** die chemische Struktur **(I-A)** aufweist und wobei die Carbonylverbindung **(II)** die chemische Struktur **(II-A)** aufweist, wobei
   X¹ = Wasserstoff;
   R¹ ausgewählt ist aus der Gruppe bestehend aus
   Wasserstoff,
   unverzweigte oder verzweigte Alkylgruppe mit 1 bis 8, bevorzugt 1 bis 6, Kohlenstoffatomen;
   R²⁴, R²⁵ ausgewählt sind aus der Gruppe bestehend aus
   Wasserstoff,
   unverzweigte oder verzweigte Alkylgruppe mit 1 bis 8, bevorzugt 1 bis 6, Kohlenstoffatomen;
   wobei R²⁴, R²⁵ jeweils unabhängig voneinander ausgewählt sind, bis auf die Ausnahme, dass nicht gilt: R²⁴ = R²⁵ = Wasserstoff.
1.12 Verfahren nach einem oder mehreren der Punkte 1.1 bis 1.11, wobei die Triacetondiaminverbindung **(I)** die chemische Struktur **(I-A)** aufweist und wobei die Carbonylverbindung **(II)** die chemische Struktur **(II-A)** aufweist, wobei X¹ = H; R¹ = H; R²⁴ ausgewählt aus der Gruppe bestehend aus Wasserstoff, Methyl ist; R²⁵ eine unverzweigte oder verzweigte Alkylgruppe mit 1 bis 8 Kohlenstoffatomen ist.
1.13 Verfahren nach einem oder mehreren der Punkte 1.1 bis 1.12, wobei man es in mindestens einem Lösungsmittel durchführt, wobei das Lösungsmittel ausgewählt ist aus der Gruppe bestehend aus aliphatische Lösungsmittel, aromatische Lösungsmittel, Ether, halogenierte Lösungsmittel, Amide, Thioverbindungen, Carbonsäuren, Alkohole, Wasser.
1.14 Verfahren nach einem oder mehreren der Punkte 1.1 bis 1.13, wobei man es bei einer Temperatur im Bereich von 20 °C bis 350 °C und einem Druck im Bereich von 2 bar bis 500 bar durchführt.

Die vorliegende Erfindung betrifft in einem zweiten Aspekt ein Verfahren gemäß den folgenden Punkten 2.1 bis 2.20.
2.1 Verfahren zur Herstellung einer *N*-substituierten Triacetondiaminverbindung,
   dadurch gekennzeichnet, dass man mindestens eine Triacetondiaminverbindung **(I)** mit mindestens einer Carbonylverbindung **(II)** unter reduktiven Bedingungen umsetzt,
   wobei die Triacetondiaminverbindung **(I)** ausgewählt ist aus der Gruppe bestehend aus den chemischen Strukturen **(I-A)**, **(I-B)**, **(I-C)**, **(I-D)**, **(I-E)** mit 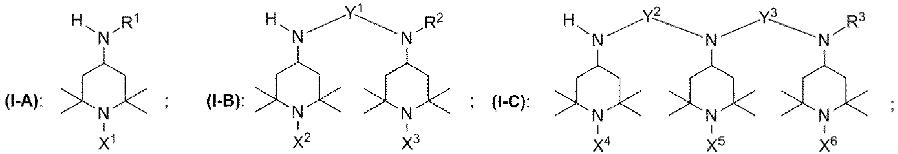 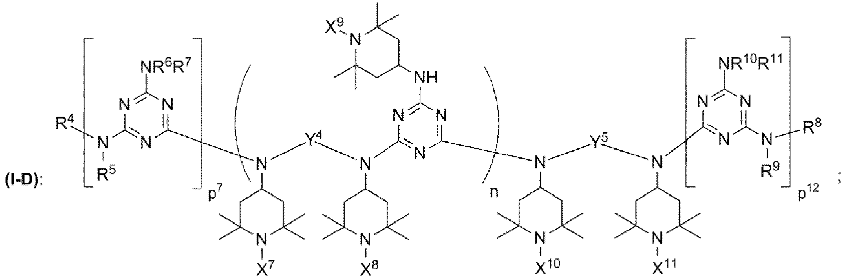 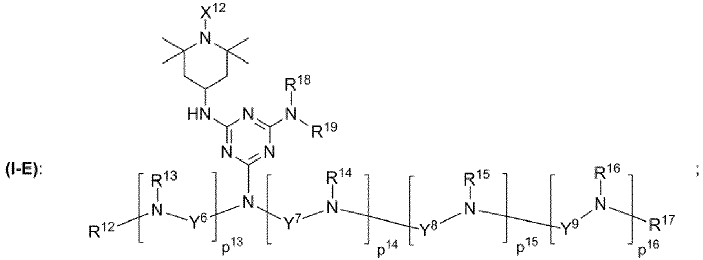
   wobei n eine ganze Zahl aus dem Bereich 1 bis 20 ist;
   wobei p⁷, p¹², p¹³, p¹⁴, p¹⁵, p¹⁶ unabhängig voneinander jeweils 0 oder 1 ist;
   wobei X¹, X², X³, X⁴, X⁵, X⁶, X⁷, X⁸, X⁹, X¹⁰, X¹¹, X¹² unabhängig voneinander jeweils ausgewählt ist aus der Gruppe bestehend aus Wasserstoff, OH, -O·, unverzweigte oder verzweigte Alkylgruppe mit 1 bis 10 Kohlenstoffatomen, unverzweigte oder verzweigte Alkoxygruppe mit 1 bis 10 Kohlenstoffatomen;
   wobei Y¹, Y², Y³, Y⁴, Y⁵, Y⁶, Y⁷, Y⁸, Y⁹ unabhängig voneinander jeweils ausgewählt ist aus der Gruppe bestehend aus
      unverzweigte oder verzweigte Alkylengruppe mit 1 bis 30 Kohlenstoffatomen,
      zweiwertige gesättigte Kohlenwasserstoffgruppe mit 3 bis 30 Kohlenstoffatomen, welche mindestens einen gesättigten Ring aus 3 bis 30 Kohlenstoffatomen aufweist;
   wobei die Reste R¹, R², R³, R⁴, R⁵, R⁶, R⁷, R⁸, R⁹, R¹⁰, R¹¹ unabhängig voneinander jeweils ausgewählt sind aus der Gruppe bestehend aus
      Wasserstoff,
      unverzweigte oder verzweigte Alkylgruppe mit 1 bis 30 Kohlenstoffatomen, bei welcher mindestens ein Wasserstoffrest durch einen Rest ausgewählt aus der Gruppe bestehend aus -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann,
      unverzweigte oder verzweigte Acylgruppe mit 1 bis 30 Kohlenstoffatomen, bei welcher mindestens ein Wasserstoffrest durch einen Rest ausgewählt aus der Gruppe bestehend aus -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann,
      einem Rest, der eine chemischen Struktur ausgewählt aus der Gruppe bestehend aus **(iii), (iv), (v), (vi), (vii), (viii), (ix)** mit 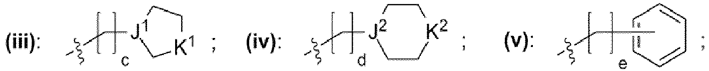 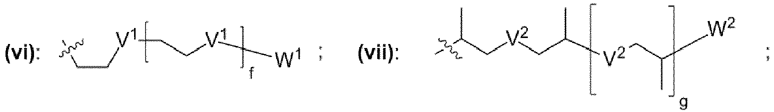 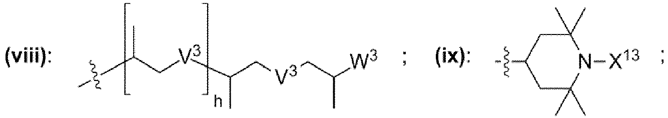
      aufweist,
         wobei J¹, J² unabhängig voneinander jeweils ausgewählt aus der Gruppe bestehend aus CH, N sind,
         wobei K¹, K² unabhängig voneinander jeweils ausgewählt aus der Gruppe bestehend aus -O-, -NH-, -N(CH₃)-, -N(CH₂CH₃)-, -S-, -CH₂- sind,
         wobei V¹, V², V³ unabhängig voneinander jeweils ausgewählt aus der Gruppe bestehend aus -O-, -S-, -NH-, -NR"- mit R" = unverzweigte oder verzweigte Alkylgruppe mit 1 bis 6 Kohlenstoffatomen sind,
         wobei W¹, W², W³ unabhängig voneinander jeweils ausgewählt aus der Gruppe bestehend aus H, Methyl, Ethyl sind,
         wobei c, d, e, f, g, h unabhängig voneinander jeweils eine ganze Zahl aus dem Bereich 0 bis 50 ist,
         wobei X¹³ ausgewählt ist aus der Gruppe bestehend aus Wasserstoff, -OH, -O·, unverzweigte oder verzweigte Alkylgruppe mit 1 bis 10 Kohlenstoffatomen, unverzweigte oder verzweigte Alkoxygruppe mit 1 bis 10 Kohlenstoffatomen,
      wobei in den chemischen Strukturen **(iii), (iv), (v), (vi), (vii), (viii), (ix)** mindestens ein an ein Kohlenstoffatom gebundener Wasserstoffrest durch einen Rest ausgewählt aus der Gruppe bestehend aus
      -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann;
   wobei die Reste R¹², R¹³, R¹⁴, R¹⁵, R¹⁶, R¹⁷ unabhängig voneinander jeweils ausgewählt sind aus der Gruppe bestehend aus
      Wasserstoff,
         unverzweigte oder verzweigte Alkylgruppe mit 1 bis 30 Kohlenstoffatomen,
         einer Gruppe mit der chemischen Struktur **(x)** mit 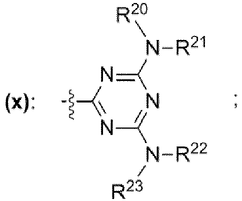
   wobei die Reste R¹⁸, R¹⁹, R²⁰, R²¹, R²², R²³ unabhängig voneinander jeweils ausgewählt sind aus der Gruppe bestehend aus
      Wasserstoff,
      unverzweigte oder verzweigte Alkylgruppe mit 1 bis 30 Kohlenstoffatomen, bei welcher mindestens ein Wasserstoffrest durch einen Rest ausgewählt aus der Gruppe bestehend aus -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann,
      unverzweigte oder verzweigte Acylgruppe mit 1 bis 30 Kohlenstoffatomen, bei welcher mindestens ein Wasserstoffrest durch einen Rest ausgewählt aus der Gruppe bestehend aus -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann,
      einem Rest, der eine chemischen Struktur ausgewählt aus der Gruppe bestehend aus **(xi), (xii), (xiii), (xiv), (xv), (xvi), (xvii)** mit 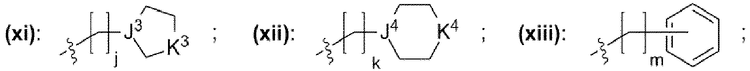 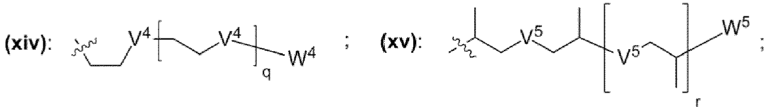 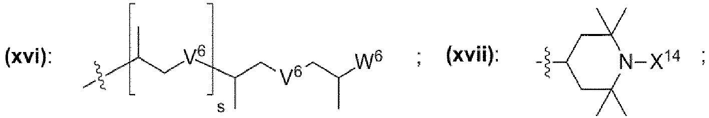
      aufweist,
         wobei J³, J⁴ unabhängig voneinander jeweils ausgewählt aus der Gruppe bestehend aus CH, N sind,
         wobei K³, K⁴ unabhängig voneinander jeweils ausgewählt aus der Gruppe bestehend aus -O-, -NH-, -N(CH₃)-, -N(CH₂CH₃)-, -S-, -CH₂- sind,
         wobei V⁴, V⁵, V⁶ unabhängig voneinander jeweils ausgewählt aus der Gruppe bestehend aus -O-, -S-, -NH-, -NR"'- mit R"' = unverzweigte oder verzweigte Alkylgruppe mit 1 bis 6 Kohlenstoffatomen sind,
         wobei W⁴, W⁵, W⁶ unabhängig voneinander jeweils ausgewählt aus der Gruppe bestehend aus H, Methyl, Ethyl sind,
         wobei j, k, m, q, r, s unabhängig voneinander jeweils eine ganze Zahl aus dem Bereich 0 bis 50 ist,
         wobei X¹⁴ ausgewählt ist aus der Gruppe bestehend aus Wasserstoff, -OH, -O·, unverzweigte oder verzweigte Alkylgruppe mit 1 bis 10 Kohlenstoffatomen, unverzweigte oder verzweigte Alkoxygruppe mit 1 bis 10 Kohlenstoffatomen,
         wobei in den chemischen Strukturen **(xi), (xii), (xiii), (xiv), (xv), (xvi), (xvii)** mindestens ein an ein Kohlenstoffatom gebundener Wasserstoffrest durch einen Rest ausgewählt aus der Gruppe bestehend aus -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann,
   und mit der Maßgabe, dass R¹² und R¹⁷ für p¹³ = p¹⁴ = p¹⁵ = p¹⁶ = 0 unabhängig voneinander jeweils auch eine Gruppe der chemischen Struktur **(xviii)** mit 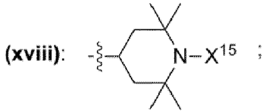
   sein können, wobei X¹⁵ ausgewählt ist aus der Gruppe bestehend aus Wasserstoff, OH, -O·, unverzweigte oder verzweigte Alkylgruppe mit 1 bis 10 Kohlenstoffatomen, unverzweigte oder verzweigte Alkoxygruppe mit 1 bis 10 Kohlenstoffatomen;
   und wobei die Carbonylverbindung **(II)** ausgewählt ist aus der Gruppe bestehend aus den chemischen Strukturen **(II-A)**, **(II-B)**, **(II-C)** mit 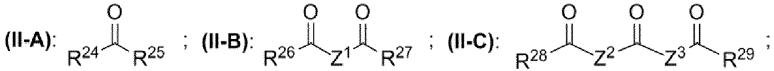
   wobei Z¹, Z², Z³ unabhängig voneinander jeweils ausgewählt sind aus der Gruppe bestehend aus
      direkte Bindung,
      unverzweigte oder verzweigte Alkylengruppe mit 1 bis 30 Kohlenstoffatomen,
      zweiwertige gesättigte Kohlenwasserstoffgruppe mit 3 bis 30 Kohlenstoffatomen, welche mindestens einen gesättigten Ring aus 3 bis 30 Kohlenstoffatomen aufweist;
   und wobei die Reste R²⁴, R²⁵, R²⁶, R²⁷, R²⁸, R²⁹ ausgewählt sind aus der Gruppe bestehend aus
      Wasserstoff,
      unverzweigte oder verzweigte Alkylgruppe mit 1 bis 30 Kohlenstoffatomen, bei welcher mindestens ein Wasserstoffrest durch einen Rest ausgewählt aus der Gruppe bestehend aus -OH, -OCH₃, -OCH₂CH₃, -N(CH₃)₂, -N(CH₂CH₃)₂, -N(CH₃)(CH₂CH₃) ersetzt sein kann,
      ein Rest, der eine chemischen Struktur ausgewählt aus der Gruppe bestehend aus **(xxi), (xxii), (xxiii), (xxiv), (xxv), (xxvi)** mit 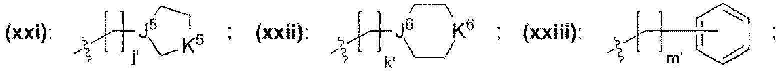 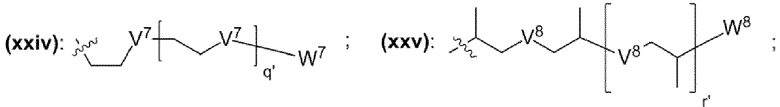 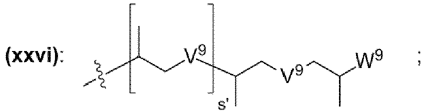
      aufweist,
         wobei J⁵, J⁶ unabhängig voneinander jeweils ausgewählt aus der Gruppe bestehend aus CH, N sind,
         wobei K⁵, K⁶ unabhängig voneinander jeweils ausgewählt aus der Gruppe bestehend aus -O-, -N(CH₃)-, -N(CH₂CH₃)-, -S-, -CH₂- sind,
         wobei V⁷, V⁸, V⁹ unabhängig voneinander jeweils ausgewählt aus der Gruppe bestehend aus -O-, -S-, -NR""'- mit R""' = unverzweigte oder verzweigte Alkylgruppe mit 1 bis 6 Kohlenstoffatomen sind,
         wobei W⁷, W⁸, W⁹ unabhängig voneinander jeweils ausgewählt aus der Gruppe bestehend aus H, Methyl, Ethyl sind,
         wobei j', k', m', q', r', s' unabhängig voneinander jeweils eine ganze Zahl ausgewählt aus dem Bereich 0 bis 50 ist,
         wobei in der chemischen Struktur **(xxi), (xxii), (xxiii), (xxiv), (xxv), (xxvi)** mindestens ein Wasserstoffrest durch einen Rest ausgewählt aus der Gruppe bestehend aus -OH, -OCH₃, -OCH₂CH₃, -N(CH₃)₂, -N(CH₂CH₃)₂, -N(CH₃)(CH₂CH₃) ersetzt sein kann;
   und wobei R²⁴, R²⁵, R²⁶, R²⁷, R²⁸, R²⁹ jeweils unabhängig voneinander ausgewählt sind, bis auf die Ausnahme, dass nicht gilt: R²⁴ = R²⁵ = Wasserstoff,
   und wobei man reduktive Bedingungen dadurch einstellt, dass man die mindestens eine Triacetondiaminverbindung **(I)** mit der mindestens einen Carbonylverbindung **(II)** in Gegenwart von Wasserstoff und in Gegenwart eines Vollkatalysators umsetzt, wobei der Vollkatalysator mindestens ein Metall **M** aufweist, wobei das Metall **M** ausgewählt ist aus der Gruppe bestehend aus Ag, V, Cr, Mo, Mn, Ni, Pd, Pt, Fe, Ru, Os, Co, Rh, Ir, Cu, insbesondere Ag, Fe, Ni, Co, Cu,, bevorzugt Ni, Co, bevorzugter Ni.
2.2 Verfahren nach Punkt 2.1, wobei Y¹, Y², Y³, Y⁴, Y⁵, Y⁶, Y⁷, Y⁸, Y⁹ unabhängig voneinander jeweils ausgewählt ist aus der Gruppe bestehend aus
   unverzweigte oder verzweigte Alkylengruppe mit 1 bis 12 Kohlenstoffatomen,
   zweiwertige gesättigte Kohlenwasserstoffgruppe mit 3 bis 12 Kohlenstoffatomen, welche mindestens einen gesättigten Ring aus 3 bis 12 Kohlenstoffatomen aufweist.
2.3 Verfahren nach Punkt 2.1 oder 2.2, wobei Z¹, Z², Z³ unabhängig voneinander jeweils ausgewählt sind aus der Gruppe bestehend aus
   direkte Bindung,
   unverzweigte oder verzweigte Alkylengruppe mit 1 bis 12 Kohlenstoffatomen,
   zweiwertige gesättigte Kohlenwasserstoffgruppe mit 3 bis 12 Kohlenstoffatomen, welche mindestens einen gesättigten Ring aus 3 bis 12 Kohlenstoffatomen aufweist.
2.4 Verfahren nach einem oder mehreren der Punkte 2.1 bis 2.3, wobei die Reste R²⁴, R²⁵, R²⁶, R²⁷, R²⁸, R²⁹ ausgewählt sind aus der Gruppe bestehend aus
   Wasserstoff,
   unverzweigte oder verzweigte Alkylgruppe mit 1 bis 30 Kohlenstoffatomen, bei welcher mindestens ein Wasserstoffrest durch einen Rest ausgewählt aus der Gruppe bestehend aus -OH, -OCH₃, -OCH₂CH₃, -N(CH₃)₂, -N(CH₂CH₃)₂, -N(CH₃)(CH₂CH₃) ersetzt sein kann;
   und wobei R²⁴, R²⁵, R²⁶, R²⁷, R²⁸, R²⁹ jeweils unabhängig voneinander ausgewählt sind, bis auf die Ausnahme, dass nicht gilt: R²⁴ = R²⁵ = Wasserstoff.
2.5 Verfahren nach einem oder mehreren der Punkte 2.1 bis 2.4, wobei die Reste R¹, R², R³, R⁴, R⁵, R⁶, R⁷, R⁸, R⁹, R¹⁰, R¹¹ unabhängig voneinander jeweils ausgewählt sind aus der Gruppe bestehend aus
   Wasserstoff,
   unverzweigte oder verzweigte Alkylgruppe mit 1 bis 12 Kohlenstoffatomen, bei welcher mindestens ein Wasserstoffrest durch einen Rest ausgewählt aus der Gruppe bestehend aus -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann,
   einem Rest, der eine chemischen Struktur **(ix)** mit 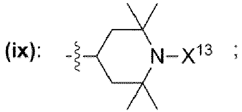
   aufweist,
   wobei X¹³ ausgewählt ist aus der Gruppe bestehend aus Wasserstoff, -OH, -O·, unverzweigte oder verzweigte Alkylgruppe mit 1 bis 10 Kohlenstoffatomen, unverzweigte oder verzweigte Alkoxygruppe mit 1 bis 10 Kohlenstoffatomen;
   und wobei die Reste R¹², R¹³, R¹⁴, R¹⁵, R¹⁶, R¹⁷ unabhängig voneinander jeweils ausgewählt sind aus der Gruppe bestehend aus
   Wasserstoff,
   unverzweigte oder verzweigte Alkylgruppe mit 1 bis 12 Kohlenstoffatomen,
   einer Gruppe mit der chemischen Struktur **(x)** mit 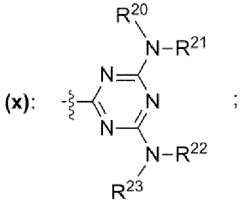
   wobei die Reste R¹⁸, R¹⁹, R²⁰, R²¹, R²², R²³ unabhängig voneinander jeweils ausgewählt sind aus der Gruppe bestehend aus
   Wasserstoff,
   unverzweigte oder verzweigte Alkylgruppe mit 1 bis 12 Kohlenstoffatomen, bei welcher mindestens ein Wasserstoffrest durch einen Rest ausgewählt aus der Gruppe bestehend aus -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann,
   einem Rest, der eine chemischen Struktur (xvii) mit 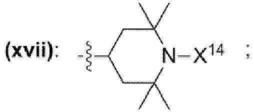
   aufweist,
      wobei X¹⁴ ausgewählt ist aus der Gruppe bestehend aus Wasserstoff, -OH, -O·, unverzweigte oder verzweigte Alkylgruppe mit 1 bis 10 Kohlenstoffatomen, unverzweigte oder verzweigte Alkoxygruppe mit 1 bis 10 Kohlenstoffatomen,
   und mit der Maßgabe, dass R¹² und R¹⁷ für p¹³ = p¹⁴ = p¹⁵ = p¹⁶ = 0 unabhängig voneinander jeweils auch eine Gruppe der chemischen Struktur **(xviii)** mit 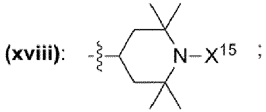 sein können, wobei X¹⁵ ausgewählt ist aus der Gruppe bestehend aus Wasserstoff, OH, -O·, unverzweigte oder verzweigte Alkylgruppe mit 1 bis 10 Kohlenstoffatomen, unverzweigte oder verzweigte Alkoxygruppe mit 1 bis 10 Kohlenstoffatomen.
2.6 Verfahren nach einem oder mehreren der Punkte 2.1 bis 2.5, wobei die Reste R²⁴, R²⁵, R²⁶, R²⁷, R²⁸, R²⁹ ausgewählt sind aus der Gruppe bestehend aus
   Wasserstoff,
   unverzweigte oder verzweigte Alkylgruppe mit 1 bis 12 Kohlenstoffatomen, bei welcher mindestens ein Wasserstoffrest durch einen Rest ausgewählt aus der Gruppe bestehend aus -OH, -OCH₃, -OCH₂CH₃, -N(CH₃)₂, -N(CH₂CH₃)₂, -N(CH₃)(CH₂CH₃) ersetzt sein kann;
   wobei R²⁴, R²⁵, R²⁶, R²⁷, R²⁸, R²⁹ jeweils unabhängig voneinander ausgewählt sind, bis auf die Ausnahme, dass nicht gilt: R²⁴ = R²⁵ = Wasserstoff.
2.7 Verfahren nach einem oder mehreren der Punkte 2.1 bis 2.6, wobei X¹ = X² = X³ = X⁴ = X⁵ = X⁶ = X⁷ = X⁸ = X⁹ = X¹⁰ = X¹¹ = X¹² = X¹³ = X¹⁴ = X¹⁵ = Wasserstoff ist.
2.8 Verfahren nach einem oder mehreren der Punkte 2.1 bis 2.7, wobei Y¹, Y², Y³, Y⁴, Y⁵, Y⁶, Y⁷, Y⁸, Y⁹ unabhängig voneinander jeweils ausgewählt ist aus der Gruppe bestehend aus unverzweigte oder verzweigte Alkylengruppe mit 1 bis 6 Kohlenstoffatomen,
   zweiwertige gesättigte Kohlenwasserstoffgruppe mit 3 bis 6 Kohlenstoffatomen, welche mindestens einen gesättigten Ring aus 3 bis 6 Kohlenstoffatomen aufweist.
2.9 Verfahren nach einem oder mehreren der Punkte 2.1 bis 2.8, wobei die Triacetondiaminverbindung **(I)** ausgewählt ist aus der Gruppe bestehend aus den chemischen Strukturen **(I-A)**, **(I-B)**
   und wobei die Reste R¹, R² unabhängig voneinander jeweils ausgewählt sind aus der Gruppe bestehend aus
   Wasserstoff,
   unverzweigte oder verzweigte Alkylgruppe mit 1 bis 12 Kohlenstoffatomen, bei welcher mindestens ein Wasserstoffrest durch einen Rest ausgewählt aus der Gruppe bestehend aus -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann.
2.10 Verfahren nach Punkt 2.9, wobei die Reste R¹, R² unabhängig voneinander jeweils ausgewählt sind aus der Gruppe bestehend aus
   Wasserstoff,
   unverzweigte oder verzweigte Alkylgruppe mit 1 bis 12 Kohlenstoffatomen.
2.11 Verfahren nach Punkt 2.10, wobei die Reste R¹, R² unabhängig voneinander jeweils ausgewählt sind aus der Gruppe bestehend aus
   Wasserstoff,
   unverzweigte oder verzweigte Alkylgruppe mit 1 bis 6 Kohlenstoffatomen.
2.12 Verfahren nach einem oder mehreren der Punkte 2.1 bis 2.11, wobei die Carbonylverbindung **(II)** die chemische Struktur **(II-A)** aufweist.
2.13 Verfahren nach Punkt 2.12, wobei die Reste R²⁴, R²⁵ ausgewählt sind aus
   Wasserstoff, unverzweigte oder verzweigte Alkylgruppe mit 1 bis 12 Kohlenstoffatomen,
   und wobei R²⁴, R²⁵ jeweils unabhängig voneinander ausgewählt sind, bis auf die Ausnahme, dass nicht gilt: R²⁴ = R²⁵ = Wasserstoff.
2.14 Verfahren nach Punkt 2.13, wobei die Reste R²⁴, R²⁵ ausgewählt sind aus
   Wasserstoff,
   unverzweigte oder verzweigte Alkylgruppe mit 1 bis 6 Kohlenstoffatomen,
   und wobei R²⁴, R²⁵ jeweils unabhängig voneinander ausgewählt sind, bis auf die Ausnahme, dass nicht gilt: R²⁴ = R²⁵ = Wasserstoff.
2.15 Verfahren nach einem oder mehreren der Punkte 2.1 bis 2.14, wobei die Triacetondiaminverbindung **(I)** die chemische Struktur **(I-A)** aufweist
   und wobei der Rest R¹ ausgewählt ist aus der Gruppe bestehend aus
   Wasserstoff,
   unverzweigte oder verzweigte Alkylgruppe mit 1 bis 6 Kohlenstoffatomen.
2.16 Verfahren nach einem oder mehreren der Punkte 2.1 bis 2.15, wobei der Rest R²⁴ ausgewählt ist aus der Gruppe bestehend aus Wasserstoff, Methyl, Ethyl und wobei der Rest R²⁵ ausgewählt ist aus der Gruppe bestehend aus Methyl, Ethyl, n-Propyl, *iso*-Propyl, *n*-Butyl, *iso*-Butyl, *sec*-Butyl, n-Pentyl, n-Hexyl, n-Heptyl, n-Octyl.
2.17 Verfahren nach Punkt 2.16, wobei der Rest R²⁴ ausgewählt aus der Gruppe bestehend aus Wasserstoff, Methyl ist, und wobei der Rest R²⁵ ausgewählt aus der Gruppe bestehend aus Methyl, Ethyl, n-Propyl, *iso*-Propyl, *n*-Butyl, *iso*-Butyl, *sec*-Butyl, n-Pentyl, n-Hexyl, n-Heptyl, n-Octyl; wobei noch bevorzugter der Rest Rest R²⁴ = Wasserstoff und der Rest R²⁵ ausgewählt aus der Gruppe bestehend aus Methyl, Ethyl, n-Propyl, *iso*-Propyl, *n*-Butyl, *iso*-Butyl, *sec*-Butyl, n-Pentyl, n-Hexyl, n-Heptyl, n-Octyl ist.
2.18 Verfahren nach Punkt 2.16, wobei der Rest R²⁴ = Methyl ist, und wobei der Rest R²⁵ ausgewählt aus der Gruppe bestehend aus Methyl, Ethyl, n-Propyl, *iso*-Propyl, *n*-Butyl, *iso*-Butyl, *sec*-Butyl, n-Pentyl, n-Hexyl, n-Heptyl, n-Octyl.
2.19 Verfahren nach einem oder mehreren der Punkte 2.1 bis 2.18 wobei man es in mindestens einem Lösungsmittel durchführt, wobei das Lösungsmittel ausgewählt ist aus der Gruppe bestehend aus aliphatische Lösungsmittel, aromatische Lösungsmittel, Ether, halogenierte Lösungsmittel, Amide, Thioverbindungen, Carbonsäuren, Alkohole, Wasser.
2.20 Verfahren nach einem oder mehreren der Punkte 2.1 bis 2.19, wobei man es bei einer Temperatur im Bereich von 20 °C bis 350 °C und einem Druck im Bereich von 2 bar bis 500 bar durchführt.

Die vorliegende Erfindung betrifft in einem dritten Aspekt ein Verfahren gemäß den folgenden Punkten 3.1 bis 3.18.
3.1 Verfahren zur Herstellung einer *N*-substituierten Triacetondiaminverbindung,
   dadurch gekennzeichnet, dass man mindestens eine Triacetondiaminverbindung **(I)** mit mindestens einer Carbonylverbindung **(II)** unter reduktiven Bedingungen umsetzt,
   wobei die Triacetondiaminverbindung **(I)** die chemische Struktur **(I-A)** aufweist mit 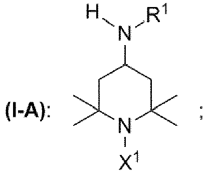
   wobei X¹ ausgewählt ist aus der Gruppe bestehend aus Wasserstoff, OH, -O·, unverzweigte oder verzweigte Alkylgruppe mit 1 bis 10 Kohlenstoffatomen, unverzweigte oder verzweigte Alkoxygruppe mit 1 bis 10 Kohlenstoffatomen;
   wobei der Rest R¹ ausgewählt ist aus der Gruppe bestehend aus
      Wasserstoff,
      unverzweigte oder verzweigte Alkylgruppe mit 1 bis 30 Kohlenstoffatomen, bei welcher mindestens ein Wasserstoffrest durch einen Rest ausgewählt aus der Gruppe bestehend aus -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann,
      unverzweigte oder verzweigte Acylgruppe mit 1 bis 30 Kohlenstoffatomen, bei welcher mindestens ein Wasserstoffrest durch einen Rest ausgewählt aus der Gruppe bestehend aus -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann,
      einem Rest, der eine chemischen Struktur ausgewählt aus der Gruppe bestehend aus **(iii), (iv), (v), (vi), (vii), (viii), (ix)** mit 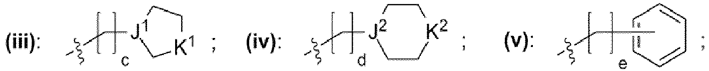 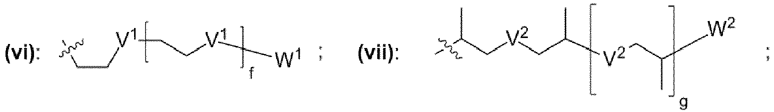 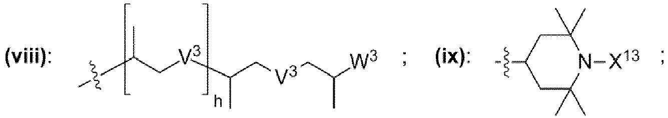
      aufweist,
         wobei J¹, J² unabhängig voneinander jeweils ausgewählt aus der Gruppe bestehend aus CH, N sind,
         wobei K¹, K² unabhängig voneinander jeweils ausgewählt aus der Gruppe bestehend aus -O-, -NH-, -N(CH₃)-, -N(CH₂CH₃)-, -S-, -CH₂- sind,
         wobei V¹, V², V³ unabhängig voneinander jeweils ausgewählt aus der Gruppe bestehend aus -O-, -S-, -NH-, -NR"- mit R" = unverzweigte oder verzweigte Alkylgruppe mit 1 bis 6 Kohlenstoffatomen sind,
         wobei W¹, W², W³ unabhängig voneinander jeweils ausgewählt aus der Gruppe bestehend aus H, Methyl, Ethyl sind,
         wobei c, d, e, f, g, h unabhängig voneinander jeweils eine ganze Zahl aus dem Bereich 0 bis 50 ist,
         wobei X¹³ ausgewählt ist aus der Gruppe bestehend aus Wasserstoff, -OH, -O·, unverzweigte oder verzweigte Alkylgruppe mit 1 bis 10 Kohlenstoffatomen, unverzweigte oder verzweigte Alkoxygruppe mit 1 bis 10 Kohlenstoffatomen,
      wobei in den chemischen Strukturen **(iii), (iv), (v), (vi), (vii), (viii), (ix)** mindestens ein an ein Kohlenstoffatom gebundener Wasserstoffrest durch einen Rest ausgewählt aus der Gruppe bestehend aus
      -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann;
      und wobei die Carbonylverbindung **(II)** ausgewählt ist aus der Gruppe bestehend aus den chemischen Strukturen **(II-A)**, **(II-B)** mit 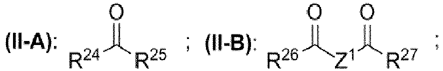
   wobei Z¹ ausgewählt ist aus der Gruppe bestehend aus
      direkte Bindung,
      unverzweigte oder verzweigte Alkylengruppe mit 1 bis 30 Kohlenstoffatomen,
      zweiwertige gesättigte Kohlenwasserstoffgruppe mit 3 bis 30 Kohlenstoffatomen, welche mindestens einen gesättigten Ring aus 3 bis 30 Kohlenstoffatomen aufweist,
      zweiwertige Kohlenwasserstoffgruppe mit 6 bis 30 Kohlenstoffatomen, von denen mindestens 6 Kohlenstoffatome in einem aromatischen System vorliegen und die übrigen Kohlenstoffatome, falls vorhanden, gesättigt sind,
      ein verbrückender Rest, der eine chemischen Struktur ausgewählt aus der Gruppe bestehend aus **(xix), (xx)** mit 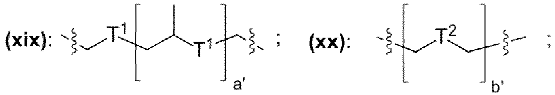
      aufweist, wobei
         T¹, T² unabhängig voneinander jeweils ausgewählt aus der Gruppe bestehend aus -O-, -S- oder -NR""- mit R"" = unverzweigte oder verzweigte Alkylgruppe mit 1 bis 6 Kohlenstoffatomen sind,
         wobei a' und b' unabhängig voneinander jeweils eine ganze Zahl ausgewählt aus dem Bereich 1 bis 50 ist;
   und wobei die Reste R²⁴, R²⁵, R²⁶, R²⁷ ausgewählt sind aus der Gruppe bestehend aus
      Wasserstoff,
      unverzweigte oder verzweigte Alkylgruppe mit 1 bis 30 Kohlenstoffatomen, bei welcher mindestens ein Wasserstoffrest durch einen Rest ausgewählt aus der Gruppe bestehend aus -OH, -OCH₃, -OCH₂CH₃, -N(CH₃)₂, -N(CH₂CH₃)₂, -N(CH₃)(CH₂CH₃) ersetzt sein kann,
      ein Rest, der eine chemischen Struktur ausgewählt aus der Gruppe bestehend aus **(xxi), (xxii), (xxiii), (xxiv), (xxv), (xxvi)** mit 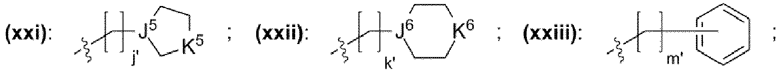 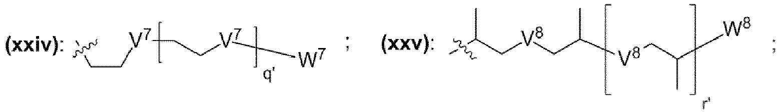 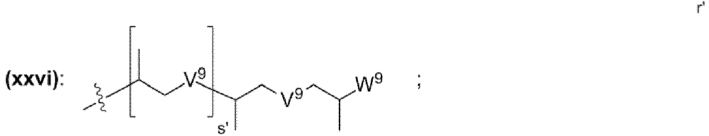
      aufweist,
         wobei J⁵, J⁶ unabhängig voneinander jeweils ausgewählt aus der Gruppe bestehend aus CH, N sind,
         wobei K⁵, K⁶ unabhängig voneinander jeweils ausgewählt aus der Gruppe bestehend aus -O-, -N(CH₃)-, -N(CH₂CH₃)-, -S-, -CH₂- sind,
         wobei V⁷, V⁸, V⁹ unabhängig voneinander jeweils ausgewählt aus der Gruppe bestehend aus -O-, -S-, -NR'""- mit R'"" = unverzweigte oder verzweigte Alkylgruppe mit 1 bis 6 Kohlenstoffatomen sind,
         wobei W⁷, W⁸, W⁹ unabhängig voneinander jeweils ausgewählt aus der Gruppe bestehend aus H, Methyl, Ethyl sind,
         wobei j', k', m', q', r', s' unabhängig voneinander jeweils eine ganze Zahl ausgewählt aus dem Bereich 0 bis 50 ist,
         wobei in der chemischen Struktur **(xxi), (xxii), (xxiii), (xxiv), (xxv), (xxvi)** mindestens ein Wasserstoffrest durch einen Rest ausgewählt aus der Gruppe bestehend aus -OH, -OCH₃, -OCH₂CH₃, -N(CH₃)₂, -N(CH₂CH₃)₂, -N(CH₃)(CH₂CH₃) ersetzt sein kann;
   und wobei R²⁴, R²⁵, R²⁶, R²⁷ jeweils unabhängig voneinander ausgewählt sind, bis auf die Ausnahme, dass nicht gilt: R²⁴ = R²⁵ = Wasserstoff,
   und wobei man reduktive Bedingungen dadurch einstellt, dass man die mindestens eine Triacetondiaminverbindung **(I)** mit der mindestens einen Carbonylverbindung **(II)** in Gegenwart von Wasserstoff und in Gegenwart eines Vollkatalysators umsetzt, wobei der Vollkatalysator mindestens ein Metall **M** aufweist, wobei das Metall **M** ausgewählt ist aus der Gruppe bestehend aus Ag, V, Cr, Mo, Mn, Ni, Pd, Pt, Fe, Ru, Os, Co, Rh, Ir, Cu, insbesondere Ag, Fe, Ni, Co, Cu, bevorzugt Ni, Co, bevorzugter Ni.
3.2 Verfahren nach Punkt 3.1, wobei der Rest R¹ ausgewählt ist aus der Gruppe bestehend aus
   Wasserstoff,
   unverzweigte oder verzweigte Alkylgruppe mit 1 bis 30 Kohlenstoffatomen, bei welcher mindestens ein Wasserstoffrest durch einen Rest ausgewählt aus der Gruppe bestehend aus -OH, -NH₂, -OCH₃, -OCH₂CH₃, -NH(CH₃), -N(CH₃)₂, -NH(CH₂CH₃), -N(CH₂CH₃)₂, - N(CH₃)(CH₂CH₃) ersetzt sein kann,
   einem Rest, der eine chemischen Struktur **(ix)** mit 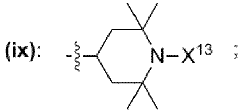
   aufweist,
   wobei X¹³ ausgewählt ist aus der Gruppe bestehend aus Wasserstoff, -OH, -O·, unverzweigte oder verzweigte Alkylgruppe mit 1 bis 10 Kohlenstoffatomen, unverzweigte oder verzweigte Alkoxygruppe mit 1 bis 10 Kohlenstoffatomen.
3.3 Verfahren nach Punkt 3.1 oder 3.2, wobei X¹ = X¹³ = Wasserstoff ist.
3.4 Verfahren nach einem oder mehreren der Punkte 3.1 bis 3.3, wobei der Rest R¹ ausgewählt ist aus der Gruppe bestehend aus
   Wasserstoff,
   unverzweigte oder verzweigte Alkylgruppe mit 1 bis 12 Kohlenstoffatomen, bei welcher mindestens ein Wasserstoffrest durch einen Rest ausgewählt aus der Gruppe bestehend aus -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann.
3.5 Verfahren nach einem oder mehreren der Punkte 3.1 bis 3.4, wobei der Rest R¹ ausgewählt ist aus der Gruppe bestehend aus
   Wasserstoff,
   unverzweigte oder verzweigte Alkylgruppe mit 1 bis 12 Kohlenstoffatomen.
3.6 Verfahren nach einem oder mehreren der Punkte 3.1 bis 3.5, wobei Z¹ ausgewählt ist aus der Gruppe bestehend aus
   direkte Bindung,
   unverzweigte oder verzweigte Alkylengruppe mit 1 bis 30 Kohlenstoffatomen,
   zweiwertige gesättigte Kohlenwasserstoffgruppe mit 3 bis 30 Kohlenstoffatomen, welche mindestens einen gesättigten Ring aus 3 bis 30 Kohlenstoffatomen aufweist.
3.7 Verfahren nach Punkt 3.6, wobei Z¹ ausgewählt ist aus der Gruppe bestehend aus
   direkte Bindung,
   unverzweigte oder verzweigte Alkylengruppe mit 1 bis 12, noch bevorzugter 1 bis 6, Kohlenstoffatomen.
3.8 Verfahren nach einem oder mehreren der Punkte 3.1 bis 3.7, wobei die Carbonylverbindung **(II)** die chemische Struktur **(II-A)** aufweist.
3.9 Verfahren nach Punkt 3.8, wobei
   X¹ = X¹³ = Wasserstoff;
   R¹ ausgewählt ist aus der Gruppe bestehend aus
   Wasserstoff,
   unverzweigte oder verzweigte Alkylgruppe mit 1 bis 12 Kohlenstoffatomen;
   und R²⁴, R²⁵ ausgewählt sind aus der Gruppe bestehend aus
   Wasserstoff,
   unverzweigte oder verzweigte Alkylgruppe mit 1 bis 12 Kohlenstoffatomen;
   und wobei R²⁴, R²⁵ jeweils unabhängig voneinander ausgewählt sind, bis auf die Ausnahme, dass nicht gilt: R²⁴ = R²⁵ = Wasserstoff.
3.10 Verfahren nach Punkt 3.9, wobei
   X¹ = X¹³ = Wasserstoff;
   R¹ ausgewählt ist aus der Gruppe bestehend aus
   Wasserstoff,
   unverzweigte oder verzweigte Alkylgruppe mit 1 bis 8 Kohlenstoffatomen;
   und R²⁴, R²⁵ ausgewählt sind aus der Gruppe bestehend aus
   Wasserstoff,
   unverzweigte oder verzweigte Alkylgruppe mit 1 bis 8 Kohlenstoffatomen;
   und wobei R²⁴, R²⁵ jeweils unabhängig voneinander ausgewählt sind, bis auf die Ausnahme, dass nicht gilt: R²⁴ = R²⁵ = Wasserstoff.
3.11 Verfahren nach Punkt 3.10, wobei
   X¹ = X¹³ = Wasserstoff;
   R¹ = Wasserstoff;
   R²⁴ ausgewählt ist aus der Gruppe bestehend aus Wasserstoff, Methyl;
   R²⁵ = unverzweigte oder verzweigte Alkylgruppe mit 1 bis 8 Kohlenstoffatomen
3.12 Verfahren nach einem oder mehreren der Punkte 3.8 bis 3.11, wobei man 0.8 bis 4.0, insbesondere 0.9 bis 3.0, bevorzugt 1.2 bis 2.6, bevorzugter 1.3 bis 2.4, noch bevorzugter 1.5 bis 2.0, am bevorzugtesten 1.6 bis 1.8 molare Äquivalente der Carbonylverbindung **(II)** der chemischen Struktur **(II-A)** pro Triacetondiaminverbindung **(I)** der chemischen Struktur **(I-A)** einsetzt.
3.13 Verfahren nach einem oder mehreren der Punkte 3.8 bis 3.12, wobei man es in mindestens einem Lösungsmittel durchführt, wobei das Lösungsmittel ausgewählt ist aus der Gruppe bestehend aus aliphatische Lösungsmittel, aromatische Lösungsmittel, Ether, halogenierte Lösungsmittel, Amide, Thioverbindungen, Carbonsäuren, Alkohole, Wasser.
3.14 Verfahren nach einem oder mehreren der Punkte 3.8 bis 3.13, wobei man es bei einer Temperatur im Bereich von 20 °C bis 350 °C und einem Druck im Bereich von 2 bar bis 500 bar durchführt.
3.15 Verfahren nach einem oder mehreren der Punkte 3.1 bis 3.7, wobei die Carbonylverbindung **(II)** die chemische Struktur **(II-B)** aufweist.
3.16 Verfahren nach Punkt 3.15, wobei man 0.4 bis 2.0, insbesondere 0.45 bis 1.5, bevorzugt 0.6 bis 1.3, bevorzugter 0.65 bis 1.2, noch bevorzugter 0.75 bis 1.0, am bevorzugtesten 0.8 bis 0.9 molare Äquivalente der Carbonylverbindung **(II)** der chemischen Struktur **(II-B)** pro Triacetondiaminverbindung **(I)** der chemischen Struktur **(I-A)** einsetzt.
3.17 Verfahren nach einem oder mehreren der Punkte 3.15 bis 3.16, wobei man es in mindestens einem Lösungsmittel durchführt, wobei das Lösungsmittel ausgewählt ist aus der Gruppe bestehend aus aliphatische Lösungsmittel, aromatische Lösungsmittel, Ether, halogenierte Lösungsmittel, Amide, Thioverbindungen, Carbonsäuren, Alkohole, Wasser.
3.18 Verfahren nach einem oder mehreren der Punkte 3.15 bis 3.17, wobei man es bei einer Temperatur im Bereich von 20 °C bis 350 °C und einem Druck im Bereich von 2 bar bis 500 bar durchführt.

Die vorliegende Erfindung betrifft in einem vierten Aspekt ein Verfahren gemäß den folgenden Punkten 4.1 bis 4.20.
4.1 Verfahren zur Herstellung einer *N*-substituierten Triacetondiaminverbindung,
   dadurch gekennzeichnet, dass man mindestens eine Triacetondiaminverbindung **(I)** mit mindestens einer Carbonylverbindung **(II)** unter reduktiven Bedingungen umsetzt,
   wobei die Triacetondiaminverbindung **(I)** die chemische Struktur **(I-B)** aufweist mit 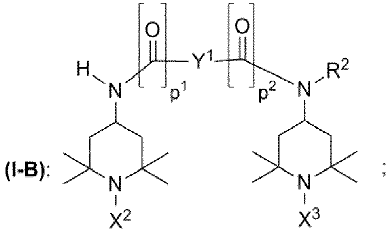
   wobei p¹, p² unabhängig voneinander jeweils 0 oder 1 ist;
   wobei X², X³ unabhängig voneinander jeweils ausgewählt ist aus der Gruppe bestehend aus Wasserstoff, OH, -O·, unverzweigte oder verzweigte Alkylgruppe mit 1 bis 10 Kohlenstoffatomen, unverzweigte oder verzweigte Alkoxygruppe mit 1 bis 10 Kohlenstoffatomen;
   wobei Y¹ ausgewählt ist aus der Gruppe bestehend aus
      unverzweigte oder verzweigte Alkylengruppe mit 1 bis 30 Kohlenstoffatomen,
      zweiwertige gesättigte Kohlenwasserstoffgruppe mit 3 bis 30 Kohlenstoffatomen, welche mindestens einen gesättigten Ring aus 3 bis 30 Kohlenstoffatomen aufweist,
      zweiwertige Kohlenwasserstoffgruppe mit 6 bis 30 Kohlenstoffatomen, von denen mindestens 6 Kohlenstoffatome in einem aromatischen System vorliegen und die übrigen Kohlenstoffatome, falls vorhanden, gesättigt sind,
      ein verbrückender Rest, der eine chemische Struktur ausgewählt aus der Gruppe bestehend aus **(i), (ii)** mit 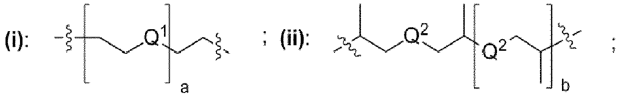
      aufweist, wobei
         Q¹, Q² unabhängig voneinander jeweils ausgewählt aus der Gruppe bestehend aus -O-, -S-, -NH- oder -NR'- mit R' = unverzweigte oder verzweigte Alkylgruppe mit 1 bis 6 Kohlenstoffatomen sind,
         wobei a eine ganze Zahl ausgewählt aus dem Bereich 1 bis 50 ist,
         wobei b eine ganze Zahl ausgewählt aus dem Bereich 0 bis 50 ist,
   und wobei Y¹ auch eine direkte Bindung sein kann, falls mindestens einer von p¹ und p² den Wert 1 aufweist,
   wobei der Rest R² ausgewählt ist aus der Gruppe bestehend aus
      Wasserstoff,
      unverzweigte oder verzweigte Alkylgruppe mit 1 bis 30 Kohlenstoffatomen, bei welcher mindestens ein Wasserstoffrest durch einen Rest ausgewählt aus der Gruppe bestehend aus -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann,
      unverzweigte oder verzweigte Acylgruppe mit 1 bis 30 Kohlenstoffatomen, bei welcher mindestens ein Wasserstoffrest durch einen Rest ausgewählt aus der Gruppe bestehend aus -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann,
      einem Rest, der eine chemischen Struktur ausgewählt aus der Gruppe bestehend aus **(iii), (iv), (v), (vi), (vii), (viii), (ix)** mit 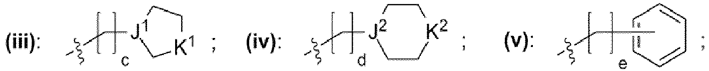 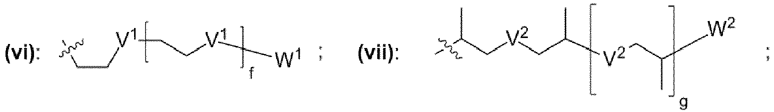 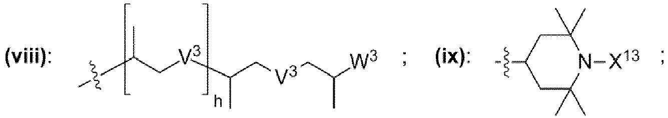
      aufweist,
         wobei J¹, J² unabhängig voneinander jeweils ausgewählt aus der Gruppe bestehend aus CH, N sind,
         wobei K¹, K² unabhängig voneinander jeweils ausgewählt aus der Gruppe bestehend aus -O-, -NH-, -N(CH₃)-, -N(CH₂CH₃)-, -S-, -CH₂- sind,
         wobei V¹, V², V³ unabhängig voneinander jeweils ausgewählt aus der Gruppe bestehend aus -O-, -S-, -NH-, -NR"- mit R" = unverzweigte oder verzweigte Alkylgruppe mit 1 bis 6 Kohlenstoffatomen sind,
         wobei W¹, W², W³ unabhängig voneinander jeweils ausgewählt aus der Gruppe bestehend aus H, Methyl, Ethyl sind,
         wobei c, d, e, f, g, h unabhängig voneinander jeweils eine ganze Zahl aus dem Bereich 0 bis 50 ist,
         wobei X¹³ ausgewählt ist aus der Gruppe bestehend aus Wasserstoff, -OH, -O·, unverzweigte oder verzweigte Alkylgruppe mit 1 bis 10 Kohlenstoffatomen, unverzweigte oder verzweigte Alkoxygruppe mit 1 bis 10 Kohlenstoffatomen,
      wobei in den chemischen Strukturen **(iii), (iv), (v), (vi), (vii), (viii), (ix)** mindestens ein an ein Kohlenstoffatom gebundener Wasserstoffrest durch einen Rest ausgewählt aus der Gruppe bestehend aus
      -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann;
   und wobei die Carbonylverbindung **(II)** ausgewählt ist aus der Gruppe bestehend aus den chemischen Strukturen **(II-A), (II-B)** mit 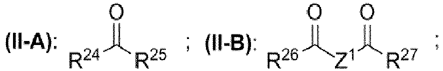
   wobei Z¹ ausgewählt ist aus der Gruppe bestehend aus
      direkte Bindung,
      unverzweigte oder verzweigte Alkylengruppe mit 1 bis 30 Kohlenstoffatomen,
      zweiwertige gesättigte Kohlenwasserstoffgruppe mit 3 bis 30 Kohlenstoffatomen, welche mindestens einen gesättigten Ring aus 3 bis 30 Kohlenstoffatomen aufweist,
      zweiwertige Kohlenwasserstoffgruppe mit 6 bis 30 Kohlenstoffatomen, von denen mindestens 6 Kohlenstoffatome in einem aromatischen System vorliegen und die übrigen Kohlenstoffatome, falls vorhanden, gesättigt sind,
      ein verbrückender Rest, der eine chemischen Struktur ausgewählt aus der Gruppe bestehend aus **(xix), (xx)** mit 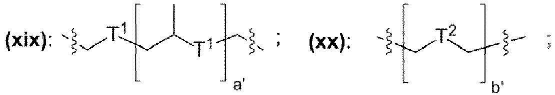
      aufweist, wobei
         T¹, T² unabhängig voneinander jeweils ausgewählt aus der Gruppe bestehend aus -O-, -S- oder -NR""- mit R"" = unverzweigte oder verzweigte Alkylgruppe mit 1 bis 6 Kohlenstoffatomen sind,
         wobei a' und b' unabhängig voneinander jeweils eine ganze Zahl ausgewählt aus dem Bereich 1 bis 50 ist;
   und wobei die Reste R²⁴, R²⁵, R²⁶, R²⁷ ausgewählt sind aus der Gruppe bestehend aus
      Wasserstoff,
      unverzweigte oder verzweigte Alkylgruppe mit 1 bis 30 Kohlenstoffatomen, bei welcher mindestens ein Wasserstoffrest durch einen Rest ausgewählt aus der Gruppe bestehend aus -OH, -OCH₃, -OCH₂CH₃, -N(CH₃)₂, -N(CH₂CH₃)₂, -N(CH₃)(CH₂CH₃) ersetzt sein kann,
      ein Rest, der eine chemischen Struktur ausgewählt aus der Gruppe bestehend aus **(xxi), (xxii), (xxiii), (xxiv), (xxv), (xxvi)** mit 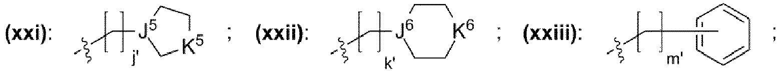 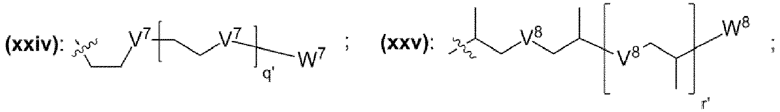 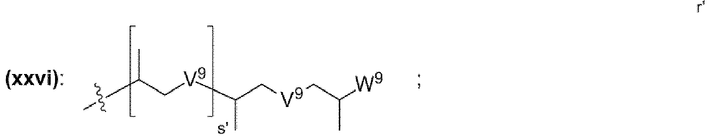
      aufweist,
         wobei J⁵, J⁶ unabhängig voneinander jeweils ausgewählt aus der Gruppe bestehend aus CH, N sind,
         wobei K⁵, K⁶ unabhängig voneinander jeweils ausgewählt aus der Gruppe bestehend aus -O-, -N(CH₃)-, -N(CH₂CH₃)-, -S-, -CH₂- sind,
         wobei V⁷, V⁸, V⁹ unabhängig voneinander jeweils ausgewählt aus der Gruppe bestehend aus -O-, -S-, -NR'""- mit R'"" = unverzweigte oder verzweigte Alkylgruppe mit 1 bis 6 Kohlenstoffatomen sind,
         wobei W⁷, W⁸, W⁹ unabhängig voneinander jeweils ausgewählt aus der Gruppe bestehend aus H, Methyl, Ethyl sind,
         wobei j', k', m', q', r', s' unabhängig voneinander jeweils eine ganze Zahl ausgewählt aus dem Bereich 0 bis 50 ist,
         wobei in der chemischen Struktur **(xxi), (xxii), (xxiii), (xxiv), (xxv), (xxvi)** mindestens ein Wasserstoffrest durch einen Rest ausgewählt aus der Gruppe bestehend aus -OH, -OCH₃, -OCH₂CH₃, -N(CH₃)₂, -N(CH₂CH₃)₂, -N(CH₃)(CH₂CH₃) ersetzt sein kann;
   und wobei R²⁴, R²⁵, R²⁶, R²⁷ jeweils unabhängig voneinander ausgewählt sind, bis auf die Ausnahme, dass nicht gilt: R²⁴ = R²⁵ = Wasserstoff,
   und wobei man reduktive Bedingungen dadurch einstellt, dass man die mindestens eine Triacetondiaminverbindung **(I)** mit der mindestens einen Carbonylverbindung **(II)** in Gegenwart von Wasserstoff und in Gegenwart eines Vollkatalysators umsetzt, wobei der Vollkatalysator mindestens ein Metall **M** aufweist, wobei das Metall **M** ausgewählt ist aus der Gruppe bestehend aus Ag, V, Cr, Mo, Mn, Ni, Pd, Pt, Fe, Ru, Os, Co, Rh, Ir, Cu, insbesondere Ag, Fe, Ni, Co, Cu, bevorzugt Ni, Co, bevorzugter Ni.
4.2 Verfahren nach Punkt 4.1, wobei p¹ = p² = 0 ist.
4.3 Verfahren nach Punkt 4.1 oder 4.2, wobei Y¹ ausgewählt ist aus der Gruppe bestehend aus
   unverzweigte oder verzweigte Alkylengruppe mit 1 bis 30 Kohlenstoffatomen,
   zweiwertige gesättigte Kohlenwasserstoffgruppe mit 3 bis 30 Kohlenstoffatomen, welche mindestens einen gesättigten Ring aus 3 bis 30 Kohlenstoffatomen aufweist;
      wobei Y¹ bevorzugt eine unverzweigte oder verzweigte Alkylengruppe mit 1 bis 12, noch bevorzugter mit 1 bis 6, Kohlenstoffatomen ist.
4.4 Verfahren nach einem oder mehreren der Punkte 4.1 bis 4.3, wobei der Rest R² ausgewählt ist aus der Gruppe bestehend aus
   Wasserstoff,
   unverzweigte oder verzweigte Alkylgruppe mit 1 bis 30 Kohlenstoffatomen, bei welcher mindestens ein Wasserstoffrest durch einen Rest ausgewählt aus der Gruppe bestehend aus -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann,
   einem Rest, der eine chemischen Struktur (ix) mit 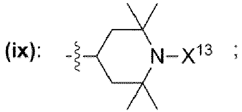
   aufweist,
      wobei X¹³ ausgewählt ist aus der Gruppe bestehend aus Wasserstoff, -OH, -O·, unverzweigte oder verzweigte Alkylgruppe mit 1 bis 10 Kohlenstoffatomen, unverzweigte oder verzweigte Alkoxygruppe mit 1 bis 10 Kohlenstoffatomen.
4.5 Verfahren nach einem oder mehreren der Punkte 4.1 bis 4.4, wobei X² = X³ = X¹³ = Wasserstoff ist.
4.6 Verfahren nach einem oder mehreren der Punkte 4.1 bis 4.5, wobei der Rest R² ausgewählt ist aus der Gruppe bestehend aus
   Wasserstoff,
   unverzweigte oder verzweigte Alkylgruppe mit 1 bis 12 Kohlenstoffatomen, bei welcher mindestens ein Wasserstoffrest durch einen Rest ausgewählt aus der Gruppe bestehend aus -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann.
4.7 Verfahren nach Punkt 4.6, wobei der Rest R² ausgewählt ist aus der Gruppe bestehend aus
   Wasserstoff,
   unverzweigte oder verzweigte Alkylgruppe mit 1 bis 12 Kohlenstoffatomen.
4.8 Verfahren nach einem oder mehreren der Punkte 4.1 bis 4.7, wobei Z¹ ausgewählt ist aus der Gruppe bestehend aus
   direkte Bindung,
   unverzweigte oder verzweigte Alkylengruppe mit 1 bis 30 Kohlenstoffatomen,
   zweiwertige gesättigte Kohlenwasserstoffgruppe mit 3 bis 30 Kohlenstoffatomen, welche mindestens einen gesättigten Ring aus 3 bis 30 Kohlenstoffatomen aufweist.
4.9 Verfahren nach Punkt 4.8, wobei Z¹ ausgewählt ist aus der Gruppe bestehend aus
   direkte Bindung,
   unverzweigte oder verzweigte Alkylengruppe mit 1 bis 12, noch bevorzugter 1 bis 6, Kohlenstoffatomen.
4.10 Verfahren nach einem oder mehreren der Punkte 4.1 bis 4.9, wobei die Carbonylverbindung **(II)** die chemische Struktur **(II-A)** aufweist.
4.11 Verfahren nach Punkt 4.10, wobei
   X² = X³ = X¹³ = Wasserstoff;
   R² ausgewählt ist aus der Gruppe bestehend aus
   Wasserstoff,
   unverzweigte oder verzweigte Alkylgruppe mit 1 bis 12 Kohlenstoffatomen;
   und R²⁴, R²⁵ ausgewählt sind aus der Gruppe bestehend aus
   Wasserstoff,
   unverzweigte oder verzweigte Alkylgruppe mit 1 bis 12 Kohlenstoffatomen;
   und wobei R²⁴, R²⁵ jeweils unabhängig voneinander ausgewählt sind, bis auf die Ausnahme, dass nicht gilt: R²⁴ = R²⁵ = Wasserstoff.
4.12 Verfahren nach Punkt 4.11, wobei
   X² = X³ = X¹³ = Wasserstoff;
   R² ausgewählt ist aus der Gruppe bestehend aus
   Wasserstoff,
   unverzweigte oder verzweigte Alkylgruppe mit 1 bis 8 Kohlenstoffatomen;
   und R²⁴, R²⁵ ausgewählt sind aus der Gruppe bestehend aus
   Wasserstoff,
   unverzweigte oder verzweigte Alkylgruppe mit 1 bis 8 Kohlenstoffatomen;
   und wobei R²⁴, R²⁵ jeweils unabhängig voneinander ausgewählt sind, bis auf die Ausnahme, dass nicht gilt: R²⁴ = R²⁵ = Wasserstoff.
4.13 Verfahren nach Punkt 4.12, wobei
   X² = X³ = X¹³ = Wasserstoff;
   R²= Wasserstoff;
   und R²⁴ ausgewählt ist aus der Gruppe bestehend aus Wasserstoff, Methyl;
   R²⁵ eine unverzweigte oder verzweigte Alkylgruppe mit 1 bis 8 Kohlenstoffatomen ist.
4.14 Verfahren nach einem oder mehreren der Punkte 4.10 bis 4.13, wobei man 0.8 bis 4.0, insbesondere 0.9 bis 3.0, bevorzugt 1.2 bis 2.6, bevorzugter 1.3 bis 2.4, noch bevorzugter 1.5 bis 2.0, am bevorzugtesten 1.6 bis 1.8 molare Äquivalente der Carbonylverbindung **(II)** der chemischen Struktur **(II-A)** pro Triacetondiaminverbindung **(I)** der chemischen Struktur **(I-A)** einsetzt.
4.15 Verfahren nach einem oder mehreren der Punkte 4.10 bis 4.14, wobei man es in mindestens einem Lösungsmittel durchführt, wobei das Lösungsmittel ausgewählt ist aus der Gruppe bestehend aus aliphatische Lösungsmittel, aromatische Lösungsmittel, Ether, halogenierte Lösungsmittel, Amide, Thioverbindungen, Carbonsäuren, Alkohole, Wasser.
4.16 Verfahren nach einem oder mehreren der Punkte 4.10 bis 4.15, wobei man es bei einer Temperatur im Bereich von 20 °C bis 350 °C und einem Druck im Bereich von 2 bar bis 500 bar durchführt.
4.17 Verfahren nach einem oder mehreren der Punkte 4.1 bis 4.9, wobei die Carbonylverbindung **(II)** die chemische Struktur **(II-B)** aufweist.
4.18 Verfahren nach Punkt 4.17, wobei man 0.4 bis 2.0, insbesondere 0.45 bis 1.5, bevorzugt 0.6 bis 1.3, bevorzugter 0.65 bis 1.2, noch bevorzugter 0.75 bis 1.0, am bevorzugtesten 0.8 bis 0.9 molare Äquivalente der Carbonylverbindung **(II)** der chemischen Struktur **(II-B)** pro Triacetondiaminverbindung **(I)** der chemischen Struktur **(I-A)** einsetzt.
4.19 Verfahren nach einem oder mehreren der Punkte 4.17 bis 4.18, wobei man es in mindestens einem Lösungsmittel durchführt, wobei das Lösungsmittel ausgewählt ist aus der Gruppe bestehend aus aliphatische Lösungsmittel, aromatische Lösungsmittel, Ether, halogenierte Lösungsmittel, Amide, Thioverbindungen, Carbonsäuren, Alkohole, Wasser.
4.20 Verfahren nach einem oder mehreren der Punkte 4.17 bis 4.19, wobei man es bei einer Temperatur im Bereich von 20 °C bis 350 °C und einem Druck im Bereich von 2 bar bis 500 bar durchführt.

Die vorliegende Erfindung betrifft in einem fünften Aspekt ein Verfahren gemäß den folgenden Punkten 5.1 bis 5.15.
5.1 Verfahren zur Herstellung einer *N*-substituierten Triacetondiaminverbindung,
   dadurch gekennzeichnet, dass man mindestens eine Triacetondiaminverbindung **(I)** mit mindestens einer Carbonylverbindung **(II)** unter reduktiven Bedingungen umsetzt,
   wobei die Triacetondiaminverbindung **(I)** die chemische Struktur **(I-C)** aufweist mit 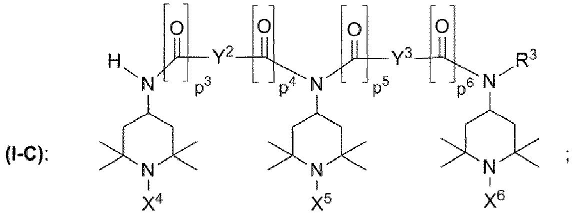
   wobei p³, p⁴, p⁵, p⁶ unabhängig voneinander jeweils 0 oder 1 ist;
   wobei X⁴, X⁵, X⁶ unabhängig voneinander jeweils ausgewählt ist aus der Gruppe bestehend aus Wasserstoff, OH, -O·, unverzweigte oder verzweigte Alkylgruppe mit 1 bis 10 Kohlenstoffatomen, unverzweigte oder verzweigte Alkoxygruppe mit 1 bis 10 Kohlenstoffatomen;
   wobei Y², Y³ unabhängig voneinander jeweils ausgewählt ist aus der Gruppe bestehend aus
      unverzweigte oder verzweigte Alkylengruppe mit 1 bis 30 Kohlenstoffatomen,
      zweiwertige gesättigte Kohlenwasserstoffgruppe mit 3 bis 30 Kohlenstoffatomen, welche mindestens einen gesättigten Ring aus 3 bis 30 Kohlenstoffatomen aufweist,
      zweiwertige Kohlenwasserstoffgruppe mit 6 bis 30 Kohlenstoffatomen, von denen mindestens 6 Kohlenstoffatome in einem aromatischen System vorliegen und die übrigen Kohlenstoffatome, falls vorhanden, gesättigt sind,
      ein verbrückender Rest, der eine chemische Struktur ausgewählt aus der Gruppe bestehend aus **(i), (ii)** mit 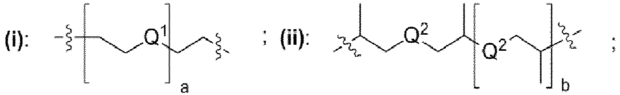
      aufweist, wobei
         Q¹, Q² unabhängig voneinander jeweils ausgewählt aus der Gruppe bestehend aus -O-, -S-, -NH- oder -NR'- mit R' = unverzweigte oder verzweigte Alkylgruppe mit 1 bis 6 Kohlenstoffatomen sind,
         wobei a eine ganze Zahl ausgewählt aus dem Bereich 1 bis 50 ist,
         wobei b eine ganze Zahl ausgewählt aus dem Bereich 0 bis 50 ist,
   und wobei Y² auch eine direkte Bindung sein kann, falls mindestens einer von p³ und p⁴ den Wert 1 aufweist,
   und wobei Y³ auch eine direkte Bindung sein kann, falls mindestens einer von p⁵ und p⁶ den Wert 1 aufweist,
   wobei der Rest R³ ausgewählt ist aus der Gruppe bestehend aus
      Wasserstoff,
      unverzweigte oder verzweigte Alkylgruppe mit 1 bis 30 Kohlenstoffatomen, bei welcher mindestens ein Wasserstoffrest durch einen Rest ausgewählt aus der Gruppe bestehend aus -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann,
      unverzweigte oder verzweigte Acylgruppe mit 1 bis 30 Kohlenstoffatomen, bei welcher mindestens ein Wasserstoffrest durch einen Rest ausgewählt aus der Gruppe bestehend aus -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann,
      einem Rest, der eine chemischen Struktur ausgewählt aus der Gruppe bestehend aus **(iii), (iv), (v), (vi), (vii), (viii), (ix)** mit 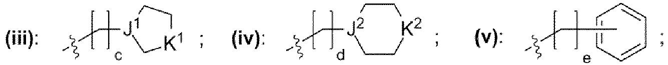 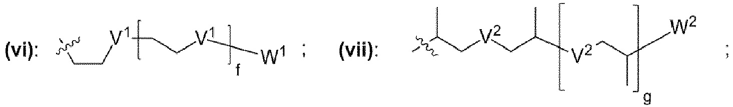 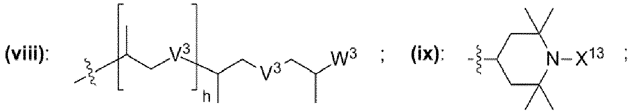
      aufweist,
         wobei J¹, J² unabhängig voneinander jeweils ausgewählt aus der Gruppe bestehend aus CH, N sind,
         wobei K¹, K² unabhängig voneinander jeweils ausgewählt aus der Gruppe bestehend aus -O-, -NH-, -N(CH₃)-, -N(CH₂CH₃)-, -S-, -CH₂- sind,
         wobei V¹, V², V³ unabhängig voneinander jeweils ausgewählt aus der Gruppe bestehend aus -O-, -S-, -NH-, -NR"- mit R" = unverzweigte oder verzweigte Alkylgruppe mit 1 bis 6 Kohlenstoffatomen sind,
         wobei W¹, W², W³ unabhängig voneinander jeweils ausgewählt aus der Gruppe bestehend aus H, Methyl, Ethyl sind,
         wobei c, d, e, f, g, h unabhängig voneinander jeweils eine ganze Zahl aus dem Bereich 0 bis 50 ist,
         wobei X¹³ ausgewählt ist aus der Gruppe bestehend aus Wasserstoff, -OH, -O·, unverzweigte oder verzweigte Alkylgruppe mit 1 bis 10 Kohlenstoffatomen, unverzweigte oder verzweigte Alkoxygruppe mit 1 bis 10 Kohlenstoffatomen,
         wobei in den chemischen Strukturen **(iii), (iv), (v), (vi), (vii), (viii), (ix)** mindestens ein an ein Kohlenstoffatom gebundener Wasserstoffrest durch einen Rest ausgewählt aus der Gruppe bestehend aus
         -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann;
   und wobei die Carbonylverbindung **(II)** ausgewählt ist aus der Gruppe bestehend aus den chemischen Strukturen **(II-A), (II-B)** mit 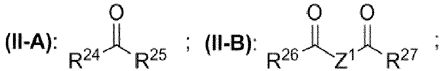
   wobei Z¹ ausgewählt ist aus der Gruppe bestehend aus
      direkte Bindung,
      unverzweigte oder verzweigte Alkylengruppe mit 1 bis 30 Kohlenstoffatomen,
      zweiwertige gesättigte Kohlenwasserstoffgruppe mit 3 bis 30 Kohlenstoffatomen, welche mindestens einen gesättigten Ring aus 3 bis 30 Kohlenstoffatomen aufweist,
      zweiwertige Kohlenwasserstoffgruppe mit 6 bis 30 Kohlenstoffatomen, von denen mindestens 6 Kohlenstoffatome in einem aromatischen System vorliegen und die übrigen Kohlenstoffatome, falls vorhanden, gesättigt sind,
      ein verbrückender Rest, der eine chemischen Struktur ausgewählt aus der Gruppe bestehend aus **(xix), (xx)** mit 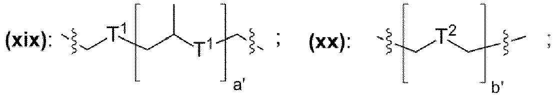
      aufweist, wobei
         T¹, T² unabhängig voneinander jeweils ausgewählt aus der Gruppe bestehend aus -O-, -S- oder -NR""- mit R"" = unverzweigte oder verzweigte Alkylgruppe mit 1 bis 6 Kohlenstoffatomen sind,
         wobei a' und b' unabhängig voneinander jeweils eine ganze Zahl ausgewählt aus dem Bereich 1 bis 50 ist;
   und wobei die Reste R²⁴, R²⁵, R²⁶, R²⁷ ausgewählt sind aus der Gruppe bestehend aus
      Wasserstoff,
      unverzweigte oder verzweigte Alkylgruppe mit 1 bis 30 Kohlenstoffatomen, bei welcher mindestens ein Wasserstoffrest durch einen Rest ausgewählt aus der Gruppe bestehend aus -OH, -OCH₃, -OCH₂CH₃, -N(CH₃)₂, -N(CH₂CH₃)₂, -N(CH₃)(CH₂CH₃) ersetzt sein kann,
      ein Rest, der eine chemischen Struktur ausgewählt aus der Gruppe bestehend aus **(xxi), (xxii), (xxiii), (xxiv), (xxv), (xxvi)** mit 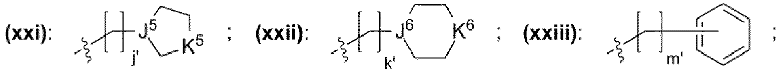 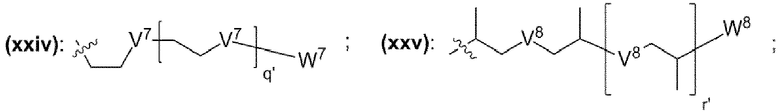 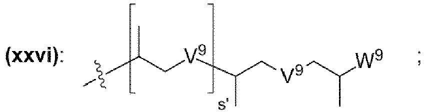
      aufweist,
         wobei J⁵, J⁶ unabhängig voneinander jeweils ausgewählt aus der Gruppe bestehend aus CH, N sind,
         wobei K⁵, K⁶ unabhängig voneinander jeweils ausgewählt aus der Gruppe bestehend aus -O-, -N(CH₃)-, -N(CH₂CH₃)-, -S-, -CH₂- sind,
         wobei V⁷, V⁸, V⁹ unabhängig voneinander jeweils ausgewählt aus der Gruppe bestehend aus -O-, -S-, -NR'""- mit R'"" = unverzweigte oder verzweigte Alkylgruppe mit 1 bis 6 Kohlenstoffatomen sind,
         wobei W⁷, W⁸, W⁹ unabhängig voneinander jeweils ausgewählt aus der Gruppe bestehend aus H, Methyl, Ethyl sind,
         wobei j', k', m', q', r', s' unabhängig voneinander jeweils eine ganze Zahl ausgewählt aus dem Bereich 0 bis 50 ist,
         wobei in der chemischen Struktur **(xxi), (xxii), (xxiii), (xxiv), (xxv), (xxvi)** mindestens ein Wasserstoffrest durch einen Rest ausgewählt aus der Gruppe bestehend aus -OH, -OCH₃, -OCH₂CH₃, -N(CH₃)₂, -N(CH₂CH₃)₂, -N(CH₃)(CH₂CH₃) ersetzt sein kann;
   und wobei R²⁴, R²⁵, R²⁶, R²⁷ jeweils unabhängig voneinander ausgewählt sind, bis auf die Ausnahme, dass nicht gilt: R²⁴ = R²⁵ = Wasserstoff,
   und wobei man reduktive Bedingungen dadurch einstellt, dass man die mindestens eine Triacetondiaminverbindung **(I)** mit der mindestens einen Carbonylverbindung **(II)** in Gegenwart von Wasserstoff und in Gegenwart eines Vollkatalysators umsetzt, wobei der Vollkatalysator mindestens ein Metall **M** aufweist, wobei das Metall **M** ausgewählt ist aus der Gruppe bestehend aus Ag, V, Cr, Mo, Mn, Ni, Pd, Pt, Fe, Ru, Os, Co, Rh, Ir, Cu, insbesondere Ag, Fe, Ni, Co, Cu, bevorzugt Ni, Co, bevorzugter Ni.
5.2 Verfahren nach Punkt 5.1, wobei p³ = p⁴ = p⁵ = p⁶ = 0 ist.
5.3 Verfahren nach Punkt 5.1 oder 5.2, wobei
   Y², Y³ unabhängig voneinander jeweils ausgewählt ist aus der Gruppe bestehend aus
   unverzweigte oder verzweigte Alkylengruppe mit 1 bis 30 Kohlenstoffatomen,
   zweiwertige gesättigte Kohlenwasserstoffgruppe mit 3 bis 30 Kohlenstoffatomen, welche mindestens einen gesättigten Ring aus 3 bis 30 Kohlenstoffatomen aufweist;
      wobei bevorzugt Y², Y³ unabhängig voneinander jeweils eine unverzweigte oder verzweigte Alkylengruppe mit 1 bis 12, noch bevorzugter 1 bis 6, Kohlenstoffatomen ist.
5.4 Verfahren nach einem oder mehreren der Punkte 5.1 bis 5.3, wobei der Rest R³ ausgewählt ist aus der Gruppe bestehend aus
   Wasserstoff,
   unverzweigte oder verzweigte Alkylgruppe mit 1 bis 30 Kohlenstoffatomen, bei welcher mindestens ein Wasserstoffrest durch einen Rest ausgewählt aus der Gruppe bestehend aus -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann,
   einem Rest, der eine chemischen Struktur **(ix)** mit 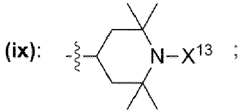
   aufweist,
   wobei X¹³ ausgewählt ist aus der Gruppe bestehend aus Wasserstoff, -OH, -O·, unverzweigte oder verzweigte Alkylgruppe mit 1 bis 10 Kohlenstoffatomen, unverzweigte oder verzweigte Alkoxygruppe mit 1 bis 10 Kohlenstoffatomen.
5.5 Verfahren nach einem oder mehreren der Punkte 5.1 bis 5.4, wobei X⁴ = X⁵ = X⁶ = Wasserstoff ist.
5.6 Verfahren nach einem oder mehreren der Punkte 5.1 bis 5.5, wobei der Rest R³ ausgewählt ist aus der Gruppe bestehend aus
   Wasserstoff,
   unverzweigte oder verzweigte Alkylgruppe mit 1 bis 12 Kohlenstoffatomen, bei welcher mindestens ein Wasserstoffrest durch einen Rest ausgewählt aus der Gruppe bestehend aus -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann.
5.7 Verfahren nach einem oder mehreren der Punkte 5.1 bis 5.6, wobei der Rest R³ ausgewählt ist aus der Gruppe bestehend aus
   Wasserstoff,
   unverzweigte oder verzweigte Alkylgruppe mit 1 bis 12 Kohlenstoffatomen.
5.8 Verfahren nach einem oder mehreren der Punkte 5.1 bis 5.7, wobei Z¹ ausgewählt ist aus der Gruppe bestehend aus
   direkte Bindung,
   unverzweigte oder verzweigte Alkylengruppe mit 1 bis 30 Kohlenstoffatomen,
   zweiwertige gesättigte Kohlenwasserstoffgruppe mit 3 bis 30 Kohlenstoffatomen, welche mindestens einen gesättigten Ring aus 3 bis 30 Kohlenstoffatomen aufweist.
5.9 Verfahren nach Punkt 5.8, wobei Z¹ ausgewählt ist aus der Gruppe bestehend aus direkte Bindung,
   unverzweigte oder verzweigte Alkylengruppe mit 1 bis 12, bevorzugt 1 bis 6, Kohlenstoffatomen.
5.10 Verfahren nach einem oder mehreren der Punkte 5.1 bis 5.9, wobei die Carbonylverbindung **(II)** die chemische Struktur **(II-A)** aufweist.
5.11 Verfahren nach Punkt 5.10, wobei
   X⁴ = X⁵ = X⁶ = X¹³ = Wasserstoff;
   R³ ausgewählt ist aus der Gruppe bestehend aus
   Wasserstoff,
   unverzweigte oder verzweigte Alkylgruppe mit 1 bis 12 Kohlenstoffatomen;
   und R²⁴, R²⁵ ausgewählt sind aus der Gruppe bestehend aus
   Wasserstoff,
   unverzweigte oder verzweigte Alkylgruppe mit 1 bis 12 Kohlenstoffatomen;
   und wobei R²⁴, R²⁵ jeweils unabhängig voneinander ausgewählt sind, bis auf die Ausnahme, dass nicht gilt: R²⁴ = R²⁵ = Wasserstoff.
5.12 Verfahren nach Punkt 5.11, wobei
   X⁴ = X⁵ = X⁶ = X¹³ = Wasserstoff;
   R³ ausgewählt ist aus der Gruppe bestehend aus
   Wasserstoff,
   unverzweigte oder verzweigte Alkylgruppe mit 1 bis 8 Kohlenstoffatomen;
   und R²⁴, R²⁵ ausgewählt sind aus der Gruppe bestehend aus
   Wasserstoff,
   unverzweigte oder verzweigte Alkylgruppe mit 1 bis 8 Kohlenstoffatomen;
   und wobei R²⁴, R²⁵ jeweils unabhängig voneinander ausgewählt sind, bis auf die Ausnahme, dass nicht gilt: R²⁴ = R²⁵ = Wasserstoff.
5.13 Verfahren nach Punkt 5.12, wobei X⁴ = X⁵ = X⁶ = X¹³ = Wasserstoff; R³ = Wasserstoff;
   R²⁴ ausgewählt ist aus der Gruppe bestehend aus Wasserstoff, Methyl;
   R²⁵ eine unverzweigte oder verzweigte Alkylgruppe mit 1 bis 8 Kohlenstoffatomen ist.
5.14 Verfahren nach einem oder mehreren der Punkte 5.1 bis 5.13, wobei man es in mindestens einem Lösungsmittel durchführt, wobei das Lösungsmittel ausgewählt ist aus der Gruppe bestehend aus aliphatische Lösungsmittel, aromatische Lösungsmittel, Ether, halogenierte Lösungsmittel, Amide, Thioverbindungen, Carbonsäuren, Alkohole, Wasser.
5.15 Verfahren nach einem oder mehreren der Punkte 5.1 bis 5.14, wobei man es bei einer Temperatur im Bereich von 20 °C bis 350 °C und einem Druck im Bereich von 2 bar bis 500 bar durchführt.

Die vorliegende Erfindung betrifft in einem sechsten Aspekt ein Verfahren gemäß den folgenden Punkten 6.1 bis 6.16.
6.1 Verfahren zur Herstellung einer *N*-substituierten Triacetondiaminverbindung,
   dadurch gekennzeichnet, dass man mindestens eine Triacetondiaminverbindung **(I)** mit mindestens einer Carbonylverbindung **(II)** unter reduktiven Bedingungen umsetzt,
   wobei die Triacetondiaminverbindung **(I)** die chemische Struktur **(I-D)** aufweist mit 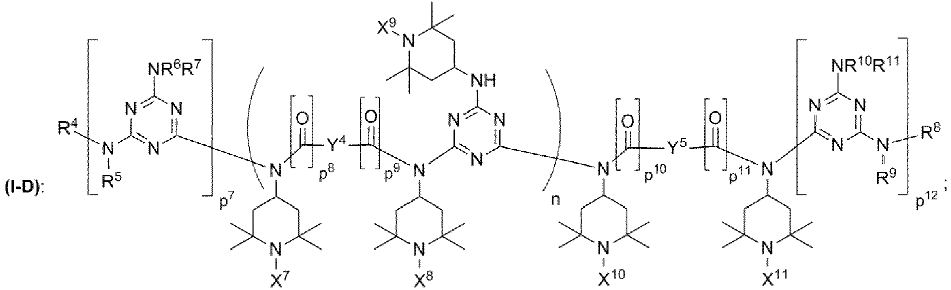
   wobei n eine ganze Zahl aus dem Bereich 1 bis 20 ist;
   wobei p⁷, p⁸, p⁹, p¹⁰, p¹¹, p¹² unabhängig voneinander jeweils 0 oder 1 ist;
   wobei X⁷, X⁸, X⁹, X¹⁰, X¹¹ unabhängig voneinander jeweils ausgewählt ist aus der Gruppe bestehend aus Wasserstoff, OH, -O·, unverzweigte oder verzweigte Alkylgruppe mit 1 bis 10 Kohlenstoffatomen, unverzweigte oder verzweigte Alkoxygruppe mit 1 bis 10 Kohlenstoffatomen;
   wobei Y⁴, Y⁵ unabhängig voneinander jeweils ausgewählt ist aus der Gruppe bestehend aus
      unverzweigte oder verzweigte Alkylengruppe mit 1 bis 30 Kohlenstoffatomen,
      zweiwertige gesättigte Kohlenwasserstoffgruppe mit 3 bis 30 Kohlenstoffatomen, welche mindestens einen gesättigten Ring aus 3 bis 30 Kohlenstoffatomen aufweist,
      zweiwertige Kohlenwasserstoffgruppe mit 6 bis 30 Kohlenstoffatomen, von denen mindestens 6 Kohlenstoffatome in einem aromatischen System vorliegen und die übrigen Kohlenstoffatome, falls vorhanden, gesättigt sind,
      ein verbrückender Rest, der eine chemische Struktur ausgewählt aus der Gruppe bestehend aus **(i), (ii)** mit 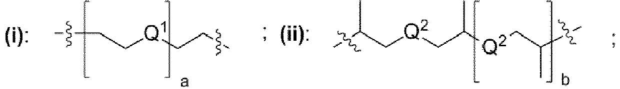
      aufweist, wobei
         Q¹, Q² unabhängig voneinander jeweils ausgewählt aus der Gruppe bestehend aus -O-, -S-, -NH- oder -NR'- mit R' = unverzweigte oder verzweigte Alkylgruppe mit 1 bis 6 Kohlenstoffatomen sind,
         wobei a eine ganze Zahl ausgewählt aus dem Bereich 1 bis 50 ist,
         wobei b eine ganze Zahl ausgewählt aus dem Bereich 0 bis 50 ist,
   und wobei Y⁴ auch eine direkte Bindung sein kann, falls mindestens einer von p⁸ und p⁹ den Wert 1 aufweist,
   und wobei Y⁵ auch eine direkte Bindung sein kann, falls mindestens einer von p¹⁰ und p¹¹ den Wert 1 aufweist;
   wobei die Reste R⁴, R⁵, R⁶, R⁷, R⁸, R⁹, R¹⁰, R¹¹ unabhängig voneinander jeweils ausgewählt sind aus der Gruppe bestehend aus
      Wasserstoff,
      unverzweigte oder verzweigte Alkylgruppe mit 1 bis 30 Kohlenstoffatomen, bei welcher mindestens ein Wasserstoffrest durch einen Rest ausgewählt aus der Gruppe bestehend aus -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann,
      unverzweigte oder verzweigte Acylgruppe mit 1 bis 30 Kohlenstoffatomen, bei welcher mindestens ein Wasserstoffrest durch einen Rest ausgewählt aus der Gruppe bestehend aus -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann,
      einem Rest, der eine chemischen Struktur ausgewählt aus der Gruppe bestehend aus **(iii), (iv), (v), (vi), (vii), (viii), (ix)** mit 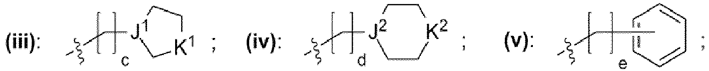 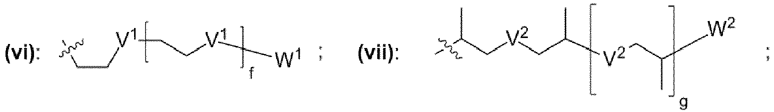 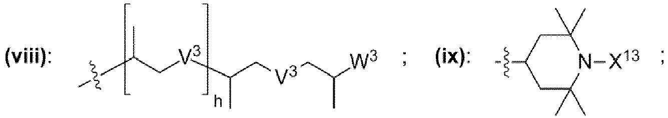
      aufweist,
         wobei J¹, J² unabhängig voneinander jeweils ausgewählt aus der Gruppe bestehend aus CH, N sind,
         wobei K¹, K² unabhängig voneinander jeweils ausgewählt aus der Gruppe bestehend aus -O-, -NH-, -N(CH₃)-, -N(CH₂CH₃)-, -S-, -CH₂- sind,
         wobei V¹, V², V³ unabhängig voneinander jeweils ausgewählt aus der Gruppe bestehend aus -O-, -S-, -NH-, -NR"- mit R" = unverzweigte oder verzweigte Alkylgruppe mit 1 bis 6 Kohlenstoffatomen sind,
         wobei W¹, W², W³ unabhängig voneinander jeweils ausgewählt aus der Gruppe bestehend aus H, Methyl, Ethyl sind,
         wobei c, d, e, f, g, h unabhängig voneinander jeweils eine ganze Zahl aus dem Bereich 0 bis 50 ist,
         wobei X¹³ ausgewählt ist aus der Gruppe bestehend aus Wasserstoff, -OH, -O·, unverzweigte oder verzweigte Alkylgruppe mit 1 bis 10 Kohlenstoffatomen, unverzweigte oder verzweigte Alkoxygruppe mit 1 bis 10 Kohlenstoffatomen,
      wobei in den chemischen Strukturen **(iii), (iv), (v), (vi), (vii), (viii), (ix)** mindestens ein an ein Kohlenstoffatom gebundener Wasserstoffrest durch einen Rest ausgewählt aus der Gruppe bestehend aus
      -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann;
   und wobei die Carbonylverbindung **(II)** ausgewählt ist aus der Gruppe bestehend aus den chemischen Strukturen **(II-A), (II-B)** mit 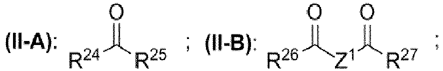
   wobei Z¹ ausgewählt ist aus der Gruppe bestehend aus
      direkte Bindung,
      unverzweigte oder verzweigte Alkylengruppe mit 1 bis 30 Kohlenstoffatomen,
      zweiwertige gesättigte Kohlenwasserstoffgruppe mit 3 bis 30 Kohlenstoffatomen, welche mindestens einen gesättigten Ring aus 3 bis 30 Kohlenstoffatomen aufweist,
      zweiwertige Kohlenwasserstoffgruppe mit 6 bis 30 Kohlenstoffatomen, von denen mindestens 6 Kohlenstoffatome in einem aromatischen System vorliegen und die übrigen Kohlenstoffatome, falls vorhanden, gesättigt sind,
      ein verbrückender Rest, der eine chemischen Struktur ausgewählt aus der Gruppe bestehend aus **(xix), (xx)** mit 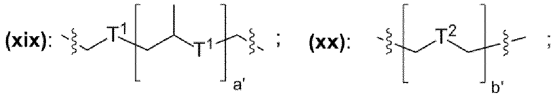
      aufweist, wobei
         T¹, T² unabhängig voneinander jeweils ausgewählt aus der Gruppe bestehend aus -O-, -S- oder -NR""- mit R"" = unverzweigte oder verzweigte Alkylgruppe mit 1 bis 6 Kohlenstoffatomen sind,
         wobei a' und b' unabhängig voneinander jeweils eine ganze Zahl ausgewählt aus dem Bereich 1 bis 50 ist;
   und wobei die Reste R²⁴, R²⁵, R²⁶, R²⁷ ausgewählt sind aus der Gruppe bestehend aus
      Wasserstoff,
      unverzweigte oder verzweigte Alkylgruppe mit 1 bis 30 Kohlenstoffatomen, bei welcher mindestens ein Wasserstoffrest durch einen Rest ausgewählt aus der Gruppe bestehend aus -OH, -OCH₃, -OCH₂CH₃, -N(CH₃)₂, -N(CH₂CH₃)₂, -N(CH₃)(CH₂CH₃) ersetzt sein kann,
      ein Rest, der eine chemischen Struktur ausgewählt aus der Gruppe bestehend aus **(xxi), (xxii), (xxiii), (xxiv), (xxv), (xxvi)** mit 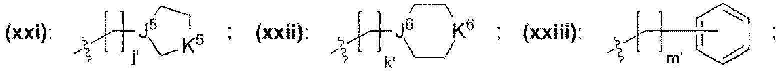 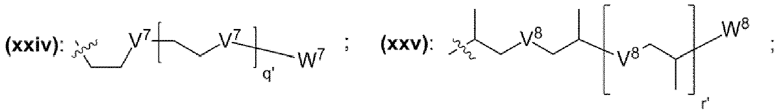 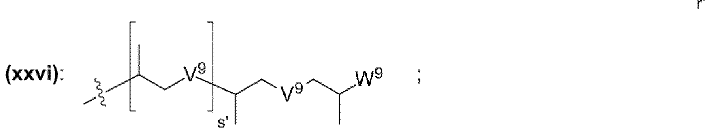
      aufweist,
         wobei J⁵, J⁶ unabhängig voneinander jeweils ausgewählt aus der Gruppe bestehend aus CH, N sind,
         wobei K⁵, K⁶ unabhängig voneinander jeweils ausgewählt aus der Gruppe bestehend aus -O-, -N(CH₃)-, -N(CH₂CH₃)-, -S-, -CH₂- sind,
         wobei V⁷, V⁸, V⁹ unabhängig voneinander jeweils ausgewählt aus der Gruppe bestehend aus -O-, -S-, -NR'""- mit R'"" = unverzweigte oder verzweigte Alkylgruppe mit 1 bis 6 Kohlenstoffatomen sind,
         wobei W⁷, W⁸, W⁹ unabhängig voneinander jeweils ausgewählt aus der Gruppe bestehend aus H, Methyl, Ethyl sind,
         wobei j', k', m', q', r', s' unabhängig voneinander jeweils eine ganze Zahl ausgewählt aus dem Bereich 0 bis 50 ist,
         wobei in der chemischen Struktur **(xxi), (xxii), (xxiii), (xxiv), (xxv), (xxvi)** mindestens ein Wasserstoffrest durch einen Rest ausgewählt aus der Gruppe bestehend aus -OH, -OCH₃, -OCH₂CH₃, -N(CH₃)₂, -N(CH₂CH₃)₂, -N(CH₃)(CH₂CH₃) ersetzt sein kann;
   und wobei R²⁴, R²⁵, R²⁶, R²⁷ jeweils unabhängig voneinander ausgewählt sind, bis auf die Ausnahme, dass nicht gilt: R²⁴ = R²⁵ = Wasserstoff,
   und wobei man reduktive Bedingungen dadurch einstellt, dass man die mindestens eine Triacetondiaminverbindung **(I)** mit der mindestens einen Carbonylverbindung **(II)** in Gegenwart von Wasserstoff und in Gegenwart eines Vollkatalysators umsetzt, wobei der Vollkatalysator mindestens ein Metall **M** aufweist, wobei das Metall **M** ausgewählt ist aus der Gruppe bestehend aus Ag, V, Cr, Mo, Mn, Ni, Pd, Pt, Fe, Ru, Os, Co, Rh, Ir, Cu, insbesondere Ag, Fe, Ni, Co, Cu, bevorzugt Ni, Co, bevorzugter Ni.
6.2 Verfahren nach Punkt 6.1, wobei p⁸ = p⁹ = p¹⁰ = p¹¹ = 0 ist und wobei p⁷, p¹² unabhängig voneinander jeweils 0 oder 1 ist.
6.3 Verfahren nach Punkt 6.1 oder 6.2, wobei Y⁴, Y⁵ unabhängig voneinander jeweils ausgewählt ist aus der Gruppe bestehend aus
   unverzweigte oder verzweigte Alkylengruppe mit 1 bis 30 Kohlenstoffatomen,
   zweiwertige gesättigte Kohlenwasserstoffgruppe mit 3 bis 30 Kohlenstoffatomen, welche mindestens einen gesättigten Ring aus 3 bis 30 Kohlenstoffatomen aufweist;
      wobei bevorzugt Y⁴, Y⁵ unabhängig voneinander jeweils eine unverzweigte oder verzweigte Alkylengruppe mit 1 bis 12, noch bevorzugter 1 bis 6, Kohlenstoffatomen ist.
6.4 Verfahren nach einem oder mehreren der Punkte 6.1 bis 6.3, wobei die Reste R⁴, R⁵, R⁶, R⁷, R⁸, R⁹, R¹⁰, R¹¹ unabhängig voneinander jeweils ausgewählt sind aus der Gruppe bestehend aus
   Wasserstoff,
   unverzweigte oder verzweigte Alkylgruppe mit 1 bis 30 Kohlenstoffatomen, bei welcher mindestens ein Wasserstoffrest durch einen Rest ausgewählt aus der Gruppe bestehend aus -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann,
   einem Rest, der eine chemischen Struktur (ix) mit 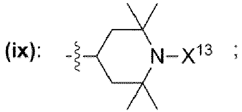
   aufweist,
      wobei X¹³ ausgewählt ist aus der Gruppe bestehend aus Wasserstoff, -OH, -O·, unverzweigte oder verzweigte Alkylgruppe mit 1 bis 10 Kohlenstoffatomen, unverzweigte oder verzweigte Alkoxygruppe mit 1 bis 10 Kohlenstoffatomen.
6.5 Verfahren nach einem oder mehreren der Punkte 6.1 bis 6.4, wobei X⁷ = X⁸ = X⁹ = X¹⁰ = X¹¹ = X¹³ = Wasserstoff ist.
6.6 Verfahren nach einem oder mehreren der Punkte 6.1 bis 6.5, wobei die Reste R⁴, R⁵, R⁶, R⁷, R⁸, R⁹, R¹⁰, R¹¹ unabhängig voneinander jeweils ausgewählt sind aus der Gruppe bestehend aus
   Wasserstoff,
   unverzweigte oder verzweigte Alkylgruppe mit 1 bis 12 Kohlenstoffatomen, bei welcher mindestens ein Wasserstoffrest durch einen Rest ausgewählt aus der Gruppe bestehend aus -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann.
6.7 Verfahren nach Punkt 6.6, wobei die Reste R⁴, R⁵, R⁶, R⁷, R⁸, R⁹, R¹⁰, R¹¹ unabhängig voneinander jeweils ausgewählt sind aus der Gruppe bestehend aus
   Wasserstoff,
   unverzweigte oder verzweigte Alkylgruppe mit 1 bis 12 Kohlenstoffatomen.
6.8 Verfahren nach einem oder mehreren der Punkte 6.1 bis 6.7, wobei Z¹ ausgewählt ist aus der Gruppe bestehend aus
   direkte Bindung,
   unverzweigte oder verzweigte Alkylengruppe mit 1 bis 30 Kohlenstoffatomen,
   zweiwertige gesättigte Kohlenwasserstoffgruppe mit 3 bis 30 Kohlenstoffatomen, welche mindestens einen gesättigten Ring aus 3 bis 30 Kohlenstoffatomen aufweist.
6.9 Verfahren nach Punkt 6.8, wobei Z¹ ausgewählt ist aus der Gruppe bestehend aus
   direkte Bindung,
   unverzweigte oder verzweigte Alkylengruppe mit 1 bis 12, bevorzugt 1 bis 6, Kohlenstoffatomen.
6.10 Verfahren nach einem oder mehreren der Punkte 6.1 bis 6.9 wobei die Carbonylverbindung **(II)** die chemische Struktur **(II-A)** aufweist.
6.11 Verfahren nach Punkt 6.10, wobei
   X⁷ = X⁸ = X⁹ = X¹⁰ = X¹¹ = X¹³ = Wasserstoff;
   R⁴, R⁵, R⁶, R⁷, R⁸, R⁹, R¹⁰, R¹¹ unabhängig voneinander jeweils ausgewählt sind aus der Gruppe bestehend aus
   Wasserstoff,
   unverzweigte oder verzweigte Alkylgruppe mit 1 bis 12 Kohlenstoffatomen;
   R²⁴, R²⁵ ausgewählt sind aus der Gruppe bestehend aus
   Wasserstoff,
   unverzweigte oder verzweigte Alkylgruppe mit 1 bis 12 Kohlenstoffatomen;
   und wobei R²⁴, R²⁵ jeweils unabhängig voneinander ausgewählt sind, bis auf die Ausnahme, dass nicht gilt: R²⁴ = R²⁵ = Wasserstoff.
6.12 Verfahren nach Punkt 6.11, wobei
   X⁷ = X⁸ = X⁹ = X¹⁰ = X¹¹ = X¹³ = Wasserstoff;
   R⁴, R⁵, R⁶, R⁷, R⁸, R⁹, R¹⁰, R¹¹ unabhängig voneinander jeweils ausgewählt sind aus der Gruppe bestehend aus
   Wasserstoff,
   unverzweigte oder verzweigte Alkylgruppe mit 1 bis 8 Kohlenstoffatomen;
   R²⁴, R²⁵ ausgewählt sind aus der Gruppe bestehend aus
   Wasserstoff,
   unverzweigte oder verzweigte Alkylgruppe mit 1 bis 8 Kohlenstoffatomen;
   und wobei R²⁴, R²⁵ jeweils unabhängig voneinander ausgewählt sind, bis auf die Ausnahme, dass nicht gilt: R²⁴ = R²⁵ = Wasserstoff.
6.13 Verfahren nach Punkt 6.12, wobei
   X⁷ = X⁸ = X⁹ = X¹⁰ = X¹¹ = X¹³ = Wasserstoff;
   R⁴, R⁵, R⁶, R⁷, R⁸, R⁹, R¹⁰, R¹¹ unabhängig voneinander jeweils ausgewählt sind aus der Gruppe bestehend aus
   Wasserstoff,
   unverzweigte oder verzweigte Alkylgruppe mit 1 bis 6 Kohlenstoffatomen;
   R²⁴ ausgewählt ist aus der Gruppe bestehend aus Wasserstoff, Methyl;
   R²⁵ eine unverzweigte oder verzweigte Alkylgruppe mit 1 bis 8 Kohlenstoffatomen ist.
   6.14 Verfahren nach Punkt 6.13, wobei
   X⁷ = X⁸ = X⁹ = X¹⁰ = X¹¹ = X¹³ = Wasserstoff;
   R⁴, R⁵, R⁶, R⁷, R⁸, R⁹, R¹⁰, R¹¹ unabhängig voneinander jeweils eine unverzweigte oder verzweigte Alkylgruppe mit 1 bis 6 Kohlenstoffatomen sind;
   R²⁴ ausgewählt ist aus der Gruppe bestehend aus Wasserstoff, Methyl;
   R²⁵ eine unverzweigte oder verzweigte Alkylgruppe mit 1 bis 8 Kohlenstoffatomen ist.
6.15 Verfahren nach einem oder mehreren der Punkte 6.1 bis 6.14, wobei man es in mindestens einem Lösungsmittel durchführt, wobei das Lösungsmittel ausgewählt ist aus der Gruppe bestehend aus aliphatische Lösungsmittel, aromatische Lösungsmittel, Ether, halogenierte Lösungsmittel, Amide, Thioverbindungen, Carbonsäuren, Alkohole, Wasser.
6.16 Verfahren nach einem oder mehreren der Punkte 6.1 bis 6.15, wobei man es bei einer Temperatur im Bereich von 20 °C bis 350 °C und einem Druck im Bereich von 2 bar bis 500 bar durchführt.

Die vorliegende Erfindung betrifft in einem siebten Aspekt ein Verfahren gemäß den folgenden Punkten 7.1 bis 7.13.
7.1 Verfahren zur Herstellung einer *N*-substituierten Triacetondiaminverbindung,
   dadurch gekennzeichnet, dass man mindestens eine Triacetondiaminverbindung **(I)** mit mindestens einer Carbonylverbindung **(II)** unter reduktiven Bedingungen umsetzt,
   wobei die Triacetondiaminverbindung **(I)** die chemische Struktur **(I-E)** aufweist mit 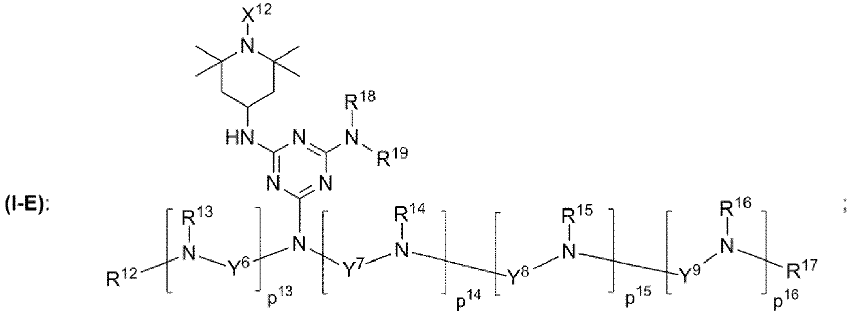
   wobei p¹³, p¹⁴, p¹⁵, p¹⁶ unabhängig voneinander jeweils 0 oder 1 ist;
   wobei X¹² ausgewählt ist aus der Gruppe bestehend aus Wasserstoff, OH, -O·, unverzweigte oder verzweigte Alkylgruppe mit 1 bis 10 Kohlenstoffatomen, unverzweigte oder verzweigte Alkoxygruppe mit 1 bis 10 Kohlenstoffatomen;
   wobei Y⁶, Y⁷, Y⁸, Y⁹ unabhängig voneinander jeweils ausgewählt ist aus der Gruppe bestehend aus
      unverzweigte oder verzweigte Alkylengruppe mit 1 bis 30 Kohlenstoffatomen,
      zweiwertige gesättigte Kohlenwasserstoffgruppe mit 3 bis 30 Kohlenstoffatomen, welche mindestens einen gesättigten Ring aus 3 bis 30 Kohlenstoffatomen aufweist,
      zweiwertige Kohlenwasserstoffgruppe mit 6 bis 30 Kohlenstoffatomen, von denen mindestens 6 Kohlenstoffatome in einem aromatischen System vorliegen und die übrigen Kohlenstoffatome, falls vorhanden, gesättigt sind,
      ein verbrückender Rest, der eine chemische Struktur ausgewählt aus der Gruppe bestehend aus **(i), (ii)** mit 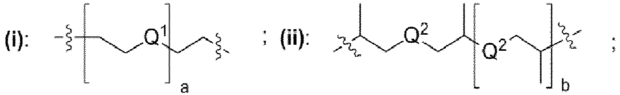
      aufweist, wobei
         Q¹, Q² unabhängig voneinander jeweils ausgewählt aus der Gruppe bestehend aus -O-, -S-, -NH- oder -NR'- mit R' = unverzweigte oder verzweigte Alkylgruppe mit 1 bis 6 Kohlenstoffatomen sind,
         wobei a eine ganze Zahl ausgewählt aus dem Bereich 1 bis 50 ist,
         wobei b eine ganze Zahl ausgewählt aus dem Bereich 0 bis 50 ist:
   wobei die Reste R¹², R¹³, R¹⁴, R¹⁵, R¹⁶, R¹⁷ unabhängig voneinander jeweils ausgewählt sind aus der Gruppe bestehend aus
      Wasserstoff,
      unverzweigte oder verzweigte Alkylgruppe mit 1 bis 30 Kohlenstoffatomen,
      einer Gruppe mit der chemischen Struktur **(x)** mit 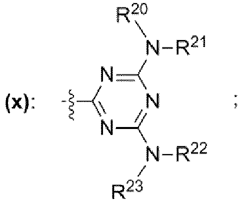
   wobei die Reste R¹⁸, R¹⁹, R²⁰, R²¹, R²², R²³ unabhängig voneinander jeweils ausgewählt sind aus der Gruppe bestehend aus
      Wasserstoff,
      unverzweigte oder verzweigte Alkylgruppe mit 1 bis 30 Kohlenstoffatomen, bei welcher mindestens ein Wasserstoffrest durch einen Rest ausgewählt aus der Gruppe bestehend aus -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann,
      unverzweigte oder verzweigte Acylgruppe mit 1 bis 30 Kohlenstoffatomen, bei welcher mindestens ein Wasserstoffrest durch einen Rest ausgewählt aus der Gruppe bestehend aus -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann,
      einem Rest, der eine chemischen Struktur ausgewählt aus der Gruppe bestehend aus **(xi), (xii), (xiii), (xiv), (xv), (xvi), (xvii)** mit 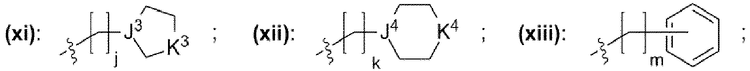 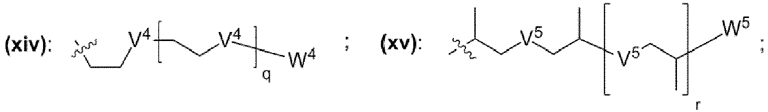 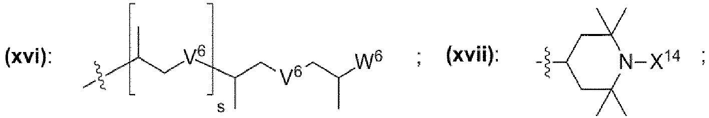
      aufweist,
         wobei J³, J⁴ unabhängig voneinander jeweils ausgewählt aus der Gruppe bestehend aus CH, N sind,
         wobei K³, K⁴ unabhängig voneinander jeweils ausgewählt aus der Gruppe bestehend aus -O-, -NH-, -N(CH₃)-, -N(CH₂CH₃)-, -S-, -CH₂- sind,
         wobei V⁴, V⁵, V⁶ unabhängig voneinander jeweils ausgewählt aus der Gruppe bestehend aus -O-, -S-, -NH-, -NR"'- mit R'" = unverzweigte oder verzweigte Alkylgruppe mit 1 bis 6 Kohlenstoffatomen sind,
         wobei W⁴, W⁵, W⁶ unabhängig voneinander jeweils ausgewählt aus der Gruppe bestehend aus H, Methyl, Ethyl sind,
         wobei j, k, m, q, r, s unabhängig voneinander jeweils eine ganze Zahl aus dem Bereich 0 bis 50 ist,
         wobei X¹⁴ ausgewählt ist aus der Gruppe bestehend aus Wasserstoff, -OH, -O·, unverzweigte oder verzweigte Alkylgruppe mit 1 bis 10 Kohlenstoffatomen, unverzweigte oder verzweigte Alkoxygruppe mit 1 bis 10 Kohlenstoffatomen,
         wobei in den chemischen Strukturen **(xi), (xii), (xiii), (xiv), (xv), (xvi), (xvii)** mindestens ein an ein Kohlenstoffatom gebundener Wasserstoffrest durch einen Rest ausgewählt aus der Gruppe bestehend
            aus -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann,
   und mit der Maßgabe, dass R¹² und R¹⁷ für p¹³ = p¹⁴ = p¹⁵ = p¹⁶ = 0 unabhängig voneinander jeweils auch eine Gruppe der chemischen Struktur **(xviii)** mit 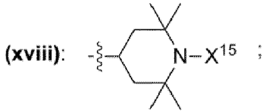
   sein können, wobei X¹⁵ ausgewählt ist aus der Gruppe bestehend aus Wasserstoff, OH, -O·, unverzweigte oder verzweigte Alkylgruppe mit 1 bis 10 Kohlenstoffatomen, unverzweigte oder verzweigte Alkoxygruppe mit 1 bis 10 Kohlenstoffatomen;
   und wobei die Carbonylverbindung **(II)** ausgewählt ist aus der Gruppe bestehend aus den chemischen Strukturen **(II-A), (II-B)** mit 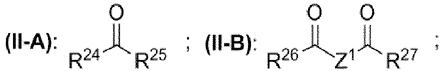
   wobei Z¹ ausgewählt ist aus der Gruppe bestehend aus
      direkte Bindung,
      unverzweigte oder verzweigte Alkylengruppe mit 1 bis 30 Kohlenstoffatomen,
      zweiwertige gesättigte Kohlenwasserstoffgruppe mit 3 bis 30 Kohlenstoffatomen, welche mindestens einen gesättigten Ring aus 3 bis 30 Kohlenstoffatomen aufweist,
      zweiwertige Kohlenwasserstoffgruppe mit 6 bis 30 Kohlenstoffatomen, von denen mindestens 6 Kohlenstoffatome in einem aromatischen System vorliegen und die übrigen Kohlenstoffatome, falls vorhanden, gesättigt sind,
      ein verbrückender Rest, der eine chemischen Struktur ausgewählt aus der Gruppe bestehend aus **(xix), (xx)** mit 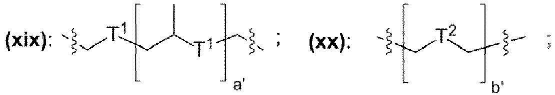
      aufweist, wobei
         T¹, T² unabhängig voneinander jeweils ausgewählt aus der Gruppe bestehend aus -O-, -S- oder -NR""- mit R"" = unverzweigte oder verzweigte Alkylgruppe mit 1 bis 6 Kohlenstoffatomen sind,
         wobei a' und b' unabhängig voneinander jeweils eine ganze Zahl ausgewählt aus dem Bereich 1 bis 50 ist;
   und wobei die Reste R²⁴, R²⁵, R²⁶, R²⁷ ausgewählt sind aus der Gruppe bestehend aus
      Wasserstoff,
      unverzweigte oder verzweigte Alkylgruppe mit 1 bis 30 Kohlenstoffatomen, bei welcher mindestens ein Wasserstoffrest durch einen Rest ausgewählt aus der Gruppe bestehend aus -OH, -OCH₃, -OCH₂CH₃, -N(CH₃)₂, -N(CH₂CH₃)₂, -N(CH₃)(CH₂CH₃) ersetzt sein kann,
      ein Rest, der eine chemischen Struktur ausgewählt aus der Gruppe bestehend aus **(xxi), (xxii), (xxiii), (xxiv), (xxv), (xxvi)** mit 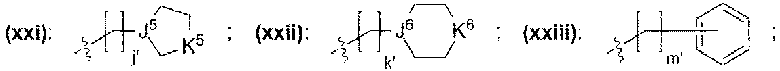 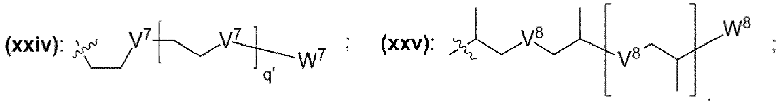 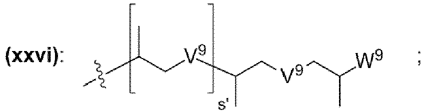
      aufweist,
         wobei J⁵, J⁶ unabhängig voneinander jeweils ausgewählt aus der Gruppe bestehend aus CH, N sind,
         wobei K⁵, K⁶ unabhängig voneinander jeweils ausgewählt aus der Gruppe bestehend aus -O-, -N(CH₃)-, -N(CH₂CH₃)-, -S-, -CH₂- sind,
         wobei V⁷, V⁸, V⁹ unabhängig voneinander jeweils ausgewählt aus der Gruppe bestehend aus -O-, -S-, -NR'""- mit R'"" = unverzweigte oder verzweigte Alkylgruppe mit 1 bis 6 Kohlenstoffatomen sind,
         wobei W⁷, W⁸, W⁹ unabhängig voneinander jeweils ausgewählt aus der Gruppe bestehend aus H, Methyl, Ethyl sind,
         wobei j', k', m', q', r', s' unabhängig voneinander jeweils eine ganze Zahl ausgewählt aus dem Bereich 0 bis 50 ist,
         wobei in der chemischen Struktur **(xxi), (xxii), (xxiii), (xxiv), (xxv), (xxvi)** mindestens ein Wasserstoffrest durch einen Rest ausgewählt aus der Gruppe bestehend aus -OH, -OCH₃, -OCH₂CH₃, -N(CH₃)₂, -N(CH₂CH₃)₂, -N(CH₃)(CH₂CH₃) ersetzt sein kann;
   und wobei R²⁴, R²⁵, R²⁶, R²⁷ jeweils unabhängig voneinander ausgewählt sind, bis auf die Ausnahme, dass nicht gilt: R²⁴ = R²⁵ = Wasserstoff,
   und wobei man reduktive Bedingungen dadurch einstellt, dass man die mindestens eine Triacetondiaminverbindung **(I)** mit der mindestens einen Carbonylverbindung **(II)** in Gegenwart von Wasserstoff und in Gegenwart eines Vollkatalysators umsetzt, wobei der Vollkatalysator mindestens ein Metall **M** aufweist, wobei das Metall **M** ausgewählt ist aus der Gruppe bestehend aus Ag, V, Cr, Mo, Mn, Ni, Pd, Pt, Fe, Ru, Os, Co, Rh, Ir, Cu, insbesondere Ag, Fe, Ni, Co, Cu, bevorzugt Ni, Co, bevorzugter Ni.
7.2 Verfahren nach Punkt 7.1, wobei Y⁶, Y⁷, Y⁸, Y⁹ unabhängig voneinander jeweils ausgewählt ist aus der Gruppe bestehend aus
   unverzweigte oder verzweigte Alkylengruppe mit 1 bis 30 Kohlenstoffatomen,
   zweiwertige gesättigte Kohlenwasserstoffgruppe mit 3 bis 30 Kohlenstoffatomen, welche mindestens einen gesättigten Ring aus 3 bis 30 Kohlenstoffatomen aufweist;
   wobei Y⁶, Y⁷, Y⁸, Y⁹ unabhängig voneinander jeweils bevorzugt eine unverzweigte oder verzweigte Alkylengruppe mit 1 bis 12 Kohlenstoffatomen, noch bevorzugter mit 1 bis 6 Kohlenstoffatomen ist.
7.3 Verfahren nach Punkt 7.1 oder 7.2, wobei die Reste R¹², R¹³, R¹⁴, R¹⁵, R¹⁶, R¹⁷ unabhängig voneinander jeweils ausgewählt sind aus der Gruppe bestehend aus
   Wasserstoff,
   unverzweigte oder verzweigte Alkylgruppe mit 1 bis 30 Kohlenstoffatomen,
   einer Gruppe mit der chemischen Struktur **(x)** mit 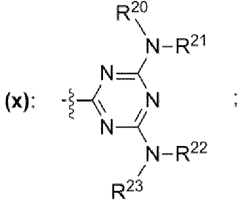
   wobei die Reste R¹⁸, R¹⁹, R²⁰, R²¹, R²², R²³ unabhängig voneinander jeweils ausgewählt sind aus der Gruppe bestehend aus
   Wasserstoff,
   unverzweigte oder verzweigte Alkylgruppe mit 1 bis 30 Kohlenstoffatomen, bei welcher mindestens ein Wasserstoffrest durch einen Rest ausgewählt aus der Gruppe bestehend aus -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann,
   einem Rest, der eine chemischen Struktur **(xvii)** mit 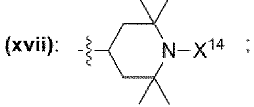
   aufweist,
      wobei X¹⁴ ausgewählt ist aus der Gruppe bestehend aus Wasserstoff, -OH, -O·, unverzweigte oder verzweigte Alkylgruppe mit 1 bis 10 Kohlenstoffatomen, unverzweigte oder verzweigte Alkoxygruppe mit 1 bis 10 Kohlenstoffatomen,
   und mit der Maßgabe, dass R¹² und R¹⁷ für p¹³ = p¹⁴ = p¹⁵ = p¹⁶ = 0 unabhängig voneinander jeweils auch eine Gruppe der chemischen Struktur **(xviii)** mit 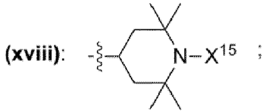 sein können, wobei X¹⁵ ausgewählt ist aus der Gruppe bestehend aus Wasserstoff, OH, -O·, unverzweigte oder verzweigte Alkylgruppe mit 1 bis 10 Kohlenstoffatomen, unverzweigte oder verzweigte Alkoxygruppe mit 1 bis 10 Kohlenstoffatomen.
7.4 Verfahren nach einem oder mehreren der Punkte 7.1 bis 7.3, wobei X¹² = X¹⁴ = X¹⁵ = Wasserstoff ist.
7.5 Verfahren nach einem oder mehreren der Punkte 7.1 bis 7.4, wobei die Reste R¹², R¹³, R¹⁴, R¹⁵, R¹⁶, R¹⁷ unabhängig voneinander jeweils ausgewählt sind aus der Gruppe bestehend aus
   Wasserstoff,
   unverzweigte oder verzweigte Alkylgruppe mit 1 bis 12 Kohlenstoffatomen
   einer Gruppe mit der chemischen Struktur **(x)** mit 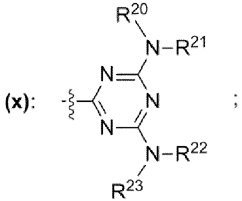
   und wobei die Reste R¹⁸, R¹⁹, R²⁰, R²¹, R²², R²³ unabhängig voneinander jeweils ausgewählt sind aus der Gruppe bestehend aus
   Wasserstoff,
   unverzweigte oder verzweigte Alkylgruppe mit 1 bis 12 Kohlenstoffatomen, bei welcher mindestens ein Wasserstoffrest durch einen Rest ausgewählt aus der Gruppe bestehend aus -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann,
   einem Rest, der eine chemischen Struktur **(xvii)** mit 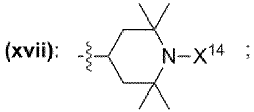
   aufweist, wobei X¹⁴ = Wasserstoff ist,
   und mit der Maßgabe, dass R¹² und R¹⁷ für p¹³ = p¹⁴ = p¹⁵ = p¹⁶ = 0 unabhängig voneinander jeweils auch eine Gruppe der chemischen Struktur **(xviii)** mit 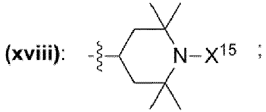 sein können, wobei X¹⁵ = Wasserstoff ist.
7.6 Verfahren nach einem oder mehreren der Punkte 7.1 bis 7.5, wobei die Reste R¹², R¹³, R¹⁴, R¹⁵, R¹⁶, R¹⁷ unabhängig voneinander jeweils ausgewählt sind aus der Gruppe bestehend aus
   unverzweigte oder verzweigte Alkylgruppe mit 1 bis 12 Kohlenstoffatomen
   einer Gruppe mit der chemischen Struktur **(x)** mit 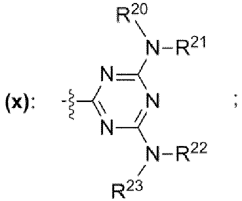
   wobei die Reste R¹⁸, R¹⁹, R²⁰, R²¹, R²², R²³ unabhängig voneinander jeweils ausgewählt sind aus der Gruppe bestehend aus
   Wasserstoff,
   unverzweigte oder verzweigte Alkylgruppe mit 1 bis 12 Kohlenstoffatomen,
   einem Rest, der eine chemischen Struktur (xvii) mit 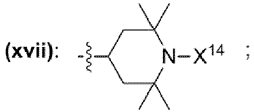
   aufweist, wobei X¹⁴ Wasserstoff ist,
   und mit der Maßgabe, dass R¹² und R¹⁷ für p¹³ = p¹⁴ = p¹⁵ = p¹⁶ = 0 unabhängig voneinander jeweils auch eine Gruppe der chemischen Struktur **(xviii)** mit 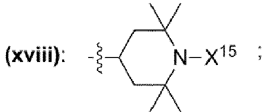 sein können, wobei X¹⁵ Wasserstoff ist.
7.7 Verfahren nach einem oder mehreren der Punkte 7.1 bis 7.6, wobei Z¹ ausgewählt ist aus der Gruppe bestehend aus
   direkte Bindung,
   unverzweigte oder verzweigte Alkylengruppe mit 1 bis 30 Kohlenstoffatomen,
   zweiwertige gesättigte Kohlenwasserstoffgruppe mit 3 bis 30 Kohlenstoffatomen, welche mindestens einen gesättigten Ring aus 3 bis 30 Kohlenstoffatomen aufweist.
7.8 Verfahren nach Punkt 7.7, wobei Z¹ ausgewählt ist aus der Gruppe bestehend aus
   direkte Bindung,
   unverzweigte oder verzweigte Alkylengruppe mit 1 bis 12, noch bevorzugter 1 bis 6, Kohlenstoffatomen.
7.9 Verfahren nach einem oder mehreren der Punkte 7.1 bis 7.8, wobei die Carbonylverbindung **(II)** die chemische Struktur **(II-A)** aufweist.
7.10 Verfahren nach Punkt 7.9, wobei
   X¹² = X¹⁴ = X¹⁵ = Wasserstoff;
   und R²⁴, R²⁵ ausgewählt sind aus der Gruppe bestehend aus
   Wasserstoff,
   unverzweigte oder verzweigte Alkylgruppe mit 1 bis 12 Kohlenstoffatomen;
   und wobei R²⁴, R²⁵ jeweils unabhängig voneinander ausgewählt sind, bis auf die Ausnahme, dass nicht gilt: R²⁴ = R²⁵ = Wasserstoff.
7.11 Verfahren nach Punkt 7.10, wobei
   X¹² = X¹⁴ = X¹⁵ = Wasserstoff;
   und R²⁴, R²⁵ ausgewählt sind aus der Gruppe bestehend aus
   Wasserstoff,
   unverzweigte oder verzweigte Alkylgruppe mit 1 bis 8 Kohlenstoffatomen, bevorzugt 1 bis 6 Kohlenstoffatomen;
   und wobei R²⁴, R²⁵ jeweils unabhängig voneinander ausgewählt sind, bis auf die Ausnahme, dass nicht gilt: R²⁴ = R²⁵ = Wasserstoff.
7.12 Verfahren nach einem oder mehreren der Punkte 7.1 bis 7.11, wobei man es in mindestens einem Lösungsmittel durchführt, wobei das Lösungsmittel ausgewählt ist aus der Gruppe bestehend aus aliphatische Lösungsmittel, aromatische Lösungsmittel, Ether, halogenierte Lösungsmittel, Amide, Thioverbindungen, Carbonsäuren, Alkohole, Wasser.
7.13 Verfahren nach einem oder mehreren der Punkte 7.1 bis 7.12, wobei man es bei einer Temperatur im Bereich von 20 °C bis 350 °C und einem Druck im Bereich von 2 bar bis 500 bar durchführt.

In einem achten Aspekt betrifft die Erfindung ein Verfahren, welches wie unter den obigen Punkten 1.1 bis 1.14, 2.1 bis 2.20, 3.1 bis 3.18, 4.1 bis 4.20, 5.1 bis 5.15, 6.1 bis 6.16 oder 7.1 bis 7.13 definiert ist, wobei aber das Merkmal
"bei welcher mindestens ein Wasserstoffrest durch einen Rest ausgewählt aus der Gruppe bestehend aus -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann"
ersetzt ist durch
"bei welcher mindestens ein Wasserstoffrest durch einen Rest ausgewählt aus der Gruppe bestehend aus -OH, -OCH₃, -OCH₂CH₃, -N(CH₃)₂, -N(CH₂CH₃)₂, -N(CH₃)(CH₂CH₃) ersetzt sein kann";
und wobei das Merkmal
"ausgewählt aus der Gruppe bestehend aus -O-, -NH-, -N(CH₃)-, -N(CH₂CH₃)-, -S-, -CH₂-"
ersetzt ist durch
"ausgewählt aus der Gruppe bestehend aus -O-, -N(CH₃)-, -N(CH₂CH₃)-, -S-, -CH₂-";
und wobei das Merkmal
"ausgewählt aus der Gruppe bestehend aus -O-, -S-, -NH- oder -NR'- mit R' = unverzweigte oder verzweigte Alkylgruppe mit 1 bis 6 Kohlenstoffatomen sind"
ersetzt ist durch
"ausgewählt aus der Gruppe bestehend aus -O-, -S- oder -NR'- mit R' = unverzweigte oder verzweigte Alkylgruppe mit 1 bis 6 Kohlenstoffatomen sind";
und wobei das Merkmal
"ausgewählt aus der Gruppe bestehend aus -O-, -S-, -NH-, -NR"- mit R" = unverzweigte oder verzweigte Alkylgruppe mit 1 bis 6 Kohlenstoffatomen sind"
ersetzt ist durch
"ausgewählt aus der Gruppe bestehend aus -O-, -S-, -NR"- mit R" = unverzweigte oder verzweigte Alkylgruppe mit 1 bis 6 Kohlenstoffatomen sind";
und wobei das Merkmal
"ausgewählt aus der Gruppe bestehend aus -O-, -S-, -NH-, -NR"'- mit R'" = unverzweigte oder verzweigte Alkylgruppe mit 1 bis 6 Kohlenstoffatomen sind"
ersetzt ist durch
"ausgewählt aus der Gruppe bestehend aus -O-, -S-, -NR"'- mit R'" = unverzweigte oder verzweigte Alkylgruppe mit 1 bis 6 Kohlenstoffatomen sind".

### Allgemeine Begriffe

Im Sinne der Erfindung ist eine "unverzweigte oder verzweigte Alkylgruppe" ein einwertiger gesättigter Kohlenwasserstoffrest der allgemeinen chemische Struktur (a) mit
**(a)**: 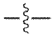 C_{w}H_{2w+1} ;
   aufweist.

Die Kette an Kohlenstoffatomen "-C_{w}H_{2w+1}" kann dabei linear sein, dann handelt es sich um eine unverzweigte Alkylgruppe. Sie kann aber auch Verzweigungen aufweisen, dann handelt es sich um eine verzweigte Alkylgruppe.

Dabei ist w in der chemischen Struktur **(a)** eine ganze Zahl. w ist bei einer unverzweigten oder verzweigten Alkylgruppe mit 1 bis 30 Kohlenstoffatomen ausgewählt aus dem Bereich 1 bis 30. w ist bei einer unverzweigten oder verzweigten Alkylgruppe mit 1 bis 29 Kohlenstoffatomen ausgewählt aus dem Bereich 1 bis 29. w ist bei einer unverzweigten oder verzweigten Alkylgruppe mit 1 bis 12 Kohlenstoffatomen ausgewählt aus dem Bereich 1 bis 12. w ist bei einer unverzweigten oder verzweigten Alkylgruppe mit 1 bis 10 Kohlenstoffatomen ausgewählt aus dem Bereich 1 bis 10. w ist bei einer unverzweigten oder verzweigten Alkylgruppe mit 1 bis 8 Kohlenstoffatomen ausgewählt aus dem Bereich 1 bis 8. w ist bei einer unverzweigten oder verzweigten Alkylgruppe mit 1 bis 6 Kohlenstoffatomen ausgewählt aus dem Bereich 1 bis 6.

Im Sinne der Erfindung ist eine "unverzweigte oder verzweigte Alkylgruppe mit 1 bis 30 Kohlenstoffatomen" insbesondere ausgewählt aus Methyl, Ethyl, *n*-Propyl, *iso*-Propyl, *n*-Butyl, *sec*-Butyl, *iso*-Butyl, *tert*-Butyl, *n*-Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, *n*-Hexyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, *n*-Heptyl, *n*-Octyl, *n*-Nonyl, *n*-Decyl, *n*-Undecyl, *n*-Dodecyl, *n*-Tridecyl, *n*-Tetradecyl, *n*-Pentadecyl, *n*-Hexadecyl, *n*-Heptadecyl, *n*-Octadecyl, *n*-Nonadecyl, *n*-Eicosyl, *n*-Heneicosyl, *n*- Docosyl, *n*-Tricosyl, *n*-Tetracosyl, *n*-Pentacosyl, *n*-Hexacosyl, *n*-Heptacosyl, *n*-Octocosyl, *n*-Nonacosyl, *n*-Tricontyl.

Im Sinne der Erfindung ist eine "unverzweigte oder verzweigte Alkylgruppe mit 1 bis 12 Kohlenstoffatomen" insbesondere ausgewählt aus der Gruppe bestehend aus Methyl, Ethyl, *n*-Propyl, *iso*-Propyl, *n*-Butyl, *sec*-Butyl, *iso*-Butyl, *tert*-Butyl, *n*-Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, *n*-Hexyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, *n*-Heptyl, *n*-Octyl, *n*-Nonyl, *n*-Decyl, *n*-Undecyl, *n*-Dodecyl.

Im Sinne der Erfindung ist eine "unverzweigte oder verzweigte Alkylgruppe mit 1 bis 10 Kohlenstoffatomen" insbesondere ausgewählt aus der Gruppe bestehend aus Methyl, Ethyl, *n*-Propyl, *iso*-Propyl, *n*-Butyl, *sec*-Butyl, *iso*-Butyl, *tert*-Butyl, n-Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, *n*-Hexyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, *n*-Heptyl, *n*-Octyl, *n*-Nonyl, *n*-Decyl.

Im Sinne der Erfindung ist eine "unverzweigte oder verzweigte Alkylgruppe mit 1 bis 8 Kohlenstoffatomen" insbesondere ausgewählt aus der Gruppe bestehend aus Methyl, Ethyl, *n*-Propyl, *iso*-Propyl, *n*-Butyl, *sec*-Butyl, *iso*-Butyl, *tert*-Butyl, *n*-Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, *n*-Hexyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, *n*-Heptyl, *n*-Octyl.

Im Sinne der Erfindung ist eine "unverzweigte oder verzweigte Alkylgruppe mit 1 bis 6 Kohlenstoffatomen" insbesondere ausgewählt aus der Gruppe bestehend aus Methyl, Ethyl, *n*-Propyl, *iso*-Propyl, *n*-Butyl, *sec*-Butyl, *iso*-Butyl, *tert*-Butyl, *n*-Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, *n*-Hexyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl.

Der Begriff "unverzweigte oder verzweigte Alkylengruppe" bezeichnet im Sinne der Erfindung einen zweiwertigen gesättigten Kohlenwasserstoffrest, welcher durch die allgemeine chemische Struktur **(b)** mit
**(b)**: 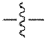 CₓH₂ₓ 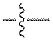 ; beschrieben werden kann. Die Kette an Kohlenstoffatomen"-CₓH₂ₓ" kann dabei linear sein, dann handelt es sich um eine unverzweigte Alkylengruppe. Sie kann aber auch Verzweigungen aufweisen, dann handelt es sich um eine verzweigte Alkylengruppe. Dabei ist x in der chemischen Struktur **(b)** eine ganze Zahl.
   x ist bei einer unverzweigten oder verzweigten Alkylengruppe mit 1 bis 30 Kohlenstoffatomen ausgewählt aus dem Bereich 1 bis 30.
   x ist bei einer unverzweigten oder verzweigten Alkylengruppe mit 1 bis 12 Kohlenstoffatomen ausgewählt aus dem Bereich 1 bis 12.
   x ist bei einer unverzweigten oder verzweigten Alkylengruppe mit 1 bis 6 Kohlenstoffatomen ausgewählt aus dem Bereich 1 bis 6.
   Im Sinne der Erfindung ist eine "zweiwertige gesättigte Kohlenwasserstoffgruppe mit 3 bis 30 Kohlenstoffatomen, welche mindestens einen gesättigten Ring aus 3 bis 30 Kohlenstoffatomen aufweist" insbesondere eine chemische Struktur **(c)** mit
**(c)**: 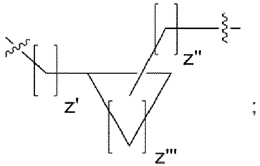 wobei z' eine ganze Zahl zwischen 0 und 27 ist; wobei z" eine ganze Zahl zwischen 0 und 27 ist; wobei z'" eine ganze Zahl zwischen 1 und 28 ist; und wobei gleichzeitig gilt, dass z' + z" + z'" ≤ 28.

Eine "zweiwertige gesättigte Kohlenwasserstoffgruppe mit 3 bis 12 Kohlenstoffatomen, welche mindestens einen gesättigten Ring aus 3 bis 12 Kohlenstoffatomen aufweist" weist im Sinne der Erfindung eine chemische Struktur **(c)** auf, wobei z' eine ganze Zahl zwischen 0 und 9 ist; wobei z" eine ganze Zahl zwischen 0 und 9 ist; wobei z'" eine ganze Zahl zwischen 1 und 10 ist; und wobei gleichzeitig gilt, dass z' + z" + z'" ≤ 10.

Bevorzugt ist eine "zweiwertige gesättigte Kohlenwasserstoffgruppe mit 3 bis 12 Kohlenstoffatomen, welche mindestens einen gesättigten Ring aus 3 bis 12 Kohlenstoffatomen aufweist" ausgewählt aus der Gruppe bestehend aus Cyclopropylen, Cyclobutylen, Cyclopentylen, Cyclohexylen, Cycloheptylen, Cyclooctylen, Cyclononylen, Cyclodecylen, Cycloundecylen, Cyclododecylen.

Eine "zweiwertige gesättigte Kohlenwasserstoffgruppe mit 3 bis 6 Kohlenstoffatomen, welche mindestens einen gesättigten Ring aus 3 bis 6 Kohlenstoffatomen aufweist" weist im Sinne der Erfindung eine chemische Struktur **(c)** auf, wobei z' eine ganze Zahl zwischen 0 und 3 ist; wobei z" eine ganze Zahl zwischen 0 und 3 ist; wobei z'" eine ganze Zahl zwischen 1 und 4 ist; und wobei gleichzeitig gilt, dass z' + z" + z'" ≤ 4.

Bevorzugt ist eine "zweiwertige gesättigte Kohlenwasserstoffgruppe mit 3 bis 6 Kohlenstoffatomen, welche mindestens einen gesättigten Ring aus 3 bis 6 Kohlenstoffatomen aufweist" ausgewählt aus der Gruppe bestehend aus Cyclopropylen, Cyclobutylen, Cyclopentylen, Cyclohexylen.

Im Sinne der Erfindung ist eine "zweiwertige Kohlenwasserstoffgruppe mit 6 bis 30 Kohlenstoffatomen, von denen mindestens 6 Kohlenstoffatome in einem aromatischen System vorliegen und die übrigen Kohlenstoffatome, falls vorhanden, gesättigt sind" insbesondere eine "zweiwertige Kohlenwasserstoffgruppe mit 6 bis 30 Kohlenstoffatomen, von denen 6, 10 oder 14 Kohlenstoffatome in einem aromatischen System vorliegen und die übrigen Kohlenstoffatome, falls vorhanden, gesättigt sind" und bevorzugter ausgewählt aus der Gruppe bestehend aus Naphthylen, Anthrylen, Phenanthrylen sowie der folgenden chemischen Struktur **(d):**
(d): 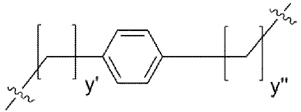 wobei y' eine ganze Zahl zwischen 0 und 24 ist; wobei y" eine ganze Zahl zwischen 0 und 24 ist; und wobei gleichzeitig gilt, dass y' + y" ≤ 24.

Noch bevorzugter handelt es sich dabei um eine "zweiwertige Kohlenwasserstoffgruppe mit 6 bis 30 Kohlenstoffatomen, von denen 6 oder 10 Kohlenstoffatome in einem aromatischen System vorliegen und die übrigen Kohlenstoffatome, falls vorhanden, gesättigt sind" und am bevozugtesten ist diese dann ausgewählt aus der Gruppe bestehend aus Naphthylen, sowie der folgenden chemischen Struktur **(d):**
**(d)**: 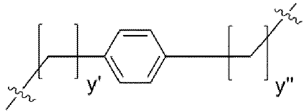 wobei y' eine ganze Zahl zwischen 0 und 24 ist; wobei y" eine ganze Zahl zwischen 0 und 24 ist; und wobei gleichzeitig gilt, dass y' + y" ≤ 24.

Im Sinne der Erfindung ist eine "unverzweigte oder verzweigte Alkoxygruppe" ein organischer Rest der chemischen Struktur 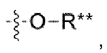 in welchem R** eine unverzweigte oder verzweigte Alkylgruppe ist. Bei einer "unverzweigten oder verzweigten Alkoxygruppe mit 1 bis 30 Kohlenstoffatomen" ist R** eine unverzweigte oder verzweigte Alkylgruppe mit 1 bis 30 Kohlenstoffatomen.

Bei einer "unverzweigten oder verzweigten Alkoxygruppe mit 1 bis 10 Kohlenstoffatomen" ist R** eine unverzweigte oder verzweigte Alkylgruppe mit 1 bis 10 Kohlenstoffatomen.

Im Sinne der Erfindung ist eine "unverzweigte oder verzweigte Alkoxygruppe mit 1 bis 10 Kohlenstoffatomen" insbesondere ausgewählt aus der Gruppe bestehend aus Methoxy, Ethoxy, *n*-Propoxy, *iso*-Propoxy, *n*-Butoxy, *sec*-Butoxy, *iso*-Butoxy, *tert*-Butoxy, *n*-Pentoxy, 1-Methylbutoxy, 2-Methylbutoxy, 3-Methylbutoxy, 1,1-Dimethylpropoxy, 1,2-Dimethylpropoxy, 2,2-Dimethylpropoxy, 1-Ethylpropoxy, *n*-Hexoxy, 1-Methylpentoxy, 2-Methylpentoxy, 3-Methylpentoxy, 4-Methylpentoxy, 1,1-Dimethylbutoxy, 1,2-Dimethylbutoxy, 1,3-Dimethylbutoxy, 2,2-Dimethylbutoxy, 2,3-Dimethylbutoxy, 3,3-Dimethylbutoxy, 1-Ethylbutoxy, 2-Ethylbutoxy, 1,1,2-Trimethylpropoxy, 1,2,2-Trimethylpropoxy, 1-Ethyl-1-methylpropoxy, 1-Ethyl-2-methylpropoxy, *n*-Heptoxy, *n*-Octoxy, *n*-Nonoxy, *n*-Decoxy.

Im Sinne der Erfindung ist eine "unverzweigte oder verzweigte Acylgruppe mit 1 bis 30 Kohlenstoffatomen" ein organischer Rest der chemischen Struktur 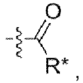 in welchem R* ein unverzweigter oder verzweigter Alkylrest mit 1 bis 29 Kohlenstoffatomen ist.

Insbesondere ist R* ausgewählt aus der Gruppe bestehend aus Methyl, Ethyl, *n*-Propyl, *iso*-Propyl, *n*-Butyl, *sec*-Butyl, *iso*-Butyl, *tert*-Butyl, *n*-Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, *n*-Hexyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, *n*-Heptyl, n-Octyl, *n*-Nonyl, *n*-Decyl, *n*-Undecyl, *n*-Dodecyl, *n*-Tridecyl, *n*-Tetradecyl, *n*-Pentadecyl, *n*-Hexadecyl, *n*-Heptadecyl, *n*-Octadecyl, *n*-Nonadecyl, *n*-Eicosyl, *n*-Heneicosyl, *n*-Docosyl, *n*-Tricosyl, *n*-Tetracosyl, *n*-Pentacosyl, *n*-Hexacosyl, *n*-Heptacosyl, *n*-Octocosyl, *n*-Nonacosyl.

"-O·" bezeichnet im Sinne der Erfindung einen radikalischen Sauerstoffrest.

Im Sinne der Erfindung bedeutet die Formulierung "mindestens ein Wasserstoffrest durch einen Rest ausgewählt aus der Gruppe bestehend
aus -OH, -NH₂, -OCH₃, -OCH₂CH₃, -NH(CH₃), -N(CH₃)₂, -NH(CH₂CH₃), -N(CH₂CH₃)₂,
-N(CH₃)(CH₂CH₃) ersetzt sein kann", dass die betreffende Gruppe unsubstituiert vorliegt oder in der betreffenden Gruppe mindestens ein an ein Kohlenstoffatom gebundener Wasserstoffrest, bevorzugt 1 bis 5, noch bevorzugter 1 bis 3, am bevorzugtesten 1 bis 2 an das selbe oder unterschiedliche Kohlenstoffatom(e) gebundene(r) Wasserstoffrest(e), durch einen Rest ausgewählt aus der Gruppe bestehend
aus -OH, -NH₂, -OCH₃, -OCH₂CH₃, -NH(CH₃), -N(CH₃)₂, -NH(CH₂CH₃), -N(CH₂CH₃)₂,
-N(CH₃)(CH₂CH₃) substituiert ist.

Im Sinne der Erfindung bedeutet die Formulierung "mindestens ein Wasserstoffrest durch einen Rest ausgewählt aus der Gruppe bestehend aus -OH, -OCH₃, -OCH₂CH₃, -N(CH₃)₂, -N(CH₂CH₃)₂, -N(CH₃)(CH₂CH₃) ersetzt sein kann", dass die betreffende Gruppe unsubstituiert vorliegt oder in der betreffenden Gruppe mindestens ein an ein Kohlenstoffatom gebundener Wasserstoffrest, bevorzugt 1 bis 5, noch bevorzugter 1 bis 3, am bevorzugtesten 1 bis 2 an das selbe oder unterschiedliche Kohlenstoffatom(e) gebundene(r) Wasserstoffrest(e), durch einen Rest ausgewählt aus der Gruppe bestehend aus --OH, -OCH₃, -OCH₂CH₃, -N(CH₃)₂, -N(CH₂CH₃)₂, -N(CH₃)(CH₂CH₃) substituiert ist.

### Erfindungsgemäßes Verfahren

Das erfindungsgemäße Verfahren stellt eine reduktive Aminierung dar, in welcher die Carbonylgruppe der Carbonylverbindung **(II)** mit einem Stickstoffatom der Triacetondiaminverbindung **(I)** reagiert. Dabei wird ein Wasserstoffrest einer primären oder sekundären Amingruppe der Triacetondiaminverbindung **(I)** durch den mit dem Sauerstoffatom der Carbonylgruppe verbundenen organischen Rest der Carbonylverbindung **(II)** substituiert. Die *N-*substituierte Triacetondiaminverbindung ist somit das Reaktionsprodukt der reduktiven Aminierung einer primären oder sekundären Amingruppe der Triacetondiaminverbindung **(I)** mit einer Carbonylgruppe der Carbonylverbindung **(II)**.

Durch das erfindungsgemäße Verfahren werden die diskutierten Nachteile der Verfahren des Standes der Technik überraschenderweise gelöst: So wird im erfindungsgemäßen Verfahren von Triacetondiamin bzw. einer Triacetondiaminverbindung ausgegangen. Dadurch vermeidet das erfindungsgemäße Verfahren den Einsatz des instabilen Eduktes Triacetonamin, so dass dessen Vorliegen im resultierenden Produktgemisch von Vorneherein ausgeschlossen ist und die bei den Verfahren des Standes der Technik auftretende mangelhafte Farbstabilität der Produkte drastisch verbessert wird.

Zusätzlich sind über das erfindungsgemäße Verfahren im Vergleich zu den herkömmlichen Verfahren auch höhere Ausbeuten an Triacetondiaminverbindungen möglich. Das erfindungsgemäße Verfahren ermöglicht außerdem auch die Herstellung von Triacetondiaminverbindungen, welche verzweigte Alkylreste am exozyklischen Stickstoffatom tragen - solche Triacetondiaminverbindungen sind mit den Verfahren des Standes der Technik in deutlich schlechteren Ausbeuten erhältlich. Außerdem sind auch am exozyklischen Stickstoffatom disubstituierte Verbindungen mit dem erfindungsgemäßen Verfahren leichter zugänglich.

Beispielsweise würde eine TAD-Verbindung **(I)** der chemischen Struktur **(I-A)** mit einer Carbonylverbindung **(II)** der Struktur **(II-A)** im erfindungsgemäßen Verfahren, bei äquimolarem Einsatz von **(I-A)** und **(II-A),** wie folgt reagieren:

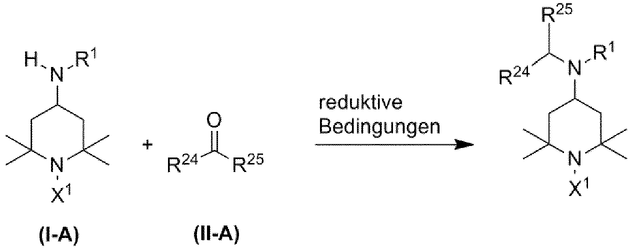

Es versteht sich von selbst, dass bei einem molaren Überschuss an Carbonylverbindung **(II)** in Bezug auf Triacetondiaminverbindung **(I)** (falls diese mehrere aminische Gruppen aufweist), auch weitere primäre und/oder sekundäre Amingruppen dieser Triacetondiaminverbindung **(I)** eine reduktive Aminierung in der Reihenfolge gemäß ihrer Reaktivität durchlaufen können oder eine primäre Amingruppe der Triacetondiaminverbindung **(I)** diese zweifach durchlaufen kann.

Ebenso versteht es sich von selbst, dass bei einem molaren Überschuss an Triacetondiaminverbindung **(I)** in Bezug auf Carbonylverbindung **(II)** [falls diese mehrere Carbonylgruppen aufweist, wie es zum Beispiel bei den chemischen Strukturen **(II-B)** oder **(II-C)** der Fall ist], auch weitere Carbonylgruppen der Carbonylverbindung **(II)** in der Reihenfolge gemäß ihrer Reaktivität eine reduktive Aminierung durchlaufen würden.

Eine noch gezieltere Substitution von Wasserstoffatomen an der 4-Amino-Gruppe des 2,2,6,6-Tetramethylpiperidinylrestes der Triacetondiaminverbindung **(I)** ist möglich, wenn man das erfindungsgemäße Verfahren wie im achten Aspekt beschrieben durchführt.

Das erfindungsgemäße Verfahren kann man lösungsmittelfrei oder auch in mindestens einem Lösungsmittel durchführen, bevorzugt in mindestens einem Lösungsmittel. Als Lösungsmittel eignen sich dabei alle Lösungsmittel, in denen sich die Reaktanden gut lösen und welche auch keinen störenden Einfluss auf das erfindungsgemäße Verfahren haben. Insbesondere ist das Lösungsmittel ausgewählt aus der Gruppe bestehend aus aliphatische Lösungsmittel, aromatische Lösungsmittel, Ether, halogenierte Lösungsmittel, Amide, Thioverbindungen, Carbonsäuren, Alkohole, Wasser; bevorzugt ist das Lösungsmittel ausgewählt aus der Gruppe bestehend aus aliphatische Lösungsmittel, aromatische Lösungsmittel, Ether, Alkohole, Wasser; bevorzugter ist das Lösungsmittel ausgewählt aus der Gruppe bestehend aus Ether, Alkohole, Wasser; noch bevorzugter ist das Lösungsmittel ausgewählt aus der Gruppe bestehend aus Alkohole (zum Beispiel Methanol), Wasser.

Aliphatische Lösungsmittel sind insbesondere ausgewählt aus der Gruppe bestehend aus Pentan, Hexan, Heptan, Octan, Decan, Cyclopentan, Cyclohexan, Methylcyclohexan, Petrolether.

Aromatische Lösungsmittel sind insbesondere ausgewählt aus der Gruppe bestehend aus Benzol, Toluol, Xylol, Ethylbenzol, Cumol, Brombenzol, Chlorbenzol, Dichlorbenzol, Furan.

Ether sind insbesondere ausgewählt aus der Gruppe bestehend aus Diethylether, Dipropylether, Dibutylether, Methyl-*tert*-Butylether, Ethylenglykolmonomethylether, Ethylenglykolmonoethylether, Ethylenglykoldimethylether, Ethylenglykoldiethylether, Diethylenglykolmonomethylether, Diethylenglykolmonoethylether, Diethylenglykoldimethylether, Diethylenglykoldiethylether, Triethylenglykolmonomethylether, Triethylenglykolmonoethylether, Triethylenglykoldimethylether, Triethylenglykoldiethylether, Polyethylenglykolmonomethylether, Polyethylenglykolmonoethylether, Polyethylenglykoldimethylether, Polyethylenglykoldiethylether, 1,4-Dioxan, 1,3-Dioxan, Tetrahydrofuran.

Halogenierte Lösungsmittel sind insbesondere ausgewählt aus der Gruppe bestehend aus Dichlormethan, Chloroform, Tetrachlormethan.

Amide sind insbesondere ausgewählt aus der Gruppe bestehend aus Dimethylformamid, Dimethylacetamid.

Thioverbindungen sind insbesondere ausgewählt aus der Gruppe bestehend aus Dimethylsulfoxid, Sulfolan.

Carbonsäuren sind insbesondere ausgewählt aus der Gruppe bestehend aus Ameisensäure, Essigsäure, Propionsäure, Butansäure, Pentansäure.

Alkohole, sind insbesondere ausgewählt aus der Gruppe bestehend aus Methanol, Ethanol, Propanol, *iso*-Propanol, 1,2-Propandiol, 1,3-Propandiol, Glycerin, Butanol, *sec*-Butanol, *iso*-Butanol, *tert*-Butanol, 1,2-Butandiol, 1,3-Butandiol, 1,4-Butandiol, 1-Pentanol, 2-Pentanol, 3-Pentanol, *tert*-Amylalkohol, 1,2-Pentandiol, 1,3-Pentandiol, 1,4-Pentandiol, 1,5-Pentandiol, Cyclopentanol, Hexanol, Cyclohexanol Heptanol, Octanol, Nonanol, Decanol, Ethylenglykol, Diethylenglykol, Triethylenglykol, Polyethylenglykol, Benzylalkohol, Phenol; bevorzugt ausgewählt aus Methanol, Ethanol, *n*-Propanol, *iso*-Propanol.

Das Verhältnis der molaren Mengen an eingesetzten Reaktanden ist grundsätzlich nicht beschränkt und hängt davon ab, an wie vielen Stickstoffatomen des TAD-Derivates **(I)** wie viele Alkylreste eingeführt werden sollen.

Beispielsweise kann man bei Umsetzung von TAD mit einer Carbonylverbindung **(II)** der chemischen Struktur **(II-A)** eine, bezogen auf die zu substituierenden NH-Funktionen, äquimolare Menge von **(II-A)** einsetzen.

Im Falle der Umsetzung von TAD (wenn die exozyklische 4-Aminogruppe substituiert werden soll) mit einer Carbonylverbindung **(II)** der chemischen Struktur **(II-A)** ergäbe sich dann folgende Reaktion:

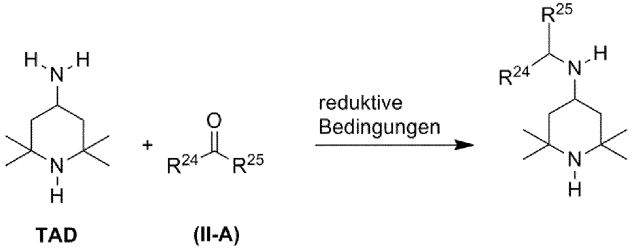

Im Falle einer Umsetzung eines Triacetondiaminverbindung **(I)** mit einer Carbonylverbindung **(II)** der chemischen Struktur **(II-A)** werden bevorzugt 0.8 bis 2.0 molare Äquivalente der Verbindung **(II-A)** pro zu substituierender NH-Funktion der Triacetondiaminverbindung **(I)** eingesetzt; bevorzugter 0.8 bis 1.5 molare Äquivalente, noch bevorzugter 0.8 bis 1.3 molare Äquivalente, am bevorzugtesten 0.9 bis 1.2 molare Äquivalente der Verbindung **(II-A)** pro zu substituierender NH-Funktion der Triacetondiaminverbindung **(I)** eingesetzt.

Im Falle einer Umsetzung eines Triacetondiaminverbindung **(I)** mit einer Carbonylverbindung **(II)** der chemischen Struktur **(II-B)** werden bevorzugt 0.4 bis 1.0 molare Äquivalente der Verbindung **(II-B)** pro zu substituierender NH-Funktion der Triacetondiaminverbindung **(I)** eingesetzt; bevorzugter 0.4 bis 0.75 molare Äquivalente, noch bevorzugter 0.4 bis 0.65 molare Äquivalente, am bevorzugtesten 0.45 bis 0.6 molare Äquivalente der Verbindung **(II-B)** pro zu substituierender NH-Funktion der Triacetondiaminverbindung **(I)** eingesetzt.

Im Falle einer Umsetzung eines Triacetondiaminverbindung **(I)** mit einer Carbonylverbindung **(II)** der chemischen Struktur **(II-C)** werden bevorzugt 0.27 bis 0.67 molare Äquivalente der Verbindung **(II-C)** pro zu substituierender NH-Funktion der Triacetondiaminverbindung **(I)** eingesetzt; bevorzugter 0.27 bis 0.5 molare Äquivalente, noch bevorzugter 0.27 bis 0.43 molare Äquivalente, am bevorzugtesten 0.3 bis 0.4 molare Äquivalente der Verbindung **(II-C)** pro zu substituierender NH-Funktion der Triacetondiaminverbindung **(I)** eingesetzt.

Das erfindungsgemäße Verfahren wird unter reduktiven Bedingungen durchgeführt. Unter "reduktiven Bedingungen" sind die Bedingungen zu verstehen, bei welchen das im Reaktionsschema **<1>** gezeigte Imin durch Wasserstoffanlagerung in das entsprechende Amin überführt wird.

Man stellt im erfindungsgemäßen Verfahren reduktive Bedingungen dadurch ein, dass man die mindestens eine Triacetondiaminverbindung **(I)** mit der mindestens einen Carbonylverbindung **(II)** in Gegenwart von Wasserstoff und in Gegenwart eines Vollkatalysators umsetzt, wobei der Vollkatalysator mindestens ein Metall **M** aufweist, wobei das Metall **M** ausgewählt ist aus der Gruppe bestehend aus Ag, V, Cr, Mo, Mn, Ni, Pd, Pt, Fe, Ru, Os, Co, Rh, Ir, Cu; insbesondere ausgewählt ist aus der Gruppe bestehend aus Ag, Fe, Cr, Mo, Mn, Ni, Co, Cu, Pd, Pt, Ru, Rh; bevorzugt ausgewählt ist aus der Gruppe bestehend aus Ag, Fe, Cr, Ni, Co, Cu, Pd, Pt; bevorzugter ausgewählt ist aus der Gruppe bestehend aus Ag, Fe, Ni, Co, Cu, Pd, Pt; noch bevorzugter ausgewählt ist aus der Gruppe bestehend aus Ag, Fe, Ni, Co, Cu; noch mehr bevorzugter ausgewählt ist aus der Gruppe bestehend Co, Ni; am bevorzugtesten Ni ist.

Die Verwendung eines Vollkatalysators aufweisend mindestens ein Metall **M** ist wesentlich für das erfindungsgemäße Verfahren.

Ein "Vollkatalysator" ist dem Fachmann bekannt und ist ein vollständig von dem katalytischen Material durchdrungener Formkörper. Er unterscheidet sich damit vom "Trägerkatalysator", bei dem die katalytisch aktive Komponente auf einem von der katalytisch aktiven Komponente verschiedenen Träger aufgebracht ist.

Das mindestens eine Metall **M** im Vollkatalysator liegt insbesondere im elementaren Zustand oder als Verbindung des Metalls **M,** beispielsweise als Oxid, Sulfid, vor, bevorzugt jedoch im elementaren Zustand.

Bevorzugt handelt es sich bei dem Vollkatalysator aufweisend mindestens ein Metall **M** um einen Vollkatalysator aufweisend ein Metall **M** ausgewählt aus Ag, Fe, Ni, Co, Cu, insbesondere Ni, Co, bevorzugt Ni.

Der Vollkatalysator kann eine Legierung des Metalls **M** sein (wobei das Metall **M** zu mindestens > 50 Gew.-% in der Legierung vorliegt, bezogen auf das Gesamtgewicht der Legierung) und beispielsweise außer **M** noch mindestens ein Metall bzw. Halbmetall ausgewählt aus Al, Si, Mg, Zn, Mo, Cr insbesondere Al, aufweisen.

Noch mehr bevorzugter ist der Vollkatalysator aufweisend mindestens ein Metall **M** ausgewählt aus der Gruppe bestehend aus Raney-Kobalt, Raney-Kupfer, Raney-Silber, Raney-Eisen, Raney-Nickel, insbesondere ausgewählt aus Raney-Nickel, Raney-Kobalt, am bevorzugtesten ausgewählt aus Raney-Nickel.

Im Raney-Nickel ist der Anteil des Nickels bezogen auf das Gesamtgewicht des Raney-Nickels insbesondere mindestens > 50 Gew.-%, bevorzugt mindestens 60 Gew.-%, bevorzugter mindestens 70 Gew.-%, noch bevorzugter mindestens 80 Gew.-%, noch mehr bevorzugter mindestens 85 Gew.-%, noch viel mehr bevorzugter mindestens 90 Gew.-%, wobei das Raney-Nickel insbesondere daneben noch weitere von Nickel verschiedene Metalle und/oder Halbmetalle (wie beispielsweise Al, Mo, Si, Mg, Zn, Mo, Cr) in einer solchen Menge aufweist, dass die Summe der Gewichte aus Nickel und den übrigen Metallen und Halbmetallen 100 Gew.-% ergibt. Das Raney-Nickel kann insbesondere mit Zn, Mo, Cr, bevorzugt Mo dotiert sein, um die katalytischen Eigenschaften zu verbessern.

Im Raney-Kobalt ist der Anteil des Kobalts bezogen auf das Gesamtgewicht des Raney-Kobalts insbesondere mindestens > 50 Gew.-%, bevorzugt mindestens 60 Gew.-%, bevorzugter mindestens 70 Gew.-%, noch bevorzugter mindestens 80 Gew.-%, noch mehr bevorzugter mindestens 85 Gew.-%, noch viel mehr bevorzugter mindestens 90 Gew.-%, wobei das Raney-Kobalt insbesondere daneben noch weitere von Kobalt verschiedene Metalle und/oder Halbmetalle (wie beispielsweise Al, Mo, Si, Mg, Zn, Mo, Cr) in einer solchen Menge aufweist, dass die Summe der Gewichte aus Kobalt und den übrigen Metallen und Halbmetallen 100 Gew.-% ergibt. Das Raney-Kobalt kann insbesondere mit Zn, Mo, Cr, bevorzugt Mo dotiert sein, um die katalytischen Eigenschaften zu verbessern.

Im Raney-Kupfer ist der Anteil des Kupfers bezogen auf das Gesamtgewicht des Raney-Kupfers insbesondere mindestens > 50 Gew.-%, bevorzugt mindestens 60 Gew.-%, bevorzugter mindestens 70 Gew.-%, noch bevorzugter mindestens 80 Gew.-%, noch mehr bevorzugter mindestens 85 Gew.-%, noch viel mehr bevorzugter mindestens 90 Gew.-%, wobei das Raney-Kupfer insbesondere daneben noch weitere von Kupfer verschiedene Metalle und/oder Halbmetalle (wie beispielsweise Al, Mo, Si, Mg, Zn, Mo, Cr) in einer solchen Menge aufweist, dass die Summe der Gewichte aus Kupfer und den übrigen Metallen und Halbmetallen 100 Gew.-% ergibt. Das Raney-Kupfer kann insbesondere mit Zn, Mo, Cr, bevorzugt Mo dotiert sein, um die katalytischen Eigenschaften zu verbessern.

Im Raney-Silber ist der Anteil des Silbers bezogen auf das Gesamtgewicht des Raney-Silbers insbesondere mindestens > 50 Gew.-%, bevorzugt mindestens 60 Gew.-%, bevorzugter mindestens 70 Gew.-%, noch bevorzugter mindestens 80 Gew.-%, noch mehr bevorzugter mindestens 85 Gew.-%, noch viel mehr bevorzugter mindestens 90 Gew.-%, wobei das Raney-Silber insbesondere daneben noch weitere von Silber verschiedene Metalle und/oder Halbmetalle (wie beispielsweise Al, Mo, Si, Mg, Zn, Mo, Cr) in einer solchen Menge aufweist, dass die Summe der Gewichte aus Silber und den übrigen Metallen und Halbmetallen 100 Gew.-% ergibt. Das Raney-Silber kann insbesondere mit Zn, Mo, Cr, bevorzugt Mo dotiert sein, um die katalytischen Eigenschaften zu verbessern.

Im Raney-Eisen ist der Anteil des Eisens bezogen auf das Gesamtgewicht des Raney-Eisens insbesondere mindestens > 50 Gew.-%, bevorzugt mindestens 60 Gew.-%, bevorzugter mindestens 70 Gew.-%, noch bevorzugter mindestens 80 Gew.-%, noch mehr bevorzugter mindestens 85 Gew.-%, noch viel mehr bevorzugter mindestens 90 Gew.-%, wobei das Raney-Eisen insbesondere daneben noch weitere von Eisen verschiedene Metalle und/oder Halbmetalle (wie beispielsweise Al, Mo, Si, Mg, Zn, Mo, Cr) in einer solchen Menge aufweist, dass die Summe der Gewichte aus Eisen und den übrigen Metallen und Halbmetallen 100 Gew.-% ergibt. Das Raney-Eisen kann insbesondere mit Zn, Mo, Cr, bevorzugt Mo dotiert sein, um die katalytischen Eigenschaften zu verbessern.

Die Herstellung der erfindungsgemäßen Vollkatalysatoren ist dem Fachmann bekannt. Die Herstellung des Raney-Nickels ist zum Beispiel in den US 1,629,190, DE 20 2010 007837 U1 beschrieben. Dazu legiert man Ni mit Al, Si, Mg oder Zn (insbesondere mit Al, bevorzugt im Verhältnis 1:1) und löst aus der Legierung nach mechanischer Zerkleinerung mit Alkalien (insbesondere NaOH) das katalytisch unwirksame Metall (AI) mindestens teilweise heraus.

Entsprechend werden auch Raney-Kupfer, Raney-Kobalt, Raney Silber oder Raney-Eisen hergestellt (beschrieben zum Beispiel in der DE 20 2010 007837 U1).

Ohne einen solchen Vollkatalysator würde man nur unerwünschte Produkte erhalten. C. Harries beschreibt zum Beispiel auf Seiten 220 bis 222 seines Artikels "Untersuchungen über die cyclischen Acetonbasen" in Justus Liebigs Annalen der Chemie, Band 417, 1918, Seiten 107 bis 191 eine Umsetzung von 4-Amino-2,2,6,6-Tetramethylaminopiperidin mit Acetanhydrid ohne Vollkatalysator (oder Trägerkatalysator), was zu hohen Ausbeuten an der entsprechenden, hier unerwünschten Amidverbindung führt.

Das erfindungsgemäße Verfahren lässt sich kontinuierlich oder nicht kontinuierlich, also batchweise, durchführen.

Die Reaktionszeit hängt vom Fortgang des Verfahrens und vom gewünschten Umsatz ab - üblicherweise strebt man einen größtmöglichen Umsatz an und führt das erfindungsgemäße Verfahren so lange fort, bis kein Eduktumsatz mehr beobachtet werden kann.

Die Temperatur beim erfindungsgemäßen Verfahren ist nicht beschränkt und liegt bevorzugt im Bereich von 20 °C bis 350 °C, bevorzugter im Bereich von 50 °C bis 300 °C, noch bevorzugter im Bereich von 50 °C bis 250 °C, am bevorzugtesten im Bereich von 70 °C bis 200 °C.

Der Druck beim erfindungsgemäßen Verfahren ist nicht beschränkt und liegt bevorzugt im Bereich von 2 bar bis 500 bar, bevorzugter im Bereich von 5 bar bis 350 bar, noch bevorzugter im Bereich von 25 bar bis 300 bar.

Die obigen Temperaturbereiche und Druckbereiche können natürlich auch in Kombination vorliegen. So kann man das Verfahren bevorzugt bei einer Temperatur im Bereich von 20 °C bis 350 °C, [bevorzugter im Bereich von 50 °C bis 300 °C, noch bevorzugter im Bereich von 50 °C bis 250 °C, am bevorzugtesten im Bereich von 70 °C bis 200 °C] und einem Druck im Bereich von 2 bar bis 500 bar [bevorzugt im Bereich von 2 bar bis 500 bar, bevorzugter im Bereich von 5 bar bis 350 bar, noch bevorzugter im Bereich von 25 bar bis 300 bar] durchführen.

Das erfindungsgemäße Verfahren löst die obige Aufgabe in völlig überraschender Weise: Die mit dem erfindungsgemäßen Verfahren hergestellten Produkte weisen eine höhere Farbstabilität auf als die mit dem Verfahren des Standes der Technik gewonnenen TAD-Verbindungen. Dies lässt sich daran überprüfen, dass die mit dem erfindungsgemäßen Verfahren erhaltenen *N*-substituierten TAD-Verbindungen sowohl direkt nach der Herstellung als auch insbesondere nach Lagerung eine geringere Hazen-Farbzahl aufweisen als die gleichen, mit den herkömmlichen Verfahren synthetisierten TAD-Verbindungen. Die Hazen-Farbzahl kann wie in der DIN EN ISO 6271 (2005) beschrieben bestimmt werden.

### Beispiele

### Erfindungsgemäße Beispiele E1 - E7:

Ein 100 ml-Druckautoklav wird mittels eines Thermostaten auf 60 °C vorgeheizt. Dann werden 25 ml Toluol und 39 g (0.25 mol) Triacetondiamin (TAD) in den Reaktor gegeben und verrührt. Im Anschluss werden 18 g (0.25 mol) Butanal zugegeben. Der Katalysator (0.2 mol-% bezogen auf TAD) wird zugegeben und der Reaktor wird verschlossen. Als Katalysatoren werden Raney-Nickel (**E1**), Ru (**E2**), Pt (**E3**), Rh (**E4**), Pd (**E5**), Ir (**E6**) bzw. Co (**E7**) genommen. In den Versuchen **E2** bis **E7** handelt es sich um pulverförmige Katalysatoren. Sämtliche Katalysatoren sind von Sigma Aldrich oder Strem erhältlich.

Unter Rühren wird Wasserstoff aufgedrückt (10 bar H₂) und die Autoklaveninnentemperatur innerhalb von 1 Stunde von 60 °C bis 90 °C erhöht, danach 3 Stunden bei 90 °C gehalten. Im Anschluss daran wird nochmal 1 Stunde lang bei 120 °C umgesetzt.

Der Reaktor wird danach abgekühlt und entspannt. Das Rohprodukt wird ausgetragen, filtriert und anschließend zunächst das Lösungsmittel entfernt (80 - 120 °C, 400 mbar). Der Rückstand wird anschließend mittels einer Vakuumdestillation unter Verwendung einer 0.5 m Füllkörperkolonne gereinigt. Die Bestimmung der Reinheit des destillierten Produktes erfolgt gaschromatographisch (z.B. mit Agilent 5890 bzw. 7890, FID-Detektor).

Die Ausbeute an *n*-Butyl-TAD beträgt 9 % (**E1**), 8 % (**E2**), 15 % (**E3**), 10 % (**E4**), 11 % (**E5**), 4 % (**E6**) und 6 % (**E7**).

### Vergleichsbeispiele V1 - V7:

Die erfindungsgemäßen Beispiele **E1** - **E7** werden wiederholt, außer dass statt TAD Triacetonamin (TAA) und statt Butanal Butylamin verwendet wird.

Die Ausbeute an *n*-Butyl-TAD entspricht der in **E1 - E7.**

Die in **V1 - V7** und **E1** - **E7** erhaltenen Produkte werden nach Lagerung in verschlossenen Probenfläschchen untersucht, in dem die Hazen-Farbzahl überprüft wird. Dabei werden folgende Intervalle verwendet:
a) nach Destillation;
b) nach Lagerung bei 70°C für 7 Tage;
c) nach Lagerung bei Raumtemperatur für 30 Tage;
d) nach Lagerung bei Raumtemperatur für 6 Monate.

### Ergebnisse

Beim Vergleich der Vergleichsbeispiele **V1 - V7** mit den erfindungsgemäßen Beispielen **E1** - **E7** zeigt sich folgender überraschender Effekt:
- Anhand des Vergleichs von **V1** mit **E1, V2** mit **E2, V3** mit **E3, V4** mit **E4, V5** mit **E5, V6** mit **E6,** bzw. **V7** mit **E7** zeigt sich, dass die Farbstabilität der in den Versuchen **E1** - **E7** erhaltenen Triacetondiaminverbindungen deutlich gegenüber den gleichen mit **V1** - **V7** erhaltenen Triacetondiaminverbindungen erhöht ist (erkennbar an den in **E1** - **E7** gegenüber **V1** - **V7** kleineren Farbzahlen).

## Patentansprüche

1. Verfahren zur Herstellung einer *N*-substituierten Triacetondiaminverbindung,
**dadurch gekennzeichnet, dass** man mindestens eine Triacetondiaminverbindung **(I)** mit mindestens einer Carbonylverbindung **(II)** unter reduktiven Bedingungen umsetzt,
wobei die Triacetondiaminverbindung **(I)** ausgewählt ist aus der Gruppe bestehend aus den chemischen Strukturen **(I-A), (I-B), (I-C), (I-D), (I-E)** mit 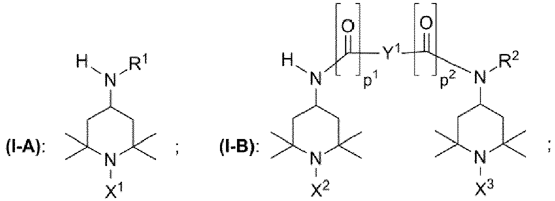 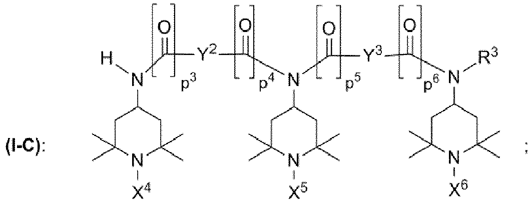 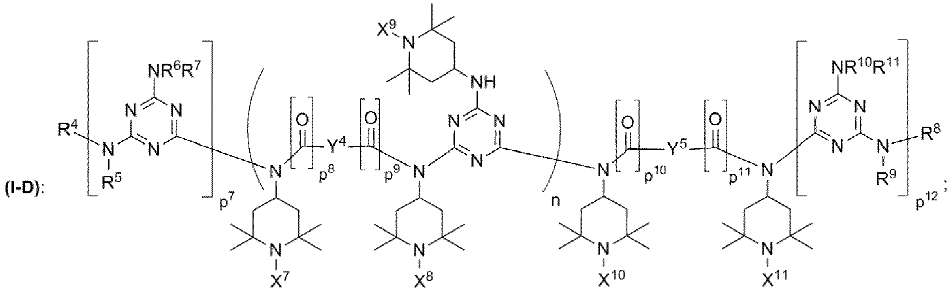 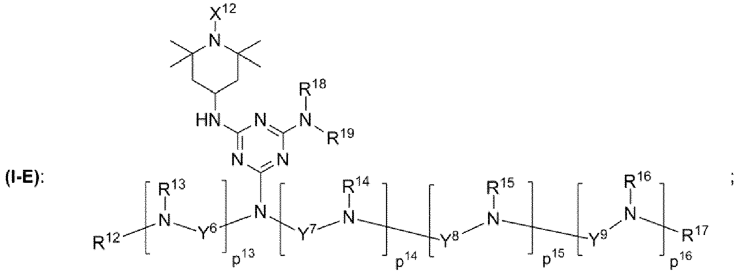
wobei n eine ganze Zahl aus dem Bereich 1 bis 20 ist;
wobei p¹, p², p³, p⁴, p⁵, p⁶, p⁷, p⁸, p⁹, p¹⁰, p¹¹, p¹², p¹³, p¹⁴, p¹⁵, p¹⁶ unabhängig voneinander jeweils 0 oder 1 ist;
wobei X¹, X², X³, X⁴, X⁵, X⁶, X⁷, X⁸, X⁹, X¹⁰, X¹¹, X¹² unabhängig voneinander jeweils ausgewählt ist aus der Gruppe bestehend aus Wasserstoff, OH, -O·, unverzweigte oder verzweigte Alkylgruppe mit 1 bis 10 Kohlenstoffatomen, unverzweigte oder verzweigte Alkoxygruppe mit 1 bis 10 Kohlenstoffatomen;
wobei Y¹, Y², Y³, Y⁴, Y⁵, Y⁶, Y⁷, Y⁸, Y⁹ unabhängig voneinander jeweils ausgewählt ist aus der Gruppe bestehend aus
unverzweigte oder verzweigte Alkylengruppe mit 1 bis 30 Kohlenstoffatomen,
zweiwertige gesättigte Kohlenwasserstoffgruppe mit 3 bis 30 Kohlenstoffatomen, welche mindestens einen gesättigten Ring aus 3 bis 30 Kohlenstoffatomen aufweist,
zweiwertige Kohlenwasserstoffgruppe mit 6 bis 30 Kohlenstoffatomen, von denen mindestens 6 Kohlenstoffatome in einem aromatischen System vorliegen und die übrigen Kohlenstoffatome, falls vorhanden, gesättigt sind,
ein verbrückender Rest, der eine chemische Struktur ausgewählt aus der Gruppe bestehend aus **(i), (ii)** mit 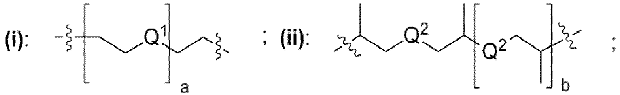
aufweist, wobei
Q¹, Q² unabhängig voneinander jeweils ausgewählt aus der Gruppe bestehend aus -O-, -S-, -NH- oder -NR'- mit R' = unverzweigte oder verzweigte Alkylgruppe mit 1 bis 6 Kohlenstoffatomen sind,
wobei a eine ganze Zahl ausgewählt aus dem Bereich 1 bis 50 ist,
wobei b eine ganze Zahl ausgewählt aus dem Bereich 0 bis 50 ist,
und wobei Y¹ auch eine direkte Bindung sein kann, falls mindestens einer von p¹ und p² den Wert 1 aufweist,
und wobei Y² auch eine direkte Bindung sein kann, falls mindestens einer von p³ und p⁴ den Wert 1 aufweist,
und wobei Y³ auch eine direkte Bindung sein kann, falls mindestens einer von p⁵ und p⁶ den Wert 1 aufweist,
und wobei Y⁴ auch eine direkte Bindung sein kann, falls mindestens einer von p⁸ und p⁹ den Wert 1 aufweist,
und wobei Y⁵ auch eine direkte Bindung sein kann, falls mindestens einer von p¹⁰ und p¹¹ den Wert 1 aufweist;
wobei die Reste R¹, R², R³, R⁴, R⁵, R⁶, R⁷, R⁸, R⁹, R¹⁰, R¹¹ unabhängig voneinander jeweils ausgewählt sind aus der Gruppe bestehend aus
Wasserstoff,
unverzweigte oder verzweigte Alkylgruppe mit 1 bis 30 Kohlenstoffatomen, bei welcher mindestens ein Wasserstoffrest durch einen Rest ausgewählt aus der Gruppe bestehend aus -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann,
unverzweigte oder verzweigte Acylgruppe mit 1 bis 30 Kohlenstoffatomen, bei welcher mindestens ein Wasserstoffrest durch einen Rest ausgewählt aus der Gruppe bestehend aus -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann,
einem Rest, der eine chemischen Struktur ausgewählt aus der Gruppe bestehend aus **(iii), (iv), (v), (vi), (vii), (viii), (ix)** mit 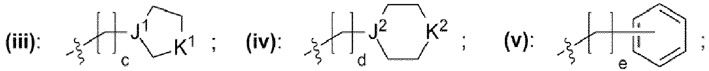 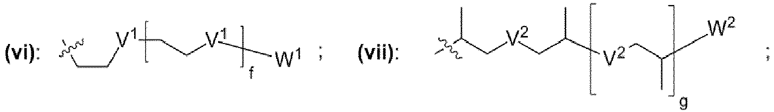 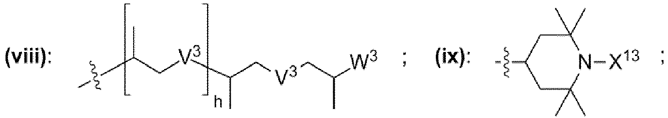
aufweist,
wobei J¹, J² unabhängig voneinander jeweils ausgewählt aus der Gruppe bestehend aus CH, N sind,
wobei K¹, K² unabhängig voneinander jeweils ausgewählt aus der Gruppe bestehend aus -O-, -NH-, -N(CH₃)-, -N(CH₂CH₃)-, -S-, -CH₂- sind,
wobei V¹, V², V³ unabhängig voneinander jeweils ausgewählt aus der Gruppe bestehend aus -O-, -S-, -NH-, -NR"- mit R" = unverzweigte oder verzweigte Alkylgruppe mit 1 bis 6 Kohlenstoffatomen sind,
wobei W¹, W², W³ unabhängig voneinander jeweils ausgewählt aus der Gruppe bestehend aus H, Methyl, Ethyl sind,
wobei c, d, e, f, g, h unabhängig voneinander jeweils eine ganze Zahl aus dem Bereich 0 bis 50 ist,
wobei X¹³ ausgewählt ist aus der Gruppe bestehend aus Wasserstoff, -OH, -O·, unverzweigte oder verzweigte Alkylgruppe mit 1 bis 10 Kohlenstoffatomen, unverzweigte oder verzweigte Alkoxygruppe mit 1 bis 10 Kohlenstoffatomen,
wobei in den chemischen Strukturen **(iii), (iv), (v), (vi), (vii), (viii), (ix)** mindestens ein an ein Kohlenstoffatom gebundener Wasserstoffrest durch einen Rest ausgewählt aus der Gruppe bestehend aus
-OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann;
wobei die Reste R¹², R¹³, R¹⁴, R¹⁵, R¹⁶, R¹⁷ unabhängig voneinander jeweils ausgewählt sind aus der Gruppe bestehend aus
Wasserstoff,
unverzweigte oder verzweigte Alkylgruppe mit 1 bis 30 Kohlenstoffatomen,
einer Gruppe mit der chemischen Struktur **(x)** mit 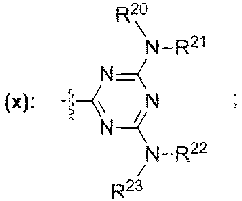
wobei die Reste R¹⁸, R¹⁹, R²⁰, R²¹, R²², R²³ unabhängig voneinander jeweils ausgewählt sind aus der Gruppe bestehend aus
Wasserstoff,
unverzweigte oder verzweigte Alkylgruppe mit 1 bis 30 Kohlenstoffatomen, bei welcher mindestens ein Wasserstoffrest durch einen Rest ausgewählt aus der Gruppe bestehend aus -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann,
unverzweigte oder verzweigte Acylgruppe mit 1 bis 30 Kohlenstoffatomen, bei welcher mindestens ein Wasserstoffrest durch einen Rest ausgewählt aus der Gruppe bestehend aus -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann,
einem Rest, der eine chemischen Struktur ausgewählt aus der Gruppe bestehend aus **(xi), (xii), (xiii), (xiv), (xv), (xvi), (xvii)** mit 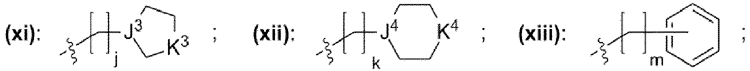 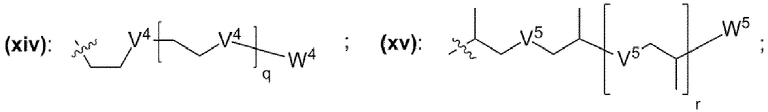 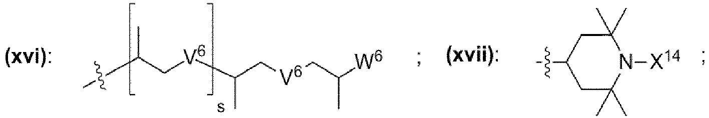
aufweist,
wobei J³, J⁴ unabhängig voneinander jeweils ausgewählt aus der Gruppe bestehend aus CH, N sind,
wobei K³, K⁴ unabhängig voneinander jeweils ausgewählt aus der Gruppe bestehend aus -O-, -NH-, -N(CH₃)-, -N(CH₂CH₃)-, -S-, -CH₂- sind,
wobei V4, V⁵, V⁶ unabhängig voneinander jeweils ausgewählt aus der Gruppe bestehend aus -O-, -S-, -NH-, -NR"'- mit R'" = unverzweigte oder verzweigte Alkylgruppe mit 1 bis 6 Kohlenstoffatomen sind,
wobei W4, W⁵, W⁶ unabhängig voneinander jeweils ausgewählt aus der Gruppe bestehend aus H, Methyl, Ethyl sind,
wobei j, k, m, q, r, s unabhängig voneinander jeweils eine ganze Zahl aus dem Bereich 0 bis 50 ist,
wobei X¹⁴ ausgewählt ist aus der Gruppe bestehend aus Wasserstoff, -OH, -O·, unverzweigte oder verzweigte Alkylgruppe mit 1 bis 10 Kohlenstoffatomen, unverzweigte oder verzweigte Alkoxygruppe mit 1 bis 10 Kohlenstoffatomen,
wobei in den chemischen Strukturen **(xi), (xii), (xiii), (xiv), (xv), (xvi), (xvii)** mindestens ein an ein Kohlenstoffatom gebundener Wasserstoffrest durch einen Rest ausgewählt aus der Gruppe bestehend
aus -OH, -NH₂, -OCH₃, -OCH₂CH₃, -NH(CH₃), -N(CH₃)₂, -NH(CH₂CH₃),-N(CH₂CH₃) 2,
-N(CH₃)(CH₂CH₃) ersetzt sein kann,
und mit der Maßgabe, dass R¹² und R¹⁷ für p¹³ = p¹⁴ = p¹⁵ = p¹⁶ = 0 unabhängig voneinander jeweils auch eine Gruppe der chemischen Struktur **(xviii)** mit 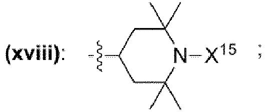
sein können, wobei X¹⁵ ausgewählt ist aus der Gruppe bestehend aus Wasserstoff, OH, -O·, unverzweigte oder verzweigte Alkylgruppe mit 1 bis 10 Kohlenstoffatomen, unverzweigte oder verzweigte Alkoxygruppe mit 1 bis 10 Kohlenstoffatomen;
und wobei die Carbonylverbindung **(II)** ausgewählt ist aus der Gruppe bestehend aus den chemischen Strukturen **(II-A), (II-B), (II-C)** mit 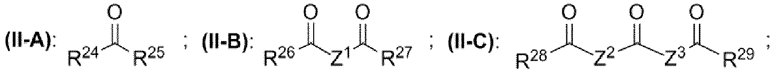
wobei Z¹, Z², Z³ unabhängig voneinander jeweils ausgewählt sind aus der Gruppe bestehend aus
direkte Bindung,
unverzweigte oder verzweigte Alkylengruppe mit 1 bis 30 Kohlenstoffatomen,
zweiwertige gesättigte Kohlenwasserstoffgruppe mit 3 bis 30 Kohlenstoffatomen, welche mindestens einen gesättigten Ring aus 3 bis 30 Kohlenstoffatomen aufweist,
zweiwertige Kohlenwasserstoffgruppe mit 6 bis 30 Kohlenstoffatomen, von denen mindestens 6 Kohlenstoffatome in einem aromatischen System vorliegen und die übrigen Kohlenstoffatome, falls vorhanden, gesättigt sind,
ein verbrückender Rest, der eine chemischen Struktur ausgewählt aus der Gruppe bestehend aus **(xix), (xx)** mit 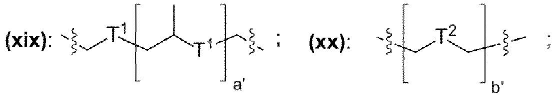
aufweist, wobei
T¹, T² unabhängig voneinander jeweils ausgewählt aus der Gruppe bestehend aus -O-, -S- oder -NR""- mit R"" = unverzweigte oder verzweigte Alkylgruppe mit 1 bis 6 Kohlenstoffatomen sind,
wobei a' und b' unabhängig voneinander jeweils eine ganze Zahl ausgewählt aus dem Bereich 1 bis 50 ist;
und wobei die Reste R²⁴, R²⁵, R²⁶, R²⁷, R²⁸, R²⁹ ausgewählt sind aus der Gruppe bestehend aus
Wasserstoff,
unverzweigte oder verzweigte Alkylgruppe mit 1 bis 30 Kohlenstoffatomen, bei welcher mindestens ein Wasserstoffrest durch einen Rest ausgewählt aus der Gruppe bestehend aus -OH, -OCH₃, -OCH₂CH₃, -N(CH₃)₂, -N(CH₂CH₃)₂, -N(CH₃)(CH₂CH₃) ersetzt sein kann,
ein Rest, der eine chemischen Struktur ausgewählt aus der Gruppe bestehend aus (xxi), **(xxii), (xxiii), (xxiv), (xxv), (xxvi)** mit 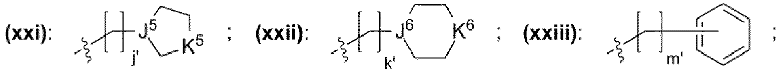 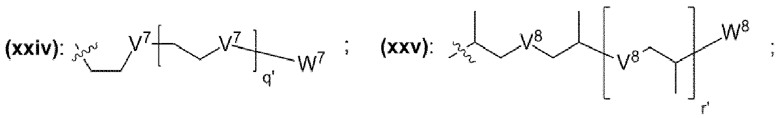 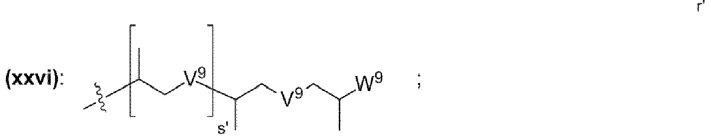
aufweist,
wobei J⁵, J⁶ unabhängig voneinander jeweils ausgewählt aus der Gruppe bestehend aus CH, N sind,
wobei K⁵, K⁶ unabhängig voneinander jeweils ausgewählt aus der Gruppe bestehend aus -O-, -N(CH₃)-, -N(CH₂CH₃)-, -S-, -CH₂- sind,
wobei V⁷, V⁸, V⁹ unabhängig voneinander jeweils ausgewählt aus der Gruppe bestehend aus -O-, -S-, -NR'""- mit R'"" = unverzweigte oder verzweigte Alkylgruppe mit 1 bis 6 Kohlenstoffatomen sind,
wobei W⁷, W⁸, W⁹ unabhängig voneinander jeweils ausgewählt aus der Gruppe bestehend aus H, Methyl, Ethyl sind,
wobei j', k', m', q', r', s' unabhängig voneinander jeweils eine ganze Zahl ausgewählt aus dem Bereich 0 bis 50 ist,
wobei in der chemischen Struktur **(xxi), (xxii), (xxiii), (xxiv), (xxv), (xxvi)** mindestens ein Wasserstoffrest durch einen Rest ausgewählt aus der Gruppe bestehend aus -OH, -OCH₃, -OCH₂CH₃, -N(CH₃)₂, -N(CH₂CH₃)₂, -N(CH₃)(CH₂CH₃) ersetzt sein kann;
und wobei R²⁴, R²⁵, R²⁶, R²⁷, R²⁸, R²⁹ jeweils unabhängig voneinander ausgewählt sind, bis auf die Ausnahme, dass nicht gilt: R²⁴ = R²⁵ = Wasserstoff,
und wobei man reduktive Bedingungen dadurch einstellt, dass man die mindestens eine Triacetondiaminverbindung **(I)** mit der mindestens einen Carbonylverbindung **(II)** in Gegenwart von Wasserstoff und in Gegenwart eines Vollkatalysators umsetzt, wobei der Vollkatalysator mindestens ein Metall **M** aufweist, wobei das Metall **M** ausgewählt ist aus der Gruppe bestehend aus Ag, V, Cr, Mo, Mn, Ni, Pd, Pt, Fe, Ru, Os, Co, Rh, Ir, Cu.

2. Verfahren nach Anspruch 1, wobei p¹ = p² = p³ = p⁴ = p⁵ = p⁶ = p⁸ = p⁹ = p¹⁰ = p¹¹ = 0 ist und wobei p⁷, p¹², p¹³, p¹⁴, p¹⁵, p¹⁶ unabhängig voneinander jeweils 0 oder 1 ist.

3. Verfahren nach Anspruch 1 oder 2, wobei Y¹, Y², Y³, Y⁴, Y⁵, Y⁶, Y⁷, Y⁸, Y⁹ unabhängig voneinander jeweils ausgewählt ist aus der Gruppe bestehend aus
unverzweigte oder verzweigte Alkylengruppe mit 1 bis 30 Kohlenstoffatomen,
zweiwertige gesättigte Kohlenwasserstoffgruppe mit 3 bis 30 Kohlenstoffatomen, welche mindestens einen gesättigten Ring aus 3 bis 30 Kohlenstoffatomen aufweist.

4. Verfahren nach einem oder mehreren der Ansprüche 1 bis 3, wobei die Reste R¹, R², R³, R⁴, R⁵, R⁶, R⁷, R⁸, R⁹, R¹⁰, R¹¹ unabhängig voneinander jeweils ausgewählt sind aus der Gruppe bestehend aus
Wasserstoff,
unverzweigte oder verzweigte Alkylgruppe mit 1 bis 30 Kohlenstoffatomen, bei welcher mindestens ein Wasserstoffrest durch einen Rest ausgewählt aus der Gruppe bestehend aus -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann,
einem Rest, der eine chemischen Struktur **(ix)** mit 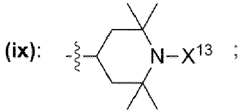
aufweist,
wobei X¹³ ausgewählt ist aus der Gruppe bestehend aus Wasserstoff, -OH, -O·, unverzweigte oder verzweigte Alkylgruppe mit 1 bis 10 Kohlenstoffatomen, unverzweigte oder verzweigte Alkoxygruppe mit 1 bis 10 Kohlenstoffatomen;
und wobei die Reste R¹², R¹³, R¹⁴, R¹⁵, R¹⁶, R¹⁷ unabhängig voneinander jeweils ausgewählt sind aus der Gruppe bestehend aus
Wasserstoff,
unverzweigte oder verzweigte Alkylgruppe mit 1 bis 30 Kohlenstoffatomen,
einer Gruppe mit der chemischen Struktur **(x)** mit 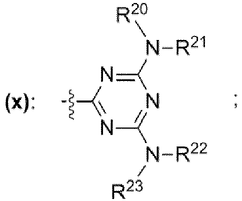
wobei die Reste R¹⁸, R¹⁹, R²⁰, R²¹, R²², R²³ unabhängig voneinander jeweils ausgewählt sind aus der Gruppe bestehend aus
Wasserstoff,
unverzweigte oder verzweigte Alkylgruppe mit 1 bis 30 Kohlenstoffatomen, bei welcher mindestens ein Wasserstoffrest durch einen Rest ausgewählt aus der Gruppe bestehend aus -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann,
einem Rest, der eine chemischen Struktur **(xvii)** mit 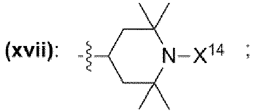
aufweist,
wobei X¹⁴ ausgewählt ist aus der Gruppe bestehend aus Wasserstoff, -OH, -O·, unverzweigte oder verzweigte Alkylgruppe mit 1 bis 10 Kohlenstoffatomen, unverzweigte oder verzweigte Alkoxygruppe mit 1 bis 10 Kohlenstoffatomen,
und mit der Maßgabe, dass R¹² und R¹⁷ für p¹³ = p¹⁴ = p¹⁵ = p¹⁶ = 0 unabhängig voneinander jeweils auch eine Gruppe der chemischen Struktur **(xviii)** mit 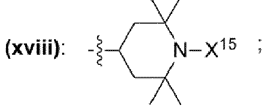 sein können, wobei X¹⁵ ausgewählt ist aus der Gruppe bestehend aus Wasserstoff, OH, -O·, unverzweigte oder verzweigte Alkylgruppe mit 1 bis 10 Kohlenstoffatomen, unverzweigte oder verzweigte Alkoxygruppe mit 1 bis 10 Kohlenstoffatomen.

5. Verfahren nach einem oder mehreren der Ansprüche 1 bis 4, wobei X¹ = X² = X³ = X⁴ = X⁵ = X⁶ = X⁷ = X⁸ = X⁹ = X¹⁰ = X¹¹ = X¹² = X¹³ = X¹⁴ = X¹⁵ = Wasserstoff ist.

6. Verfahren nach einem oder mehreren der Ansprüche 1 bis 5, wobei die Triacetondiaminverbindung **(I)** ausgewählt ist aus der Gruppe bestehend aus den chemischen Strukturen **(I-A), (I-B)** und wobei die Reste R¹, R² unabhängig voneinander jeweils ausgewählt sind aus der Gruppe bestehend aus
Wasserstoff,
unverzweigte oder verzweigte Alkylgruppe mit 1 bis 12 Kohlenstoffatomen, bei welcher mindestens ein Wasserstoffrest durch einen Rest ausgewählt aus der Gruppe bestehend aus -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann.

7. Verfahren nach einem oder mehreren der Ansprüche 1 bis 6, wobei die Triacetondiaminverbindung **(I)** ausgewählt ist aus der Gruppe bestehend aus den chemischen Strukturen **(I-A), (I-B)** und wobei die Reste R¹, R² unabhängig voneinander jeweils ausgewählt sind aus der Gruppe bestehend aus
Wasserstoff,
unverzweigte oder verzweigte Alkylgruppe mit 1 bis 12 Kohlenstoffatomen.

8. Verfahren nach einem oder mehreren der Ansprüche 1 bis 7, wobei Z¹, Z², Z³ unabhängig voneinander jeweils ausgewählt sind aus der Gruppe bestehend aus
direkte Bindung,
unverzweigte oder verzweigte Alkylengruppe mit 1 bis 30 Kohlenstoffatomen, zweiwertige gesättigte Kohlenwasserstoffgruppe mit 3 bis 30 Kohlenstoffatomen, welche mindestens einen gesättigten Ring aus 3 bis 30 Kohlenstoffatomen aufweist.

9. Verfahren nach einem oder mehreren der Ansprüche 1 bis 8, wobei die Carbonylverbindung **(II)** ausgewählt ist aus der Gruppe bestehend aus den chemischen Strukturen **(II-A), (II-B).**

10. Verfahren nach einem oder mehreren der Ansprüche 1 bis 9, wobei die Triacetondiaminverbindung **(I)** ausgewählt ist aus der Gruppe bestehend aus den chemischen Strukturen **(I-A), (I-B)** und wobei die Carbonylverbindung **(II)** ausgewählt ist aus der Gruppe bestehend aus den chemischen Strukturen **(II-A), (II-B),** wobei
p¹ = p² = 0;
X¹ = X² = X³ = Wasserstoff;
Y¹ und Z¹ unabhängig voneinander jeweils eine unverzweigte oder verzweigte Alkylengruppe mit 1 bis 12 Kohlenstoffatomen ist;
R¹, R² unabhängig voneinander jeweils ausgewählt sind aus der Gruppe bestehend aus
Wasserstoff,
unverzweigte oder verzweigte Alkylgruppe mit 1 bis 12 Kohlenstoffatomen;
R²⁴, R²⁵, R²⁶, R²⁷ ausgewählt sind aus der Gruppe bestehend aus
Wasserstoff,
unverzweigte oder verzweigte Alkylgruppe mit 1 bis 12 Kohlenstoffatomen;
wobei R²⁴, R²⁵, R²⁶, R²⁷ jeweils unabhängig voneinander ausgewählt sind, bis auf die Ausnahme, dass nicht gilt: R²⁴ = R²⁵ = Wasserstoff.

11. Verfahren nach einem oder mehreren der Ansprüche 1 bis 10, wobei die Triacetondiaminverbindung **(I)** die chemische Struktur **(I-A)** aufweist und wobei die Carbonylverbindung **(II)** die chemische Struktur **(II-A)** aufweist, wobei
X¹ = Wasserstoff;
R¹ ausgewählt ist aus der Gruppe bestehend aus
Wasserstoff,
unverzweigte oder verzweigte Alkylgruppe mit 1 bis 8 Kohlenstoffatomen;
R²⁴, R²⁵ ausgewählt sind aus der Gruppe bestehend aus
Wasserstoff,
unverzweigte oder verzweigte Alkylgruppe mit 1 bis 8 Kohlenstoffatomen;
wobei R²⁴, R²⁵ jeweils unabhängig voneinander ausgewählt sind, bis auf die Ausnahme, dass nicht gilt: R²⁴ = R²⁵ = Wasserstoff.

12. Verfahren nach einem oder mehreren der Ansprüche 1 bis 11, wobei die Triacetondiaminverbindung **(I)** die chemische Struktur **(I-A)** aufweist und wobei die Carbonylverbindung **(II)** die chemische Struktur **(II-A)** aufweist, wobei X¹ = H; R¹ = H; R²⁴ ausgewählt aus der Gruppe bestehend aus Wasserstoff, Methyl ist; R²⁵ eine unverzweigte oder verzweigte Alkylgruppe mit 1 bis 8 Kohlenstoffatomen ist.

13. Verfahren nach einem oder mehreren der Ansprüche 1 bis 12, wobei man es in mindestens einem Lösungsmittel durchführt, wobei das Lösungsmittel ausgewählt ist aus der Gruppe bestehend aus aliphatische Lösungsmittel, aromatische Lösungsmittel, Ether, halogenierte Lösungsmittel, Amide, Thioverbindungen, Carbonsäuren, Alkohole, Wasser.

14. Verfahren nach einem oder mehreren der Ansprüche 1 bis 13, wobei man es bei einer Temperatur im Bereich von 20 °C bis 350 °C und einem Druck im Bereich von 2 bar bis 500 bar durchführt.

15. Verfahren nach einem oder mehreren der Ansprüche 1 bis 14, wobei das Metall **M** ausgewählt ist aus der Gruppe bestehend aus Ni, Co, Cu, Fe, Ag.

## Claims

1. Process for preparing an N-substituted triacetonediamine compound,
**characterized in that** at least one triacetonediamine compound (I) is reacted with at least one carbonyl compound (II) under reductive conditions,
where the triacetonediamine compound (I) is selected from the group consisting of the chemical structures (I-A), (I-B), (I-C), (I-D), (I-E) with 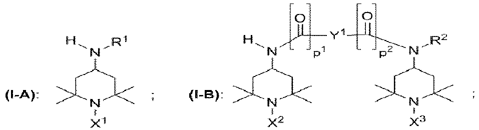 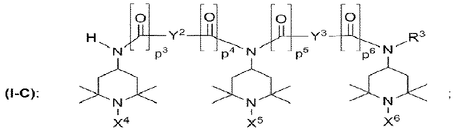 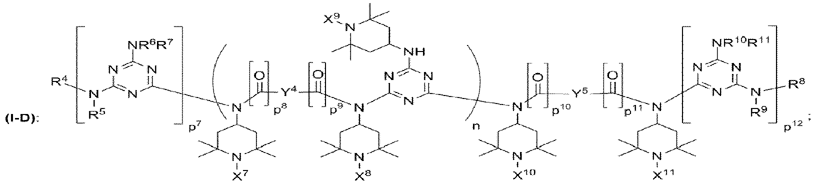 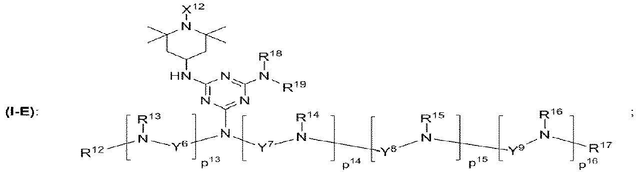
where n is an integer from the range of 1 to 20;
where p¹, p², p³, p⁴, p⁵, p⁶, p⁷, p⁸, p⁹, p¹⁰, p¹¹, p¹², p¹³, p¹⁴, p¹⁵, p¹⁶ are each independently 0 or 1;
where X¹, X², X³, X⁴, X⁵, X⁶, X⁷, X⁸, X⁹, X¹⁰, X¹¹, X¹² are each independently selected from the group consisting of hydrogen, OH, -O·, unbranched or branched alkyl group having 1 to 10 carbon atoms, unbranched or branched alkoxy group having 1 to 10 carbon atoms;
where Y¹, Y², Y³, Y⁴, Y⁵, Y⁶, Y⁷, Y⁸, Y⁹ are each independently selected from the group consisting of
unbranched or branched alkylene group having 1 to 30 carbon atoms,
divalent saturated hydrocarbyl group having 3 to 30 carbon atoms and having at least one saturated ring composed of 3 to 30 carbon atoms,
divalent hydrocarbyl group having 6 to 30 carbon atoms, of which at least 6 carbon atoms are present in an aromatic system and the other carbon atoms, if present, are saturated,
a bridging radical having a chemical structure selected from the group consisting of (i), (ii) with 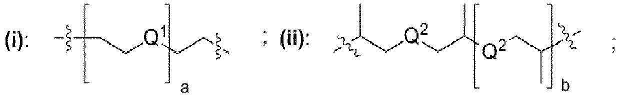
where
Q¹, Q² are each independently selected from the group consisting of -O-, -S-, -NH- and -NR'- with R' = unbranched or branched alkyl group having 1 to 6 carbon atoms,
where a is an integer selected from the range of 1 to 50,
where b is an integer selected from the range of 0 to 50,
and where Y¹ may also be a direct bond if at least one of p¹ and p² has the value of 1,
and where Y² may also be a direct bond if at least one of p³ and p⁴ has the value of 1,
and where Y³ may also be a direct bond if at least one of p⁵ and p⁶ has the value of 1,
and where Y⁴ may also be a direct bond if at least one of p⁸ and p⁹ has the value of 1,
and where Y⁵ may also be a direct bond if at least one of p¹⁰ and p¹¹ has the value of 1;
where the R¹, R², R³, R⁴, R⁵, R⁶, R⁷, R⁸, R⁹, R¹⁰, R¹¹ radicals are each independently selected from the group consisting of
hydrogen,
unbranched or branched alkyl group which has 1 to 30 carbon atoms and in which at least one hydrogen radical may be replaced by a radical selected from the group consisting of -OH, -NH₂, -OCH₃, -OCH₂CH₃, -NH(CH₃), -N(CH₃)₂,-NH(CH₂CH₃), -N(CH₂CH₃)₂,
-N(CH₃)(CH₂CH₃),
unbranched or branched acyl group which has 1 to 30 carbon atoms and in which at least one hydrogen radical may be replaced by a radical selected from the group consisting of -OH, -NH₂, -OCH₃, -OCH₂CH₃, -NH(CH₃), -N(CH₃)₂,-NH(CH₂CH₃), -N(CH₂CH₃)₂, -N(CH₃)(CH₂CH₃),
a radical having a chemical structure selected from the group consisting of (iii), (iv), (v), (vi), (vii), (viii), (ix) with 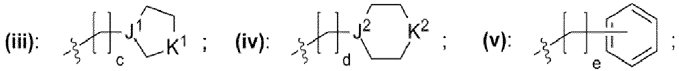 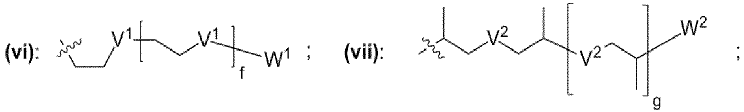 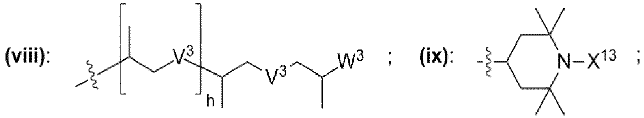
where J¹, J² are each independently selected from the group consisting of CH, N,
where K¹, K² are each independently selected from the group consisting of -O-, -NH-, -N(CH₃)-, -N(CH₂CH₃)-, -S-, -CH₂-,
where V¹, V², V³ are each independently selected from the group consisting of -O-, -S-, -NH-,-NR"- with R" = unbranched or branched alkyl group having 1 to 6 carbon atoms,
where W¹, W², W³ are each independently selected from the group consisting of H, methyl, ethyl,
where c, d, e, f, g, h are each independently an integer from the range of 0 to 50,
where X¹³ is selected from the group consisting of hydrogen, -OH, -O·, unbranched or branched alkyl group having 1 to 10 carbon atoms, unbranched or branched alkoxy group having 1 to 10 carbon atoms,
where, in the chemical structures (iii), (iv), (v), (vi), (vii), (viii), (ix), at least one hydrogen radical bonded to a carbon atom may be replaced by a radical selected from the group consisting of
-OH, -NH₂, -OCH₃, -OCH₂CH₃, -NH(CH₃), -N(CH₃)₂,-NH(CH₂CH₃), -N(CH₂CH₃)₂,
-N(CH₃)(CH₂CH₃);
where the R¹², R¹³, R¹⁴, R¹⁵, R¹⁶, R¹⁷ radicals are each independently selected from the group consisting of
hydrogen,
unbranched or branched alkyl group having 1 to 30 carbon atoms,
a group having the chemical structure (x) with 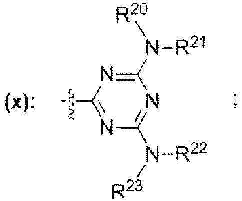
where the R¹⁸, R¹⁹, R²⁰, R²¹, R²², R²³ radicals are each independently selected from the group consisting of
hydrogen,
unbranched or branched alkyl group which has 1 to 30 carbon atoms and in which at least one hydrogen radical may be replaced by a radical selected from the group consisting of -OH, -NH₂, -OCH₃, -OCH₂CH₃, -NH(CH₃), -N(CH₃)₂,-NH(CH₂CH₃), -N(CH₂CH₃)₂, -N(CH₃)(CH₂CH₃),
unbranched or branched acyl group which has 1 to 30 carbon atoms and in which at least one hydrogen radical may be replaced by a radical selected from the group consisting
of -OH, -NH₂, -OCH₃, -OCH₂CH₃, -NH(CH₃), -N(CH₃)₂,-NH(CH₂CH₃), -N (CH₂CH₃)₂,
-N(CH₃)(CH₂CH₃);
a radical having a chemical structure selected from the group consisting of (xi), (xii), (xiii), (xiv), (xv), (xvi), (xvii) with 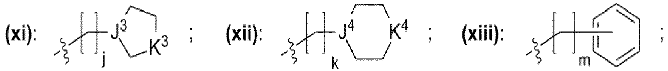 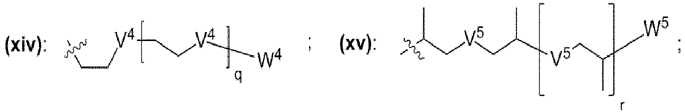 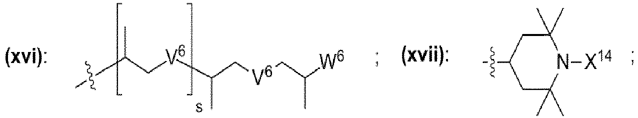
where J³, J⁴ are each independently selected from the group consisting of CH, N,
where K³, K⁴ are each independently selected from the group consisting of -O-, -NH-, -N(CH₃)-, -N(CH₂CH₃)-, -S-, -CH₂-,
where V⁴, V⁵, V⁶ are each independently selected from the group consisting of -O-, -S-, -NH-, - NR "'- with R'" = unbranched or branched alkyl group having 1 to 6 carbon atoms,
where W⁴, W⁵, W⁶ are each independently selected from the group consisting of H, methyl, ethyl,
where j, k, m, q, r, s are each independently an integer from the range of 0 to 50,
where X¹⁴ is selected from the group consisting of hydrogen, -OH, -O·, unbranched or branched alkyl group having 1 to 10 carbon atoms, unbranched or branched alkoxy group having 1 to 10 carbon atoms,
where, in the chemical structures (xi), (xii), (xiii), (xiv), (xv), (xvi), (xvii), at least one hydrogen radical bonded to a carbon atom may be replaced by a radical selected from the group consisting of -OH, -NH₂, -OCH₃, -OCH₂CH₃, -NH(CH₃), -N(CH₃)₂, -NH(CH₂CH₃), - N(CH₂CH₃)₂, -N(CH₃)(CH₂CH₃),
and with the proviso that R¹² and R¹⁷, when p¹³ = p¹⁴ = p¹⁵ = p¹⁶ = 0, may each independently also be a group of the chemical structure (xviii) with 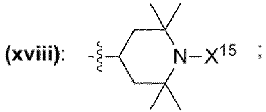
where X¹⁵ is selected from the group consisting of hydrogen, OH, -O·, unbranched or branched alkyl group having 1 to 10 carbon atoms, unbranched or branched alkoxy group having 1 to 10 carbon atoms;
and where the carbonyl compound (II) is selected from the group consisting of the chemical structures (II-A), (II-B), (II-C) with 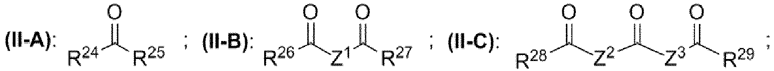
where Z¹, Z², Z³ are each independently selected from the group consisting of
direct bond,
unbranched or branched alkylene group having 1 to 30 carbon atoms,
divalent saturated hydrocarbyl group having 3 to 30 carbon atoms and having at least one saturated ring composed of 3 to 30 carbon atoms,
divalent hydrocarbyl group having 6 to 30 carbon atoms, of which at least 6 carbon atoms are present in an aromatic system and the other carbon atoms, if present, are saturated,
a bridging radical having a chemical structure selected from the group consisting of (xix), (xx) with 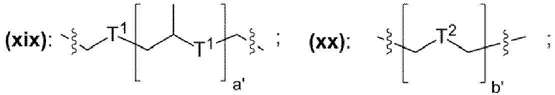
where
T¹, T² are each independently selected from the group consisting of -O-, -S- and -NR""- with R"" = unbranched or branched alkyl group having 1 to 6 carbon atoms,
where a' and b' are each independently an integer selected from the range of 1 to 50;
and where the R²⁴, R²⁵, R²⁶, R²⁷, R²⁸, R²⁹ radicals are selected from the group consisting of
hydrogen,
unbranched or branched alkyl group which has 1 to 30 carbon atoms and in which at least one hydrogen radical may be replaced by a radical selected from the group consisting of -OH, -OCH₃, -OCH₂CH₃, -N(CH₃)₂, -N(CH₂CH₃)₂, - N(CH₃)(CH₂CH₃),
a radical having a chemical structure selected from the group consisting of (xxi), (xxii), (xxiii), (xxiv), (xxv), (xxvi) with 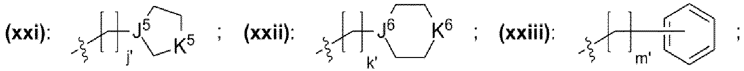 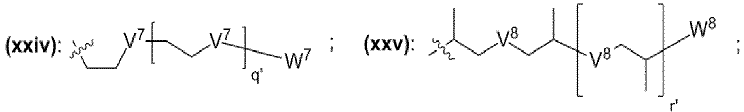 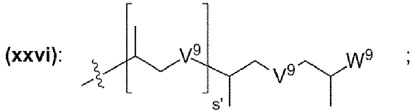
where J⁵, J⁶ are each independently selected from the group consisting of CH, N,
where K⁵, K⁶ are each independently selected from the group consisting of -O-, -N(CH₃)-, -N(CH₂CH₃)-, -S-, -CH₂-,
where V⁷, V⁸, V⁹ are each independently selected from the group consisting of -O-, -S-, -NR""'-with R"'" = unbranched or branched alkyl group having 1 to 6 carbon atoms,
where W⁷, W⁸, W⁹ are each independently selected from the group consisting of H, methyl, ethyl,
where j', k', m', q', r', s' are each independently an integer selected from the range of 0 to 50,
where, in the chemical structure (xxi), (xxii), (xxiii), (xxiv), (xxv), (xxvi), at least one hydrogen radical may be replaced by a radical selected from the group consisting of -OH, -OCH₃, - OCH₂CH₃, -N(CH₃)₂, -N(CH₂CH₃)₂, -N(CH₃)(CH₂CH₃);
and where R²⁴, R²⁵, R²⁶, R²⁷, R²⁸, R²⁹ are each selected independently, with the exclusion of: R²⁴ = R²⁵ = hydrogen,
and wherein reductive conditions are established by reacting the at least one triacetonediamine compound (I) with the at least one carbonyl compound (II) in the presence of hydrogen and in the presence of an unsupported catalyst, where the unsupported catalyst includes at least one metal M, where the metal M is selected from the group consisting of Ag, V, Cr, Mo, Mn, Ni, Pd, Pt, Fe, Ru, Os, Co, Rh, Ir, Cu.

2. Process according to Claim 1, where p¹ = p² = p³ = p⁴ = p⁵ = p⁶ = p⁸ = p⁹ = p¹⁰ = p¹¹ = 0 and where p⁷, p¹², p¹³, p¹⁴, p¹⁵, p¹⁶ are each independently 0 or 1.

3. Process according to Claim 1 or 2, where Y¹, Y², Y³, Y⁴, Y⁵, Y⁶, Y⁷, Y⁸, Y⁹ are each independently selected from the group consisting of
unbranched or branched alkylene group having 1 to 30 carbon atoms,
divalent saturated hydrocarbyl group having 3 to 30 carbon atoms and having at least one saturated ring composed of 3 to 30 carbon atoms.

4. Process according to one or more of Claims 1 to 3, where the R¹, R², R³, R⁴, R⁵, R⁶, R⁷, R⁸, R⁹, R¹⁰, R¹¹ radicals are each independently selected from the group consisting of
hydrogen,
unbranched or branched alkyl group which has 1 to 30 carbon atoms and in which at least one hydrogen radical may be replaced by a radical selected from the group consisting of -OH, -NH₂, -OCH₃, -OCH₂CH₃, -NH(CH₃), -N(CH₃)₂, - NH(CH₂CH₃), -N(CH₂CH₃)₂,
-N(CH₃)(CH₂CH₃),
a radical having a chemical structure (ix) with 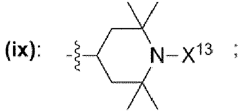
where X¹³ is selected from the group consisting of hydrogen, -OH, -O·, unbranched or branched alkyl group having 1 to 10 carbon atoms, unbranched or branched alkoxy group having 1 to 10 carbon atoms;
and where the R¹², R¹³, R¹⁴, R¹⁵, R¹⁶, R¹⁷ radicals are each independently selected from the group consisting of
hydrogen,
unbranched or branched alkyl group having 1 to 30 carbon atoms,
a group having the chemical structure (x) with 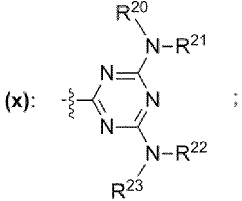
where the R¹⁸, R¹⁹, R²⁰, R²¹, R²², R²³ radicals are each independently selected from the group consisting of
hydrogen,
unbranched or branched alkyl group which has 1 to 30 carbon atoms and in which at least one hydrogen radical may be replaced by a radical selected from the group consisting of
-OH, -NH₂, -OCH₃, -OCH₂CH₃, -NH(CH₃), -N(CH₃)₂, - NH(CH₂CH₃), -N(CH₂CH₃)₂,
-N(CH₃)(CH₂CH₃),
a radical having a chemical structure (xvii) with 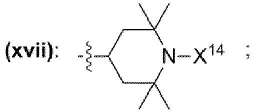
where X¹⁴ is selected from the group consisting of hydrogen, -OH, -O·, unbranched or branched alkyl group having 1 to 10 carbon atoms, unbranched or branched alkoxy group having 1 to 10 carbon atoms,
and with the proviso that R¹² and R¹⁷, when p¹³ = p¹⁴ = p¹⁵ = p¹⁶ = 0, may each independently also be a group of the chemical structure (xviii) with 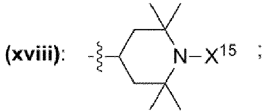 where X¹⁵ is selected from the group consisting of hydrogen, OH, -O·, unbranched or branched alkyl group having 1 to 10 carbon atoms, unbranched or branched alkoxy group having 1 to 10 carbon atoms.

5. Process according to one or more of Claims 1 to 4, where X¹ = X² = X³ = X⁴ = X⁵ = X⁶ = X⁷ = X⁸ = X⁹ = X¹⁰ = X¹¹ = X¹² = X¹³ = X¹⁴ = X¹⁵ = hydrogen.

6. Process according to one or more of Claims 1 to 5, where the triacetonediamine compound (I) is selected from the group consisting of the chemical structures (I-A), (I-B) and where the R¹, R² radicals are each independently selected from the group consisting of
hydrogen,
unbranched or branched alkyl group which has 1 to 12 carbon atoms and in which at least one hydrogen radical may be replaced by a radical selected from the group consisting of -OH, -NH₂, -OCH₃, -OCH₂CH₃, -NH(CH₃), -N(CH₃)₂, - NH(CH₂CH₃), -N(CH₂CH₃)₂, -N(CH₃)(CH₂CH₃).

7. Process according to one or more of Claims 1 to 6, where the triacetonediamine compound (I) is selected from the group consisting of the chemical structures (I-A), (I-B) and where the R¹, R² radicals are each independently selected from the group consisting of
hydrogen,
unbranched or branched alkyl group having 1 to 12 carbon atoms.

8. Process according to one or more of Claims 1 to 7, where Z¹, Z², Z³ are each independently selected from the group consisting of
direct bond,
unbranched or branched alkylene group having 1 to 30 carbon atoms,
divalent saturated hydrocarbyl group having 3 to 30 carbon atoms and having at least one saturated ring composed of 3 to 30 carbon atoms.

9. Process according to one or more of Claims 1 to 8, where the carbonyl compound (II) is selected from the group consisting of the chemical structures (II-A), (II-B).

10. Process according to one or more of Claims 1 to 9, where the triacetonediamine compound (I) is selected from the group consisting of the chemical structures (I-A), (I-B) and where the carbonyl compound (II) is selected from the group consisting of the chemical structures (II-A), (II-B), where
p¹ = p² = 0;
X¹ = X² = X³ = hydrogen;
Y¹ and Z¹ are each independently an unbranched or branched alkylene group having 1 to 12 carbon atoms;
R¹, R² are each independently selected from the group consisting of
hydrogen,
unbranched or branched alkyl group having 1 to 12 carbon atoms;
R²⁴, R²⁵, R²⁶, R²⁷ are selected from the group consisting of
hydrogen,
unbranched or branched alkyl group having 1 to 12 carbon atoms;
where R²⁴, R²⁵, R²⁶, R²⁷ are each selected independently, with the exclusion of: R²⁴ = R²⁵ = hydrogen.

11. Process according to one or more of Claims 1 to 10, where the triacetonediamine compound (I) has the chemical structure (I-A) and where the carbonyl compound (II) has the chemical structure (II-A), where
X¹ = hydrogen;
R¹ is selected from the group consisting of
hydrogen,
unbranched or branched alkyl group having 1 to 8 carbon atoms;
R²⁴, R²⁵ are selected from the group consisting of
hydrogen,
unbranched or branched alkyl group having 1 to 8 carbon atoms;
where R²⁴, R²⁵ are each selected independently, with the exclusion of: R²⁴ = R²⁵ = hydrogen.

12. Process according to one or more of Claims 1 to 11, where the triacetonediamine compound (I) has the chemical structure (I-A) and where the carbonyl compound (II) has the chemical structure (II-A), where X¹ = H; R¹ = H; R²⁴ is selected from the group consisting of hydrogen, methyl; R²⁵ is an unbranched or branched alkyl group having 1 to 8 carbon atoms.

13. Process according to one or more of Claims 1 to 12, which is conducted in at least one solvent, where the solvent is selected from the group consisting of aliphatic solvents, aromatic solvents, ethers, halogenated solvents, amides, thio compounds, carboxylic acids, alcohols, water.

14. Process according to one or more of Claims 1 to 13, which is conducted at a temperature in the range from 20°C to 350°C and a pressure in the range from 2 bar to 500 bar.

15. Process according to one of more of Claims 1 to 14, wherein the metal M is selected from the group consisting of Ni, Co, Cu, Fe, Ag.

## Revendications

1. Procédé de fabrication d'un composé de triacétone-diamine N-substitué,
**caractérisé en ce qu'**au moins un composé de triacétone-diamine (I) est mis en réaction avec au moins un composé de carbonyle (II) dans des conditions réductrices,
le composé de triacétone-diamine (I) étant choisi dans le groupe constitué par les structures chimiques (I-A), (I-B), (I-C), (I-D), (I-E) avec 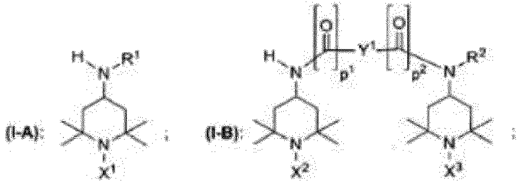 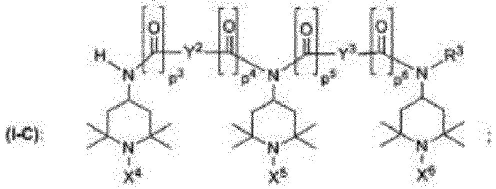 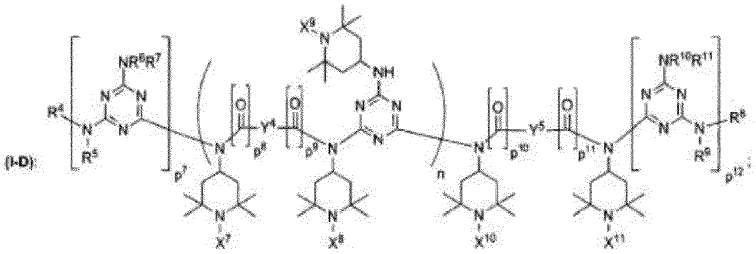 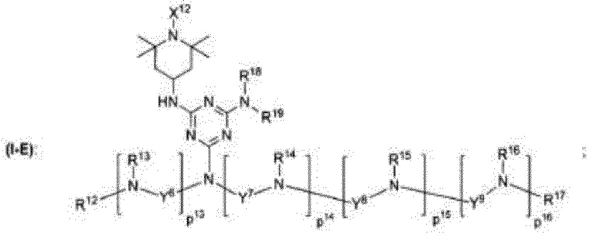
dans lesquelles n est un nombre entier dans la plage allant de 1 à 20 ;
dans lesquelles p¹, p², p³, p⁴, p⁵, p⁶, p⁷, p⁸, p⁹, p¹⁰, p¹¹, p¹², p¹³, p¹⁴, p¹⁵, p¹⁶ sont chacun indépendamment les uns des autres 0 ou 1 ;
dans lesquelles X¹, X², X³, X⁴, X⁵ X⁶, X⁷, X⁸, X⁹, X¹⁰, X¹¹, X¹² sont chacun choisis indépendamment les uns des autres dans le groupe constitué par hydrogène, OH, -O·, un groupe alkyle non ramifié ou ramifié de 1 à 10 atomes de carbone, un groupe alcoxy non ramifié ou ramifié de 1 à 10 atomes de carbone ;
dans lesquelles Y¹, Y², Y³, Y⁴, Y⁵, Y⁶, Y⁷, Y⁸, Y⁹ sont chacun choisis indépendamment les uns des autres dans le groupe constitué par :
un groupe alkylène non ramifié ou ramifié de 1 à 30 atomes de carbone,
un groupe hydrocarboné saturé bivalent de 3 à 30 atomes de carbone, qui comprend au moins un cycle saturé de 3 à 30 atomes de carbone,
un groupe hydrocarboné bivalent de 6 à 30 atomes de carbone, parmi lesquels au moins 6 atomes de carbone se situent dans un système aromatique et les autres atomes de carbone, s'ils sont présents, sont saturés,
un radical pontant, qui présente une structure chimique choisie dans le groupe constitué par (i), (ii), avec 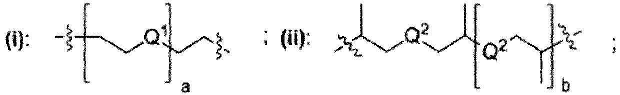
dans lesquelles
Q¹, Q² sont chacun choisis indépendamment l'un de l'autre dans le groupe constitué par -O-, -S-, -NH- ou -NR'- avec R' = groupe alkyle non ramifié ou ramifié de 1 à 6 atomes de carbone,
dans lesquelles a est un nombre entier choisi dans la plage allant de 1 à 50,
dans lesquelles b est un nombre entier choisi dans la plage allant de 0 à 50,
et dans lesquelles Y¹ peut également être une liaison directe si p¹ et/ou p² présentent la valeur 1,
et dans lesquelles Y² peut également être une liaison directe si p³ et/ou p⁴ présentent la valeur 1,
et dans lesquelles Y³ peut également être une liaison directe si p⁵ et/ou p⁶ présentent la valeur 1,
et dans lesquelles Y⁴ peut également être une liaison directe si p⁸ et/ou p⁹ présentent la valeur 1,
et dans lesquelles Y⁵ peut également être une liaison directe si p¹⁰ et/ou p¹¹ présentent la valeur 1,
dans lesquelles les radicaux R¹, R², R³, R⁴, R⁵, R⁶, R⁷, R⁸, R⁹, R¹⁰, R¹¹ sont chacun choisis indépendamment les uns des autres dans le groupe constitué par :
l'hydrogène,
un groupe alkyle non ramifié ou ramifié de 1 à 30 atomes de carbone, dans lequel au moins un radical hydrogène peut être remplacé par un radical choisi dans le groupe constitué par -OH, -NH₂, -OCH₃, -OCH₂CH₃, -NH(CH₃), - N(CH₃)₂, -NH(CH₂CH₃), -N(CH₂CH₃)₂, -N(CH₃)(CH₂CH₃),
un groupe acyle non ramifié ou ramifié de 1 à 30 atomes de carbone, dans lequel au moins un radical hydrogène peut être remplacé par un radical choisi dans le groupe constitué par -OH, -NH₂, -OCH₃, -OCH₂CH₃, -NH(CH₃), - N(CH₃)₂, -NH(CH₂CH₃), -N(CH₂CH₃)₂, -N(CH₃)(CH₂CH₃),
un radical qui présente une structure chimique choisie dans le groupe constitué par (iii), (iv), (v), (vi), (vii), (viii), (ix), avec 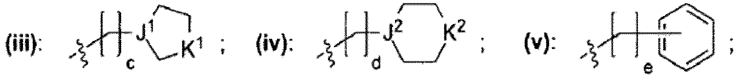 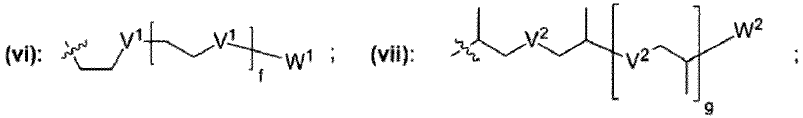 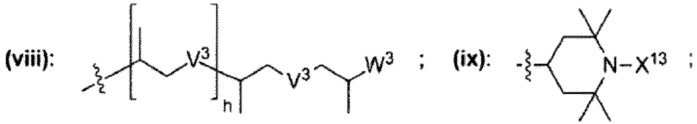
dans lesquelles J¹, J² sont chacun choisis indépendamment l'un de l'autre dans le groupe constitué par CH, N,
dans lesquelles K¹, K² sont chacun choisis indépendamment l'un de l'autre dans le groupe constitué par -O-, -NH-, -N(CH₃)-, -N(CH₂CH₃)-, -S-, -CH₂-,
dans lesquelles V¹, V², V³ sont chacun choisis indépendamment les uns des autres dans le groupe constitué par -O-, -S-, -NH-, -NR"- avec R" = groupe alkyle non ramifié ou ramifié de 1 à 6 atomes de carbone,
dans lesquelles W¹, W², W³ sont chacun choisis indépendamment les uns des autres dans le groupe constitué par H, méthyle, éthyle,
dans lesquelles c, d, e, f, g, h sont chacun indépendamment les uns des autres un nombre entier dans la plage allant de 0 à 50,
dans lesquelles X¹³ est choisi dans le groupe constitué par hydrogène, -OH, -O·, un groupe alkyle non ramifié ou ramifié de 1 à 10 atomes de carbone, un groupe alcoxy non ramifié ou ramifié de 1 à 10 atomes de carbone,
dans les structures chimiques (iii), (iv), (v), (vi), (vii), (viii), (ix), au moins un radical hydrogène relié à un atome de carbone pouvant être remplacé par un radical choisi dans le groupe constitué par :
-OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃),
dans lesquelles les radicaux R¹², R¹³, R¹⁴, R¹⁵, R¹⁶, R¹⁷ sont chacun choisis indépendamment les uns des autres dans le groupe constitué par :
l'hydrogène,
un groupe alkyle non ramifié ou ramifié de 1 à 30 atomes de carbone,
un groupe de structure chimique (x) avec 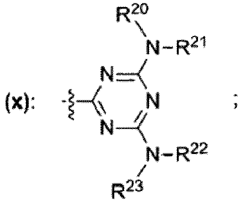
dans laquelle les radicaux R¹⁸, R¹⁹, R²⁰, R²¹, R²², R²³ sont chacun choisis indépendamment les uns des autres dans le groupe constitué par :
l'hydrogène,
un groupe alkyle non ramifié ou ramifié de 1 à 30 atomes de carbone, dans lequel au moins un radical hydrogène peut être remplacé par un radical choisi dans le groupe constitué par -OH, -NH₂, -OCH₃, -OCH₂CH₃, -NH(CH₃), - N(CH₃)₂, -NH(CH₂CH₃), -N(CH₂CH₃)₂, -N(CH₃)(CH₂CH₃),
un groupe acyle non ramifié ou ramifié de 1 à 30 atomes de carbone, dans lequel au moins un radical hydrogène peut être remplacé par un radical choisi dans le groupe constitué par -OH, -NH₂, -OCH₃, -OCH₂CH₃, -NH(CH₃), - N(CH₃)₂, -NH(CH₂CH₃), -N(CH₂CH₃)₂, -N(CH₃)(CH₂CH₃),
un radical qui présente une structure chimique choisie dans le groupe constitué par (xi), (xii), (xiii), (xiv), (xv), (xvi), (xvii), avec 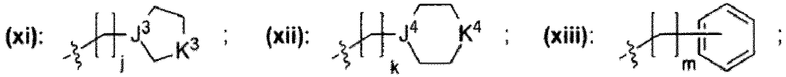 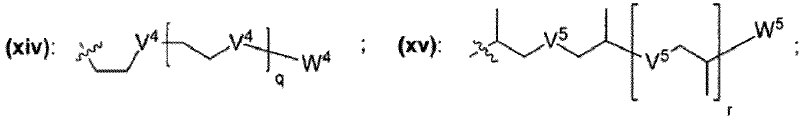 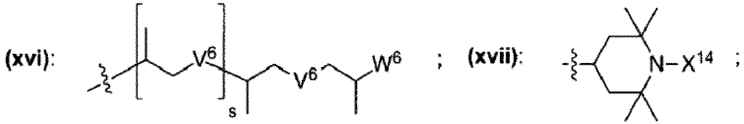
dans lesquelles J³, J⁴ sont chacun choisis indépendamment l'un de l'autre dans le groupe constitué par CH, N, dans lesquelles K³, K⁴ sont chacun choisis indépendamment l'un de l'autre dans le groupe constitué par -O-, -NH-, -N(CH₃)-, -N(CH₂CH₃)-, -S-, -CH₂-,
dans lesquelles V⁴, V⁵, V⁶ sont chacun choisis indépendamment les uns des autres dans le groupe constitué par -O-, -S-, -NH-, -NR"'- avec R'" = groupe alkyle non ramifié ou ramifié de 1 à 6 atomes de carbone,
dans lesquelles W⁴, W⁵, W⁶ sont chacun choisis indépendamment les uns des autres dans le groupe constitué par H, méthyle, éthyle,
dans lesquelles j, k, m, q, r, s sont chacun indépendamment les uns des autres un nombre entier dans la plage allant de 0 à 50,
dans lesquelles R¹⁴ est choisi dans le groupe constitué par hydrogène, -OH-, -O·, un groupe alkyle non ramifié ou ramifié de 1 à 10 atomes de carbone, un groupe alcoxy non ramifié ou ramifié de 1 à 10 atomes de carbone,
dans les structures chimiques (xi), (xii), (xiii), (xiv), (xv), (xvi), (xvii), au moins un radical hydrogène relié à un atome de carbone pouvant être remplacé par un radical choisi dans le groupe constitué par :
-OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃),
et à condition que R¹² et R¹⁷ pour p¹³ = p¹⁴ = p¹⁵ = p¹⁶ = 0 puissent chacun indépendamment l'un de l'autre également être un groupe de structure chimique (xviii) avec 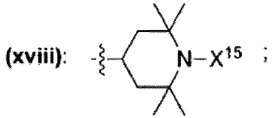
dans laquelle X¹⁵ est choisi dans le groupe constitué par hydrogène, OH, -O·, un groupe alkyle non ramifié ou ramifié de 1 à 10 atomes de carbone, un groupe alcoxy non ramifié ou ramifié de 1 à 10 atomes de carbone,
et le composé de carbonyle (II) étant choisi dans le groupe constitué par les structures chimiques (II-A), (II-B), (II-C), avec 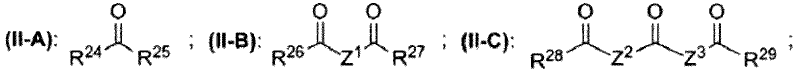
dans lesquelles Z¹, Z², Z³ sont chacun choisis indépendamment les uns des autres dans le groupe constitué par :
une liaison directe,
un groupe alkylène non ramifié ou ramifié de 1 à 30 atomes de carbone,
un groupe hydrocarboné saturé bivalent de 3 à 30 atomes de carbone, qui comprend au moins un cycle saturé de 3 à 30 atomes de carbone,
un groupe hydrocarboné bivalent de 6 à 30 atomes de carbone, parmi lesquels au moins 6 atomes de carbone se situent dans un système aromatique et les autres atomes de carbone, s'ils sont présents, sont saturés,
un radical pontant, qui présente une structure chimique choisie dans le groupe constitué par (xix), (xx), avec 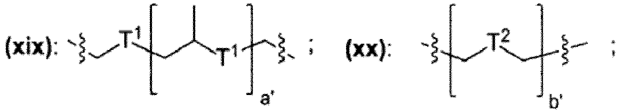
dans lesquelles
T¹, T² sont chacun choisis indépendamment l'un de l'autre dans le groupe constitué par -O-, -S- ou -NR""-, avec R"" = groupe alkyle non ramifié ou ramifié de 1 à 6 atomes de carbone,
dans lesquelles a' et b' sont chacun indépendamment l'un de l'autre un nombre entier choisi dans la plage allant de 1 à 50,
et dans lesquelles les radicaux R²⁴, R²⁵, R²⁶, R²⁷, R²⁸, R²⁹ sont choisis dans le groupe constitué par :
l'hydrogène,
un groupe alkyle non ramifié ou ramifié de 1 à 30 atomes de carbone, dans lequel au moins un radical hydrogène peut être remplacé par un radical choisi dans le groupe constitué par -OH, -OCH₃, -OCH₂CH₃, -N(CH₃)₂, -N(CH₂CH₃)₂, -N(CH₃)(CH₂CH₃),
un radical qui présente une structure chimique choisie dans le groupe constitué par (xxi), (xxii), (xxiii), (xxiv), (xxv), (xxvi), avec 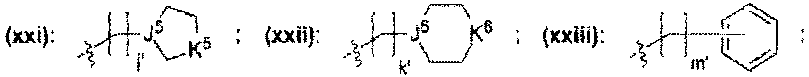 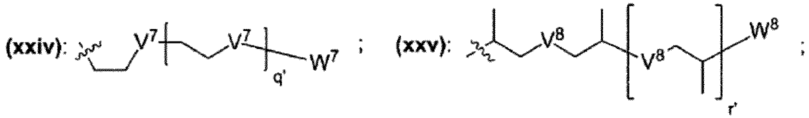 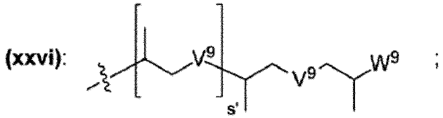
dans lesquelles J⁵, J⁶ sont chacun choisis indépendamment l'un de l'autre dans le groupe constitué par CH, N,
dans lesquelles K⁵, K⁶ sont chacun choisis indépendamment l'un de l'autre dans le groupe constitué par -O-, -N(CH₃)-, -N(CH₂CH₃)-, -S-, -CH₂-,
dans lesquelles V⁷, V⁸, V⁹ sont chacun choisis indépendamment les uns des autres dans le groupe constitué par -O-, -S-, -NR""'-, avec R"'" = groupe alkyle non ramifié ou ramifié de 1 à 6 atomes de carbone,
dans lesquelles W⁷, W⁸, W⁹ sont chacun choisis indépendamment les uns des autres dans le groupe constitué par H, méthyle, éthyle,
dans lesquelles j', k', m', q', r', s' sont chacun indépendamment les uns des autres un nombre entier choisi dans la plage allant de 0 à 50,
dans les structures chimiques (xxi), (xxii), (xxiii), (xxiv), (xxv), (xxvi), au moins un radical hydrogène pouvant être remplacé par un radical choisi dans le groupe constitué par -OH, -OCH₃, -OCH₂CH₃, -N(CH₃)₂, - N(CH₂CH₃)₂, -N(CH₃)(CH₂CH₃),
et dans lesquelles R²⁴, R²⁵, R²⁶, R²⁷, R²⁸, R²⁹ sont chacun choisis indépendamment les uns des autres, sauf lorsque R²⁴ = R²⁵ = hydrogène,
et des conditions réductrices étant ajustées par mise en réaction dudit au moins un composé de triacétone-diamine (I) avec ledit au moins un composé de carbonyle (II) en présence d'hydrogène et en présence d'un catalyseur entier, le catalyseur entier comprenant au moins un métal M, le métal M étant choisi dans le groupe constitué par Ag, V, Cr, Mo, Mn, Ni, Pd, Pt, Fe, Ru, Os, Co, Rh, Ir, Cu.

2. Procédé selon la revendication 1, dans lequel p¹ = p² = p³ = p⁴ = p⁵ = p⁶ = p⁸ = p⁹ = p¹⁰ = p¹¹ = 0 et dans lequel p⁷, p¹², p¹³, p¹⁴, p¹⁵, p¹⁶ sont chacun indépendamment les uns des autres 0 ou 1.

3. Procédé selon la revendication 1, dans lequel Y¹, Y², Y³, Y⁴, Y⁵, Y⁶, Y⁷, Y⁸, Y⁹ sont chacun choisis indépendamment les uns des autres dans le groupe constitué par :
un groupe alkylène non ramifié ou ramifié de 1 à 30 atomes de carbone,
un groupe hydrocarboné saturé bivalent de 3 à 30 atomes de carbone, qui comprend au moins un cycle saturé de 3 à 30 atomes de carbone.

4. Procédé selon une ou plusieurs des revendications 1 à 3, dans lequel les radicaux R¹, R², R³, R⁴, R⁵, R⁶, R⁷, R⁸, R⁹, R¹⁰, R¹¹ sont chacun choisis indépendamment les uns des autres dans le groupe constitué par :
l'hydrogène,
un groupe alkyle non ramifié ou ramifié de 1 à 30 atomes de carbone, dans lequel au moins un radical hydrogène peut être remplacé par un radical choisi dans le groupe constitué par -OH, -NH₂, -OCH₃, -OCH₂CH₃, -NH(CH₃), - N(CH₃)₂, -NH(CH₂CH₃), -N(CH₂CH₃)₂, -N(CH₃)(CH₂CH₃),
un radical qui présente une structure chimique (ix), avec 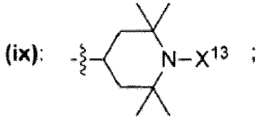
dans laquelle X¹³ est choisi dans le groupe constitué par hydrogène, -OH, -O·, un groupe alkyle non ramifié ou ramifié de 1 à 10 atomes de carbone, un groupe alcoxy non ramifié ou ramifié de 1 à 10 atomes de carbone,
et dans lesquelles les radicaux R¹², R¹³, R¹⁴, R¹⁵, R¹⁶, R¹⁷ sont chacun choisis indépendamment les uns des autres dans le groupe constitué par :
l'hydrogène,
un groupe alkyle non ramifié ou ramifié de 1 à 30 atomes de carbone,
un groupe de structure chimique (x) avec 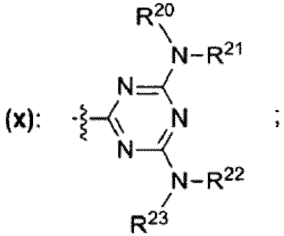
dans laquelle les radicaux R¹⁸, R¹⁹, R²⁰, R²¹, R²², R²³ sont chacun choisis indépendamment les uns des autres dans le groupe constitué par :
l'hydrogène,
un groupe alkyle non ramifié ou ramifié de 1 à 30 atomes de carbone, dans lequel au moins un radical hydrogène peut être remplacé par un radical choisi dans le groupe constitué par -OH, -NH₂, -OCH₃, -OCH₂CH₃, -NH(CH₃), - N(CH₃)₂, -NH(CH₂CH₃), -N(CH₂CH₃)₂, -N(CH₃)(CH₂CH₃),
un radical qui présente une structure chimique (xvii), avec 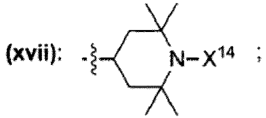
dans laquelle X¹⁴ est choisi dans le groupe constitué par hydrogène, -OH, -O·, un groupe alkyle non ramifié ou ramifié de 1 à 10 atomes de carbone, un groupe alcoxy non ramifié ou ramifié de 1 à 10 atomes de carbone,
et à condition que R¹² et R¹⁷ pour p¹³ = p¹⁴ = p¹⁵ = p¹⁶ = 0 puissent chacun indépendamment l'un de l'autre également être un groupe de structure chimique (xviii) avec 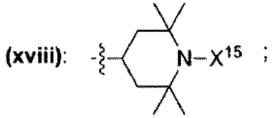
dans laquelle X¹⁵ est choisi dans le groupe constitué par hydrogène, OH, -O·, un groupe alkyle non ramifié ou ramifié de 1 à 10 atomes de carbone, un groupe alcoxy non ramifié ou ramifié de 1 à 10 atomes de carbone.

5. Procédé selon une ou plusieurs des revendications 1 à 4, dans lequel X¹ = X² = X³ = X⁴ = X⁵ = X⁶ = X⁷ = X⁸ = X⁹ = X¹⁰ = X¹¹ = X¹² = X¹³ = X¹⁴ = X¹⁵ = hydrogène.

6. Procédé selon une ou plusieurs des revendications 1 à 5, dans lequel le composé de triacétone-diamine (I) est choisi dans le groupe constitué par les structures chimiques (I-A), (I-B) et les radicaux R¹, R² sont chacun choisis indépendamment l'un de l'autre dans le groupe constitué par :
l'hydrogène,
un groupe alkyle non ramifié ou ramifié de 1 à 12 atomes de carbone, dans lequel au moins un radical hydrogène peut être remplacé par un radical choisi dans le groupe constitué par -OH, -NH₂, -OCH₃, -OCH₂CH₃, -NH(CH₃), - N(CH₃)₂, -NH(CH₂CH₃), -N(CH₂CH₃)₂, -N(CH₃)(CH₂CH₃).

7. Procédé selon une ou plusieurs des revendications 1 à 6, dans lequel le composé de triacétone-diamine (I) est choisi dans le groupe constitué par les structures chimiques (I-A), (I-B) et les radicaux R¹, R² sont chacun choisis indépendamment l'un de l'autre dans le groupe constitué par :
l'hydrogène,
un groupe alkyle non ramifié ou ramifié de 1 à 12 atomes de carbone.

8. Procédé selon une ou plusieurs des revendications 1 à 7, dans lequel Z¹, Z², Z³ sont chacun choisis indépendamment les uns des autres dans le groupe constitué par :
une liaison directe,
un groupe alkylène non ramifié ou ramifié de 1 à 30 atomes de carbone,
un groupe hydrocarboné saturé bivalent de 3 à 30 atomes de carbone, qui comprend au moins un cycle saturé de 3 à 30 atomes de carbone.

9. Procédé selon une ou plusieurs des revendications 1 à 8, dans lequel le composé de carbonyle (II) est choisi dans le groupe constitué par les structures chimiques (II-A), (II-B).

10. Procédé selon une ou plusieurs des revendications 1 à 9, dans lequel le composé de triacétone-diamine (I) est choisi dans le groupe constitué par les structures chimiques (I-A), (I-B), et dans lequel le composé de carbonyle (II) est choisi dans le groupe constitué par les structures chimiques (II-A), (II-B), avec
p¹ = p² = 0,
X¹ = X² = X³ = hydrogène,
Y¹ et Z¹ sont chacun indépendamment l'un de l'autre un groupe alkylène non ramifié ou ramifié de 1 à 12 atomes de carbone,
R¹, R² sont chacun choisis indépendamment l'un de l'autre dans le groupe constitué par :
l'hydrogène,
un groupe alkyle non ramifié ou ramifié de 1 à 12 atomes de carbone,
R²⁴, R²⁵, R²⁶, R²⁷ sont choisis dans le groupe constitué par
l'hydrogène,
un groupe alkyle non ramifié ou ramifié de 1 à 12 atomes de carbone,
R²⁴, R²⁵, R²⁶, R²⁷ étant chacun choisis indépendamment les uns des autres sauf lorsque R²⁴ = R²⁵ = hydrogène.

11. Procédé selon une ou plusieurs des revendications 1 à 10, dans lequel le composé de triacétone-diamine (I) présente la structure chimique (I-A) et dans lequel le composé de carbonyle (II) présente la structure chimique (II-A), avec
X¹ = hydrogène,
R¹ est choisi dans le groupe constitué par l'hydrogène,
un groupe alkyle non ramifié ou ramifié de 1 à 8 atomes de carbone,
R²⁴, R²⁵ sont choisis dans le groupe constitué par :
l'hydrogène,
un groupe alkyle non ramifié ou ramifié de 1 à 8 atomes de carbone,
R²⁴, R²⁵ étant chacun choisis indépendamment l'un de l'autre sauf lorsque R²⁴ = R²⁵ = hydrogène.

12. Procédé selon une ou plusieurs des revendications 1 à 11, dans lequel le composé de triacétone-diamine (I) présente la structure chimique (I-A) et dans lequel le composé de carbonyle (II) présente la structure chimique (II-A), avec X¹ = H ; R¹ = H ; R²⁴ est choisi dans le groupe constitué par hydrogène, méthyle ; R²⁵ est un groupe alkyle non ramifié ou ramifié de 1 à 8 atomes de carbone.

13. Procédé selon une ou plusieurs des revendications 1 à 12, dans lequel il est réalisé dans au moins un solvant, le solvant étant choisi dans le groupe constitué par les solvants aliphatiques, les solvants aromatiques, les éthers, les solvants halogénés, les amides, les composés thio, les acides carboxyliques, les alcools, l'eau.

14. Procédé selon une ou plusieurs des revendications 1 à 13, dans lequel il est réalisé à une température dans la plage allant de 20 °C à 350 °C et à une pression dans la plage allant de 2 bar à 500 bar.

15. Procédé selon une ou plusieurs des revendications 1 à 14, dans lequel le métal M est choisi dans le groupe constitué par Ni, Co, Cu, Fe, Ag.
